# EUROPEAN PATENT APPLICATION

(11) **EP 4 796 555 A1**
(43) Date of publication of application: **26.08.2026**
(21) Application number: 24879286.3
(22) Date of filing: 21.10.2024
(51) Int. Cl.: C07H 15/04, C07H 15/12, A61K 9/51, A61K 48/00, A61P 27/02, A61P 1/16

(54) **LIPID COMPOUND AND COMPOSITION FOR TISSUE-SPECIFIC DELIVERY OF ACTIVE SUBSTANCE**

(30) Priority: 20.10.2023 KR 20230140899
(71) Applicant: Korea Research Institute of Bioscience and Biotechnology, Daejeon 34141 (KR)
(72) Inventor: KIM, Yoonkyung, Daejeon 34141 (KR); LEE, Ji Yoon, Daejeon 34141 (KR); JUNG, Hye-Youn, Daejeon 34141 (KR); JEON, Hae-Geun, Daejeon 34141 (KR); HO, Viet Cuong, Daejeon 34141 (KR)
(74) Representative: Vossius & Partner Patentanwälte Rechtsanwälte mbB
(86) International application number: PCT/IB2024/060320
(87) International publication number: WO 2025/083657

(57) **Abstract**

The present invention relates to a novel lipid compound for tissue-specific delivery and a lipid nanoparticle (LNP) composition comprising the same. The lipid nanoparticle comprises, as a component, a lipid compound modified such that an active substance therein is selectively delivered into cells of a specific tissue such as lymph nodes, spleen, retina, tumors, brain, liver, and the like *in vivo*, thereby preventing side effects and safely achieving desired therapeutic efficacy. These tissue-specific, non-viral LNP delivery systems can be effectively utilized for the prevention of infectious diseases and the treatment of rare and intractable (inherited) diseases (e.g., macular degeneration, diabetic retinopathy, inherited retinal diseases, cancer, brain diseases, liver diseases, etc.), where efficient and selective delivery to the targeted site in the body is crucial.

## Description

### Technical Field

The present disclosure relates to a lipid compound that selectively delivers an active substance into a cell of a specific tissue *in vivo*, and a lipid nanoparticle composition comprising the lipid compound.

### Background Art

During the recent COVID-19 pandemic, characterized by the urgent and successive emergence of new variants, mRNA-based vaccines were proven to be the only effective platform offering a rapid response and high efficacy. This is enabled by the use of lipid nanoparticles (LNPs), which serve as versatile and relatively safe delivery systems that allow mass production at a relatively low cost and require only replacement of the mRNA sequence. Accordingly, not only vaccines but also LNP-based therapeutic technologies have been rapidly developed. The LNPs maintain the structural stability of mRNA, and enhance mRNA delivery into the cytoplasm through high intracellular delivery efficiency and efficient endosomal escape, thereby improving the efficacy of the mRNA vaccines or therapeutics, and thus demand for the LNPs is expected to gradually increase.

LNPs were commercialized after being used as a delivery system for Onpattro, the first siRNA-based therapeutic approved by the U.S. Food and Drug Administration (FDA) in 2018. LNPs can reduce immune response and long-term toxicity, which are limitations of gene therapeutics using conventional viral vectors such as adeno-associated virus (AAV) and lentivirus, and can also overcome the limitation of cargo capacity (i.e., gene size) (<4.7 kb) of AAV Accordingly, LNPs are expected to serve as more safe and effective delivery system for various genes (RNA, siRNA, shRNA, rRNA, tRNA, mRNA, miRNA, saRNA, circRNA, DNA, cDNA, plasmid, DNAzyme, ribozyme, PNA, aptamer, antisense oligonucleotide, CRISPR, etc.) for the treatment of cancer and various rare and intractable inherited diseases (brain diseases, retinal diseases, etc.).

Meanwhile, to maximize the efficacy of the gene therapeutics using LNPs as a delivery system and to ensure safety by avoiding adverse effects caused by off-target delivery to non-target tissues, tissue-specific delivery (targeting) to the intended disease site (tissue) is essential. Experts regard the achievement of precise tissue-targeting properties as one of the core directions in the development of next-generation gene therapeutics.

As a representative example, inherited retinal dystrophy (IRD) is a progressive disorder caused by dysfunction or degeneration of retinal cells, leading to visual impairments such as decreased visual acuity, night blindness, and visual field constriction, ultimately resulting in blindness. Because the eye is an immune-privileged site, the development of gene therapeutics using delivery systems such as viral vectors has been actively conducted, and in 2017, Luxturna of Spark Therapeutics in the United States became the first FDA-approved gene therapy for the treatment of Leber congenital amaurosis (LCA), a subtype of IRD. Luxturna uses AAV, a viral vector widely used in recently commercialized gene therapeutics, as a delivery system, and is administered via subretinal injection. The subretinal administration generally enables efficient delivery to retinal pigment epithelium (RPE) or photoreceptor cells, where pathological phenomena occur, but the subretinal administration requires highly sophisticated surgery, carries a risk of complications, and limits repeated administration. In addition, AAV can load relatively small genes, and thus large causative genes such as EYS, USH2A, and ABCA4, which account for a substantial proportion of IRD cannot be delivered using AAV. Meanwhile, a non-negligible proportion of individuals have pre-existing antibodies against AAV, and immune (inflammatory) responses depending on the route of administration raise concerns regarding potential severe adverse effects, despite the immune-privileged site of the eye. Notably, atrophy of retinal pigment epithelial cells around the macular fovea due to immune responses to AAV has been reported several years after administration of AAV-based gene therapeutics to the retina of primates or humans. These findings highlight increasing concerns regarding the long-term safety of the gene therapies for subretinal administration using AAV as a delivery system.

Therefore, there is an increasing demand for the development of gene delivery systems that can overcome the limitations of the conventional AAV-based vectors and enable administration via intravitreal injection, which is safer and less technically demanding than subretinal injection, while also allowing repeated administration, unlike in the case of Luxturna. Although there have been reports of gene therapeutics using non-viral LNPs administered via subretinal injection, there have been no reports demonstrating effective delivery of active substance to the RPE and the photoreceptor cells in the outer retina, where pathological changes occur, via intravitreal injection.

Such tissue-specific, non-viral LNPs enable selective delivery of active substances into cells of specific tissues *in vivo*, such as lymph nodes, spleen, retina, tumors, brain, and liver*.* Therefore, LNP systems can be effectively utilized for the prevention of infectious diseases and the treatment of rare and intractable (inherited) diseases, including macular degeneration, diabetic retinopathy, inherited retinal diseases, cancer, brain diseases, liver diseases, etc.

Accordingly, to address the aforementioned problems, the present inventors developed a lipid nanoparticle composition comprising lipid compounds that selectively deliver an active substance, such as a gene, into cells of specific tissues *in vivo*. The efficacy of these systems has been confirmed, thereby completing the present disclosure.

### Disclosure of Invention

### Technical Problem

One object of the present disclosure is to provide a lipid compound for tissue-specific delivery of an active substance, an isomer thereof, or a pharmaceutically acceptable salt thereof.

Another object of the present disclosure is to provide a lipid nanoparticle composition for tissue-specific delivery of an active substance.

Another object of the present disclosure is to provide a method for preparing an active substance-lipid nanoparticle.

Another object of the present disclosure is to provide a composition for nucleic acid delivery.

Another object of the present disclosure is to provide a tissue-specific drug delivery system.

Another object of the present disclosure is to provide a drug delivery system for the treatment of retinal diseases.

Another object of the present disclosure is to provide a drug delivery system for the prevention of infectious diseases.

Another object of the present disclosure is to provide a drug delivery system for the treatment of cancer.

Another object of the present disclosure is to provide a drug delivery system for the treatment of brain diseases.

Another object of the present disclosure is to provide a drug delivery system for the treatment of liver diseases.

### Solution to Problem

To achieve the above object, one aspect of the present disclosure provides a lipid compound of the following Formula 1, an isomer thereof, or a pharmaceutically acceptable salt thereof.

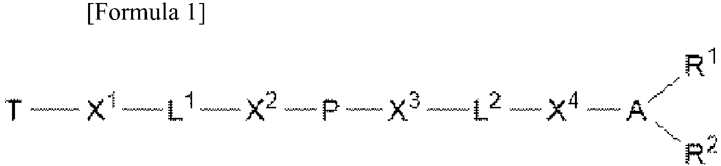

In Formula 1,
T is a targeting ligand, and is one selected from the group consisting of a monosaccharide, a polysaccharide, folic acid, transferrin, an RGD peptide, a cyclic RGD peptide, a TAT peptide, an R9 peptide, a CADY peptide, an HA2 peptide, a monoclonal antibody, an antigen-binding fragment or an antibody fragment, a single-chain variable fragment (scFv), and an aptamer, or a branched targeting ligand of the following Formula 2;
X¹, X², X³, and X⁴ are each independently one selected from the group consisting of a single bond, C₁-C₁₄ alkylene, C₁-C₁₄ heteroalkylene, C₂-C₁₄ alkenylene, C₁-C₆ alkyl-C(=O)-, C₂-C₆ alkenyl-C(=O)-, -(OCH₂CH₂)ₘ-, -(CH₂CH₂O)ₘ-, -O-, -S-, -S(=O)-, - S(=O)₂-, -S(=O)(=NR)-, -C(=O)-, -C(=O)O-, -OC(=O)-, -OC(=O)O-, -C(=O)C(=O)-, - C(=N-OR)-, -O-N=CR-, -OC=N-O-, -C(=O)NR-, -NRC(=O)-, -OC(=O)NR-, - NRC(=O)O-, -C(=O)NRC(=O)-, -S(=O)₂NR-, -NRS(=O)₂-, -NRC(=O)NR-, - NRC(=S)NR-, -C(=O)NH-N=CR-, -RC=N-NHC(=O)-, -NH-N=CR-, -RC=N-NH-, -NR-, -N(OR)-, -S-S-, 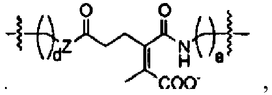 and functional groups bonded by a clickchemistry, or a combination thereof,
wherein m is an integer from 0 to 10,
Z is -NH-, -O-, or -S-, and
d and e are each an integer from 1 to 10;
L¹ and L² are each independently a single bond, C₁-C₁₄ alkylene, C₂-C₁₄ alkenylene, MR^{a}M¹, or R^{a}MR^{b};
P is a single bond, -(CH₂CH₂O)_{q}-, -(OCH₂CH₂)_{q}-, -CH₂O(CH₂CH₂O)_{q}CH₂-, or - CH₂CH₂O(CH₂CH₂O)_{q}CH₂-, wherein q is an integer from 0 to 120;
   A is CR^{d} or N;
R¹ and R² are each independently C₁-C₃₀ alkyl, C₂-C₃₀ alkenyl, or R^{c}MR^{d}, wherein C₁-C₃₀ alkyl and C₂-C₃₀ alkenyl are each independently unsubstituted or substituted with 1 to 3 C₁-C₁₆ alkyl or C₂-C₁₆ alkenyl;
M and M¹ are each independently C₁-C₁₄ alkylene, C₂-C₁₄ alkenylene, - NHC(=O)-, -C(=O)NH-, -C(=O)O-, -OC(=O)-, -C(=O)-, -NH-, -N⁺R₂-, -S-, -S-S-, -O-, - S(O)₂-, -C(=O)S-, -SC(=O)-, -NHC(=O)NH-, -NHC(=O)O-, -OC(=O)NH-, - OP(=O)(OR)O-, or -OP(=O)(OR)O(CH₂)_{g}-, wherein g is an integer from 1 to 10;
R is hydrogen, C₁-C₂₀ alkyl, C₂-C₂₀ alkenyl, C₂-C₂₀ alkynyl, or C₁-C₂₀ heteroalkyl,
R^{a} and R^{b} are each independently a single bond or C₁-C₁₀ alkylene;
R^{c} is a single bond, C₁-C₁₄ alkylene, or C₂-C₁₄ alkenylene;
R^{d} is hydrogen, C₁-C₁₈ alkyl, or C₂-C₁₈ alkenyl, wherein C₁-C₁₈ alkyl and C₂-C₁₈ alkenyl are each independently unsubstituted or substituted with 1 to 3 C₁-C₁₂ alkyl or C₂-C₁₂ alkenyl;
The heteroalkyl, the heteroalkylene, the heterocyclyl, and the heteroaryl each independently comprise 1 to 6 heteroatoms selected from N, O, and S;
The branched targeting ligand is represented by Formula 2 below,

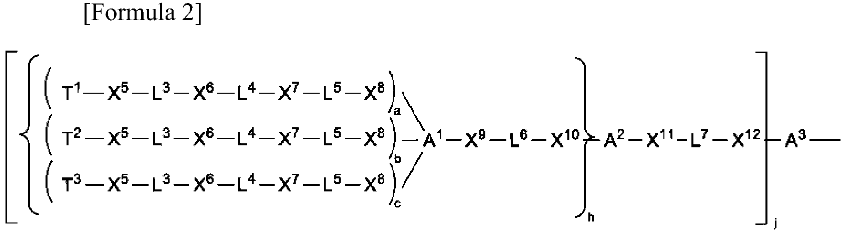

In Formula 2,
a, b, and c are each independently 0 or 1, wherein at least one of a, b, and c is 1, and h and j are 1 to 3;
T¹, T², and T³ are targeting ligands, and are each independently one selected from the group consisting of a monosaccharide, a polysaccharide, folic acid, transferrin, an RGD peptide, a cyclic RGD peptide, a TAT peptide, an R9 peptide, a CADY peptide, an HA2 peptide, a monoclonal antibody, an antigen-binding fragment or an antibody fragment, a single-chain variable fragment (scFv), and an aptamer;
X⁵, X⁶, X⁷, X⁸, X⁹, X¹⁰, X¹¹, and X¹² are each independently one selected from the group consisting of a single bond, C₁-C₁₄ alkylene, C₁-C₁₄ heteroalkylene, C₂-C₁₄ alkenylene, C₁-C₆ alkyl-C(=O)-, C₂-C₆ alkenyl-C(=O)-, -(OCH₂CH₂)ₘ-, -(CH₂CH₂O)ₘ-, -O-, -S-, -S(=O)-, -S(=O)₂-, -S(=O)(=NR)-, -C(=O)-, -C(=O)O-, -OC(=O)-, -OC(=O)O-, -C(=O)C(=O)-, -C(=N-OR)-, -O-N=CR-, -OC=N-O-, -C(=O)NR-, -NRC(=O)-, - OC(=O)NR-, -NRC(=O)O-, -C(=O)NRC(=O)-, -S(=O)₂NR-, -NRS(=O)₂-, - NRC(=O)NR-, -NRC(=S)NR-, -C(=O)NH-N=CR-, -RC=N-NHC(=O)-, -NH-N=CR-, - RC=N-NH-, -NR-, -N(OR)-, -S-S-, 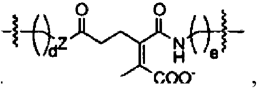 and functional groups bonded by a click chemistry, or a combination thereof,
   wherein m is an integer from 0 to 10,
Z is -NH-, -O-, or -S-, and
d and e are each an integer from 1 to 10;
L³, L⁴, L⁵, L⁶, and L⁷ are each independently a single bond, C₁-C₁₄ alkylene, C₂-C₁₄ alkenylene, MR^{a}M¹, or R^{a}MR^{b};
A¹, A², and A³ are each independently a single bond, CR^{d}, or N; and
The heteroalkyl and the heteroalkylene each independently comprise 1 to 6 heteroatoms selected from N, O, and S.

Another aspect of the present disclosure provides a lipid nanoparticle composition comprising the lipid compound represented by Formula 1, an isomer thereof, or a pharmaceutically acceptable salt thereof.

Another aspect of the present disclosure provides a method of preparing active substance-lipid nanoparticles, the method comprising mixing an organic phase comprising the lipid compound represented by Formula 1, an isomer thereof, a or pharmaceutically acceptable salt thereof, and an aqueous phase in which an active substance is dissolved.

Another aspect of the present disclosure provides a tissue-specific drug delivery system comprising the lipid compound represented by Formula 1, an isomer thereof, or a pharmaceutically acceptable salt thereof.

In addition, the present disclosure may provide a drug delivery system comprising the lipid compound represented by Formula 1, an isomer thereof, or a pharmaceutically acceptable salt thereof, for the treatment of retinal diseases.

In addition, the present disclosure may provide a drug delivery system comprising the lipid compound represented by Formula 1, an isomer thereof, or a pharmaceutically acceptable salt thereof, for the prevention of infectious diseases.

In addition, the present disclosure may provide a drug delivery system comprising the lipid compound represented by Formula 1, an isomer thereof, or a pharmaceutically acceptable salt thereof, for the treatment of cancer, brain diseases, or liver diseases.

In addition, the present disclosure may provide a method of delivering a drug, the method comprising administering the lipid compound represented by Formula 1, an isomer thereof, or a pharmaceutically acceptable salt thereof to a subject in need thereof.

In addition, the present disclosure may provide a use of the lipid compound represented by Formula 1, an isomer thereof, or a pharmaceutically acceptable salt thereof as a drug delivery system.

In addition, the present disclosure may provide a pharmaceutical composition or a pharmaceutical formulation comprising: a lipid nanoparticle comprising the lipid compound represented by Formula 1, an isomer thereof, or a pharmaceutically acceptable salt thereof; and a payload.

In addition, the present disclosure may provide a method of treatment of diseases, the method comprising administering the pharmaceutical composition or the pharmaceutical formulation to a subject.

### Advantageous Effects of Invention

The lipid nanoparticle composition comprising the lipid compound according to the present disclosure has excellent tissue-specific delivery efficiency, and thus may be useful as a targeting drug delivery system.

### Brief Description of Drawings

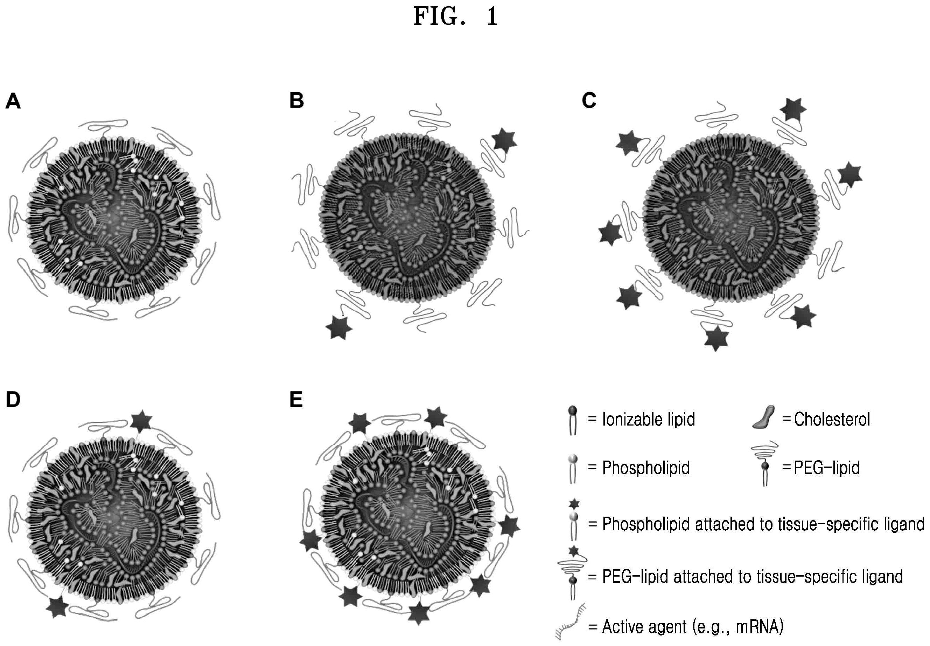

FIG. 1 is a schematic diagram showing the structure of mRNA-LNPs for tissue-specific delivery of an active substance according to an embodiment of the present disclosure. A is an LNP to which a tissue-specific ligand is not conjugated; B is an LNP in which only a portion of the surface is modified with PEG-lipids attached to a tissue-specific ligand; C is an LNP in which most of the surface is modified with PEG-lipids conjugated with a tissue-specific ligand; D is an LNP in which only a portion of the surface is modified with phospholipids conjugated with a tissue-specific ligand; and E is an LNP in which most of the surface is modified with phospholipids conjugated with a tissue-specific ligand.

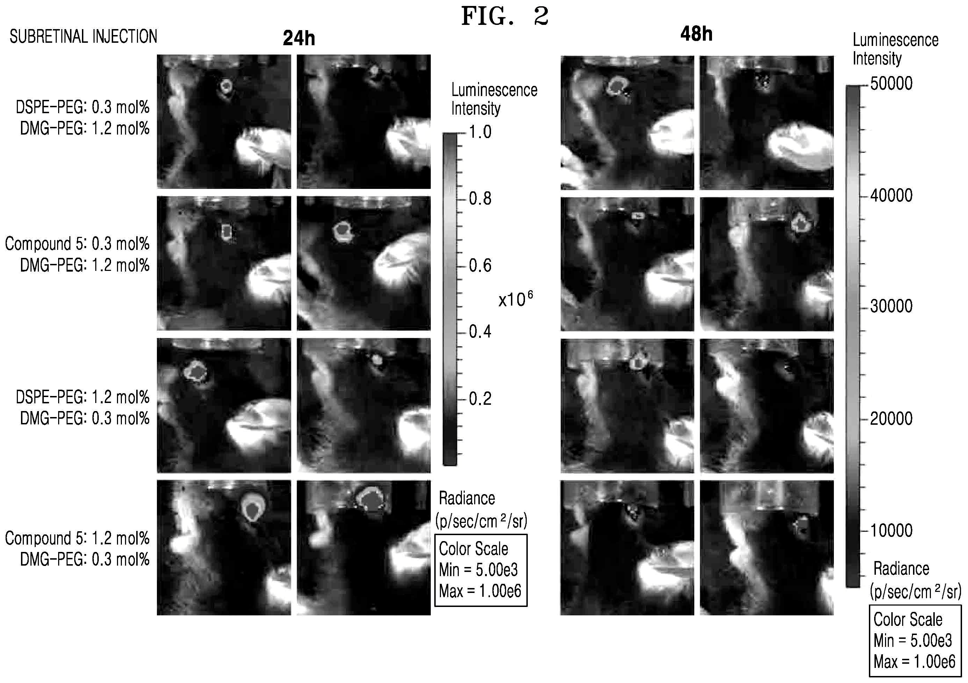

FIG. 2 shows IVIS images acquired at 24 and 48 h after administration of fLuc mRNA-LNPs comprising compound **5**, which is a modified PEG-lipid synthesized according to Example 5, into the eye of mice via subretinal injection, wherein the fLuc mRNA-LNPs were prepared according to Example 14 and injected according to Experimental Example 5.

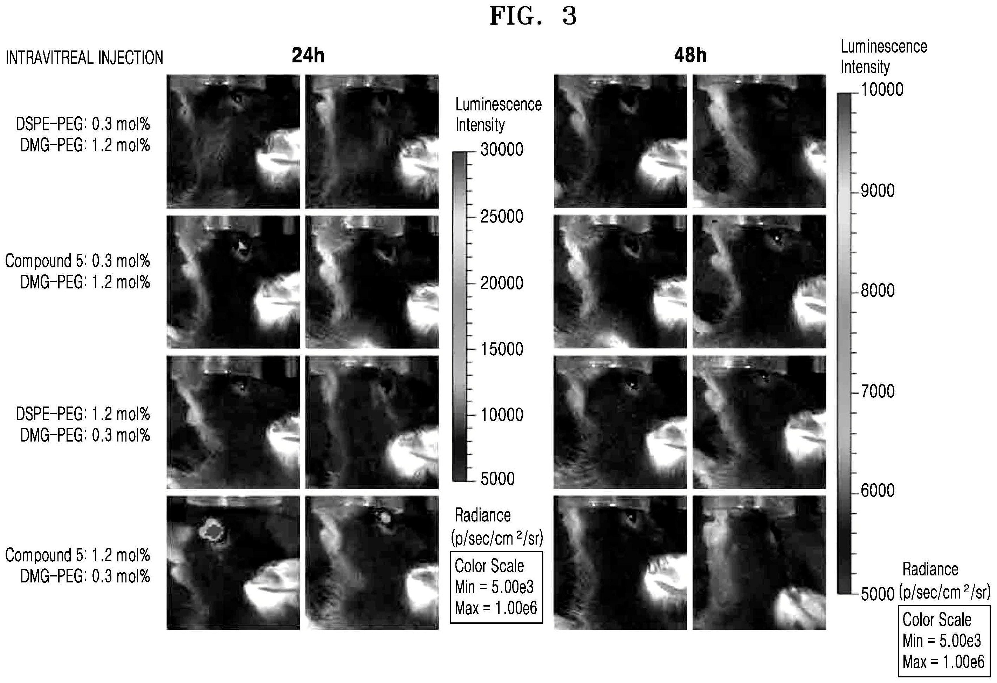

FIG. 3 shows IVIS images acquired at 24 and 48 h after administration of fLuc mRNA-LNPs comprising Compound **5**, which is a modified PEG-lipid synthesized according to Example 5, into the eye of mice via intravitreal injection, wherein the fLuc mRNA-LNPs were prepared according to Example 14 and injected according to Experimental Example 6.

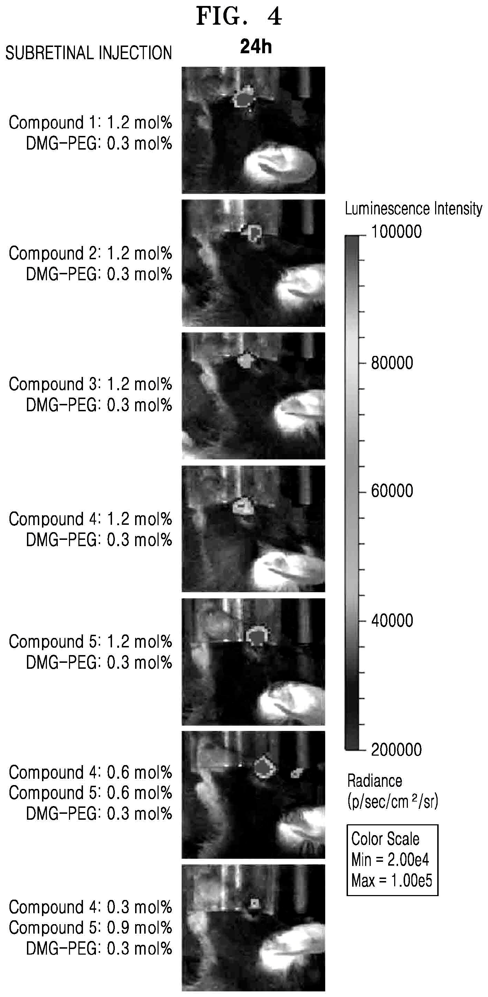

FIG. 4 shows IVIS images acquired at 24 h after administration of fLuc mRNA-LNPs comprising compounds **1**-**5**, which are modified PEG-lipids synthesized according to Examples 1 to 5, into the eye of mice via subretinal injection, wherein the fLuc mRNA-LNPs were prepared according to Example 14 and injected according to Experimental Example 5.

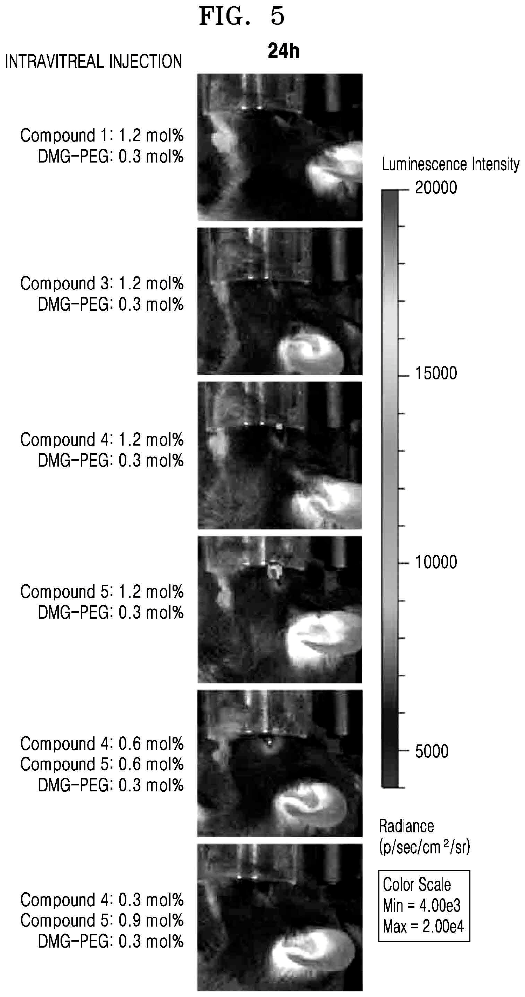

FIG. 5 shows IVIS images acquired at 24 h after administration of fLuc mRNA-LNPs comprising compounds **1**-**5**, which are modified PEG-lipids synthesized according to Examples 1 to 5, into the eye of mice via intravitreal injection, wherein the fLuc mRNA-LNPs were prepared according to Example 14 and injected according to Experimental Example 6.

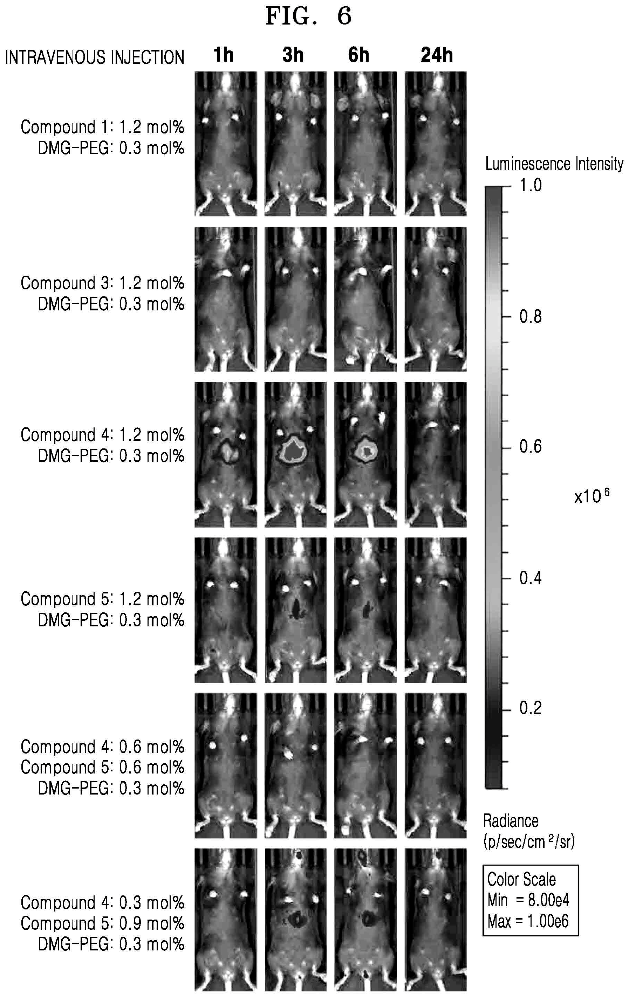

FIG. 6 shows IVIS images acquired at 1, 3, 6, and 24 h after administration of fLuc mRNA-LNPs comprising compounds **1**-**5**, which are modified PEG-lipids synthesized according to Examples 1 to 5, into the mice via intravenous injection through the tail vein, wherein the fLuc mRNA-LNPs were prepared according to Example 14 and injected according to Experimental Example 7.

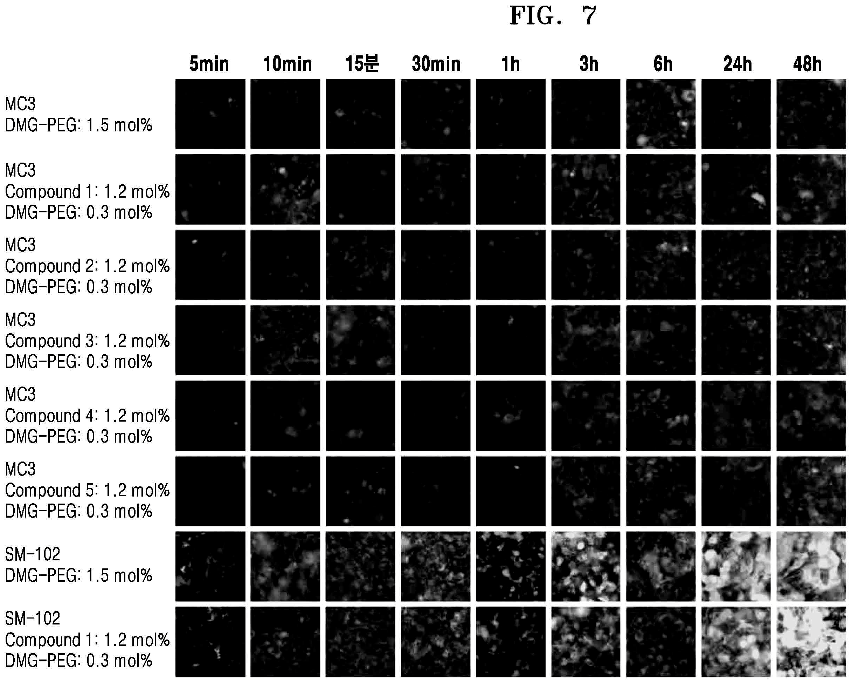

FIG. 7 shows fluorescence microscopy images acquired to confirm GFP expression patterns over time after administration of EGFP mRNA-LNPs w to ARPE-19 retinal cells according to an embodiment of the present disclosure. Red fluorescence corresponds to sulfo-cyanine5.5(Cy5.5)-DSPE incorporated as a portion (1 mol%) of phospholipids, constituting the LNP, for tracking the LNP.

### Mode for the Invention

Hereinafter, the present disclosure will be described in detail.

### Terms

Unless otherwise stated, the term "halogen" as used herein refers to F, Cl, Br, or I.

The term "hydroxy" refers to -OH group.

In the present disclosure, the term "alkyl" as used herein refers to a linear or branched saturated hydrocarbon functional group. For example, a "C₁-C₆ alkyl" has 1 to 6 carbon atoms. Specifically, the C₁-C₆ alkyl includes, but is not limited to, methyl, ethyl, n-propyl, isopropyl, n-butyl, isobutyl, tert-butyl, sec-butyl, n-pentyl, isopentyl, neopentyl, tert-pentyl, 1-methylbutyl, n-hexyl, 1-methylpentyl, 2-methylpentyl, 4-methyl-2-pentyl, 3,3-dimethylbutyl, 2-ethylbutyl, and the like. The alkyl group may be independently substituted with one or more substituents; for example, a methyl group may be substituted with functional groups such as 1 to 3 halogens, hydroxy, thiol, or selenol, as well as a C₁-C₆ alkyl and other hydrocarbon groups.

The term "alkylene" refers to a linear or branched saturated hydrocarbon chain without hydrogen at both ends such as ethylene (-CH₂CH₂-) and propylene (-CH₂CH₂CH₂-). The alkylene group may be independently substituted with one or more substituents.

The term "alkenyl" refers to a linear or branched unsaturated hydrocarbon group containing one or more double bonds, and the alkenyl group may be independently substituted with one or more substituents. For example, a "C₂-C₆ alkenyl" has 2 to 6 carbon atoms. Specifically, the C₂-C₆ alkenyl includes, but is not limited to, ethenyl (vinyl group), n-propenyl, n-butenyl, 1-methyl-2-propenyl, 2-methyl-2-propenyl, n-pentenyl, 1-methyl-2-butenyl, 2-methyl-2-butenyl, n-hexenyl, and the like.

The term "alkenylene" refers to a linear or branched unsaturated hydrocarbon chain having at least one double bond and no hydrogen at both ends such as propenylene (-CH=CHCH₂-) and 2-pentenylene (-CH₂CH=CHCH₂CH₂-). The alkenylene group may be independently substituted with one or more substituents.

The term "alkoxy" refers to the chemical formula '-O-alkyl', and the alkoxy group may be independently substituted with one or more substituents. For example, C₁-C₆ alkoxy includes, but is not limited to, methoxy, ethoxy, n-propoxy, isopropoxy, n-butoxy, isobutoxy, tert-butoxy, n-pentoxy, isopentoxy, tert-pentoxy, sec-pentoxy, neopentoxy, hexyloxy, and the like. In an embodiment, the alkoxy group may be substituted with one or more substituents; for example, 1 to 3 halogens, hydroxy, C₁-C₆ alkyl, and other hydrocarbon groups.

The term "alkenyloxy" refers to the chemical formula '-O-alkenyl', and the alkenyloxy group may be independently substituted with one or more substituents. For example, C₂-C₆ alkenyloxy includes, but is not limited to, ethenyloxy, n-propenyloxy, isopropenyloxy, n-butenyloxy, 1-methyl-2-propenyloxy, 2-methyl-2-propenyloxy, n-pentenyloxy, 1-methyl-2-butenyloxy, 2-methyl-2-butenyloxy, n-hexenyloxy, and the like. In an embodiment, the alkenyloxy group may be substituted with one or more substituents; for example, 1 to 3 halogens, hydroxy, C₁-C₆ alkyl, and other hydrocarbon groups.

The term "cycloalkyl" refers to one or more saturated rings or one or more non-aromatic ring hydrocarbon groups, wherein the non-aromatic ring may have a certain degree of unsaturation. Unless otherwise defined, the cycloalkyl may be a monocyclic ring or polycyclic rings. Wherein, the polycyclic rings include all of the fused rings, bridged rings, and spiro rings. For example, a "C₃-C₁₄ cycloalkyl" refers to cycloalkyl having 3 to 14 carbon atoms forming the ring. Specifically, the C₃-C₁₄ cycloalkyl includes, but is not limited to, cyclopropyl, cyclobutyl, cyclopentyl, cyclohexyl, cycloheptyl, cyclopentenyl, cyclopentadienyl, cyclohexenyl, cyclohexadienyl, cyclooctyl, bicyclo[1.1.0]butyl, bicyclo[2.2.1]heptyl, adamantyl, and the like. In an embodiment, the cycloalkyl group may be optionally substituted with one or more substituents. In an embodiment, 0, 1, 2, 3, or 4 atoms of each ring of the cycloalkyl group may be substituted with a substituent.

The term "aryl" refers to a monocyclic or bicyclic aromatic ring hydrocarbon group. That is, in the present disclosure, the aryl may include phenyl, naphthyl, biaryl, and the like, unless otherwise defined. For example, C₆-C₁₀ aryl refers to aromatic rings having 6 to 10 carbon atoms. In an embodiment, 0, 1, 2, 3, 4, 5, or 6 atoms of each ring of the aryl group may be substituted with a substituent.

The terms "heteroalkyl" and "heteroalkylene" each independently contain 1 to 6 heteroatoms selected from N, O, and S.

The term "heteroaryl" refers to a 5- to 10-membered monocyclic or bicyclic aromatic heterocycle containing 1 to 6 heteroatoms selected from N, O, and S. Unless otherwise defined, the heteroaryl may be a monocyclic ring or polycyclic rings. For example, "5- to 10-membered heteroaryl" may be a 5- or 6-membered aromatic heterocycle containing 1 to 4 heteroatoms selected from N, O, and S, or may be a bicyclic ring in which the heteroaryl ring is fused to a benzene ring or other heteroaryl ring. In an embodiment, 0, 1, 2, 3, or 4 atoms of each ring of the heteroaryl group may be substituted with a substituent. Examples of the monocyclic heteroaryl include, but are not limited to, pyrrolyl, furanyl, thiophenyl, pyrazolyl, imidazolyl, isoxazolyl, oxazolyl, isothiazolyl, thiazolyl, 1,2,3-triazolyl, 1,3,4-triazolyl, 1-oxa-2,3-diazolyl, 1-oxa-2,4-diazolyl, 1-oxa-2,5-diazolyl, 1-oxa-3,4-diazolyl, 1-thia-2,3-diazolyl, 1-thia-2,4-diazolyl, 1-thia-2,5-diazolyl, 1-thia-3,4-diazolyl, tetrazolyl, pyridinyl, pyridazinyl, pyrimidinyl, pyrazinyl, and groups similar thereto. Examples of the bicyclic heteroaryl include, but are not limited to, indolyl, isoindolyl, benzofuranyl, benzothiophenyl, indazolyl, benzimidazolyl, benzotriazolyl, pyrrolo[2,3-*b*]pyridinyl, pyrrolo[2,3-*c*]pyridinyl, pyrrolo[3,2-*c*]pyridinyl, pyrrolo[3,2-*b*]pyridinyl, imidazo[4,5-*b*]pyridinyl, imidazo[4,5-*c*]pyridinyl, pyrazolo[4,3-*d*]pyridinyl, pyrazolo[4,3-*c*]pyridinyl, pyrazolo[3,4-*c*]pyridinyl, pyrazolo[3,4-*b*]pyridinyl, purinyl, indolizinyl, imidazo[1,2-*a*]pyridinyl, imidazo[1,5-*a*]pyridinyl, pyrazolo[1,5-*a*]pyridinyl, pyrrolo[1,2-*b*]pyridazinyl, imidazo[1,2-*c*]pyrimidinyl, quinolinyl, isoquinolinyl, cinnolinyl, azaquinazolinyl, quinoxalinyl, phthalazinyl, 1,6-naphthyridinyl, 1,7-naphthyridinyl, 1,8-naphthyridinyl, 1,5-naphthyridinyl, 2,6-naphthyridinyl, 2,7-naphthyridinyl, pyrido[3,2-*d*]pyrimidinyl, pyrido[4,3-*d*]pyrimidinyl, pyrido[3,4-*d*]pyrimidinyl, pyrido[2,3-*b*]pyrimidinyl, pyrido[2,3-*b*]pyrazinyl, pyrido[3,4-*b*]pyrazinyl, pyrimido[5,4-*d*]pyrimidinyl, pyrazino[2,3-*b*]pyrazinyl, pyrimido[4,5-*d*]pyrimidinyl, and groups similar thereto.

The term "heterocyclyl" refers to a saturated or partially unsaturated ring containing 1 to 6 heteroatoms selected from N, O, and S in addition to carbon atoms. Unless otherwise defined, the heterocyclyl may be a monocyclic ring or polycyclic rings. For example, "3- to 14-membered heterocyclyl" refers to an aliphatic heterocycle containing 3 to 14 atoms forming a ring, and may include a 3- to 6-membered aliphatic heterocycle, or a bicyclic ring in which the heterocyclic ring is fused to a benzene ring or another heterocyclic ring. In an embodiment, 0, 1, 2, 3, or 4 atoms of each ring of the heterocyclyl group may be substituted with a substituent. For example, the heterocyclyl includes, but is not limited to, azetidinyl, pyrrolidinyl, tetrahydrofuranyl, tetrahydrothienyl, pyrazolidinyl, imidazolidinyl, oxazolidinyl, isoxazolidinyl, thiazolidinyl, dioxolyl, piperidinyl, tetrahydropyranyl, tetrahydrothiopyranyl, piperazinyl, morpholinyl, thiomorpholinyl, 1,1-dioxo-thiomorpholin-4-yl, azepanyl, diazepanyl, homopiperazinyl, oxazepanyl, indolyl, isoindolyl, dihydroindolyl, dioxoisoindolinyl, dihydrofuryl, dihydroimidazolinyl, dihydrooxazolyl, dihydrobenzodioxinyl, tetrahydropyridinyl, dihydropyranyl, dihydrobenzofuranyl, benzodioxolyl, benzodioxanyl, and groups similar thereto.

The term "substitution" refers to replacing an atom or a functional group in a molecular structure with another atom or a functional group (i.e., a substituent) without exceeding the valence of the specified atom, such that a chemically stable compound is formed. For example, that "group A is substituted with substituent B" may mean that an atom bound to another atom such as a carbon constituting the backbone of group A is substituted with substituent B, thereby forming a covalent bond between group A and substituent B.

The term "substituent" refers to another group bound to a parent scaffold group and the substituent may be one or more. When there are multiple substituents, each substituent may be identical or different from each other. When both the parent scaffold group and the substituent are hydrocarbon groups, the number of carbon atoms of the parent scaffold group does not include the number of carbon atoms of the substituent. For example, a butyl group (-C₄H₉) having a methoxy group (-O-CH₃) as a substituent is classified as a C1 alkoxy group and a C4 alkyl group.

The term "N/P ratio" is, for example, a molar ratio of ionizable (e.g., in a physiological pH range) nitrogen atoms in a lipid (or lipids) to phosphate groups in a nucleic acid molecular entity (or nucleic acid molecular entities) in a nanoparticle composition comprising a lipid component and RNA.

The term "isomer" refers to multiple stereoisomers. In embodiments of the present disclosure, multiple stereoisomers may be generated during a preparation process. Unless specific stereoisomers are indicated, all stereoisomers that may be generated during the reaction may be included.

In embodiments of the present disclosure, the following abbreviations are used throughout: "Ac" refets to acetyl; "AcO" or "OAc" refers to acetoxy; "ACN" refers to acetonitrile; "aq" refers to aqueous; "BOC", "Boc", or "boc" refers to *N*-*tert-*butoxycarbonyl; "Bn" refers to benzyl; "Bu" refers to butyl; "nBu" refers to normal-butyl; "tBu" refers to *tert*-butyl; "Cbz" refers to benzyloxycarbonyl; "DCC" refers to *N*,*N*'-dicyclohexylcarbodiimide; "DCM" refers to methylene chloride (CH₂Cl₂); "DEA" refers to diethylamine; "DIPEA" refers to diisopropylethylamine; "DMF" refers to *N*,*N-*dimethylformamide; "DMSO" refers to dimethyl sulfoxide; "EDC" refers to 1-ethyl-3-(3-dimethylaminopropyl)carbodiimide; "EDTA" refers to ethylenediaminetetraacetic acid; "Et" refers to ethyl; "EtOAc" refers to ethyl acetate; "EtOH" refers to ethanol; "HATU" refers to 1-[bis(dimethylamino)methylene]-1H-1,2,3-triazolo[4,5-*b*]pyridinium 3-oxide hexafluorophosphate; "HOAc" or "AcOH" refers to acetic acid; "IPA" refers to isopropyl alcohol; "LAH" refers to lithium aluminum hydride; "mCPBA" refers to *meta-*chloroperoxybenzoic acid; "Me" refers to methyl; "MeOH" refers to methanol; "MS" refers to mass spectrometry; "MTBE" refers to methyl *tert*-butyl ether; "NHS" refers to N-hydroxysuccinimide; "Ph" refers to phenyl; "PyBOP" refers to benzotriazol-1-yloxytripyrrolidinophosphonium hexafluorophosphate; "TFA" refers to trifluoroacetic acid; "THF" refers to tetrahydrofuran; "TLC" refers to thin-layer chromatography; "Rf" refers to retention factor; "rt" refers to retention time; "r.t." refers to room temperature; "h" refers to hour(s); "min" refers to minute(s); "s" refers to second(s); "equiv." refers to equivalent(s); and "sat." refers to saturated.

The terms "active substance", "drug", or "payload" refer to a biologically active substance, and refer to any substance that has a therapeutic, diagnostic, and/or prophylactic effect and/or induces a desired biological and/or pharmacological effect when administered to a subject. Such an active substance includes, but is not limited to, cytotoxins, radioactive ions, chemotherapeutic agents, small molecule drugs, proteins, nucleic acids, and the like. When delivered to cells or organs, the active substance may exhibit desirable changes in cells, organs, or another body tissues or systems, and may be useful in the treatment of one or more diseases, disorders, or pathologies. As a specific example, the active substance may refer to RNA, siRNA, shRNA, rRNA, tRNA, mRNA, miRNA, saRNA, circRNA, DNA, cDNA, plasmid, DNAzyme, ribozyme, PNA, aptamer, antisense oligonucleotide, CRISPR, protein, carbohydrate, drug, or the like.

As used in the present disclosure, the term "prevention" refers to any action that inhibits or delays the occurrence, spread, and recurrence of the disease by administration of the compound or the pharmaceutical composition according to the present disclosure, and the term "treatment" refers to any action that ameliorates or beneficially alters symptoms of the disease by administration of the compound or the pharmaceutical composition according to the present disclosure.

As used in the present disclosure, the terms "poly(ethylene glycol)", "PEG", a "PEG moiety", and a "PEG derivative" include a functional group corresponding to poly(ethylene glycol), and may be used interchangeably. Combinations of the above-described elements in all possible variations thereof are encompassed unless otherwise indicated herein or otherwise clearly contradicted by context.

A "dosage unit" refers to a form in which a pharmaceutical formulation is provided, such as a pill, a tablet, or other dosage units known in the art. In certain embodiments, the dosage unit is a vial containing a lyophilized antisense oligonucleotide. In certain embodiments, the dosage unit is a vial containing a reconstituted antisense oligonucleotide.

A "dose" refers to a specific amount of a medicament provided in a single administration or provided over a specific period of time. In certain embodiments, the dose may be administered in one, two, or more boluses, tablets, or injections. For example, in certain embodiments where subcutaneous administration is desired, the desired dose requires a volume that is not easily accommodated by a single injection, and thus two or more injections may be used to achieve the desired dose. In certain embodiments, the pharmaceutical formulation is administered over an extended period of time or continuously by an infusion. The dose may be expressed as an amount of the pharmaceutical formulation per hour, day, week, or month. The dose may also be expressed as mg/kg or g/kg.

The present disclosure provides a compound of the following Formula 1, an isomer thereof, or a pharmaceutically acceptable salt thereof:

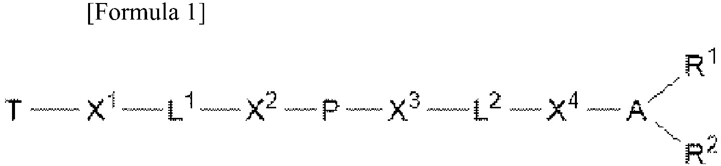

In Formula 1,
T is a targeting ligand, and is one selected from the group consisting of a monosaccharide, a polysaccharide, folic acid, transferrin, an RGD peptide, a cyclic RGD peptide, a TAT peptide, an R9 peptide, a CADY peptide, an HA2 peptide, a monoclonal antibody, an antigen-binding fragment or an antibody fragment, a single-chain variable fragment (scFv), and an aptamer, or a branched targeting ligand of the following Formula 2;
X¹, X², X³, and X⁴ are each independently one selected from the group consisting of a single bond, C₁-C₁₄ alkylene, C₁-C₁₄ heteroalkylene, C₂-C₁₄ alkenylene, C₁-C₆ alkyl-C(=O)-, C₂-C₆ alkenyl-C(=O)-, -(OCH₂CH₂)ₘ-, -(CH₂CH₂O)ₘ-, -O-, -S-, -S(=O)-, - S(=O)₂-, -S(=O)(=NR)-, -C(=O)-, -C(=O)O-, -OC(=O)-, -OC(=O)O-, -C(=O)C(=O)-, - C(=N-OR)-, -O-N=CR-, -OC=N-O-, -C(=O)NR-, -NRC(=O)-, -OC(=O)NR-, - NRC(=O)O-, -C(=O)NRC(=O)-, -S(=O)₂NR-, -NRS(=O)₂-, -NRC(=O)NR-, - NRC(=S)NR-, -C(=O)NH-N=CR-, -RC=N-NHC(=O)-, -NH-N=CR-, -RC=N-NH-, -NR-, -N(OR)-, -S-S-, 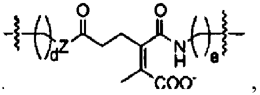 and functional groups bonded by a click chemistry, or a combination thereof,
wherein m is an integer of 0 to 10,
Z is -NH-, -O-, or -S-, and
d and e are each an integer from 1 to 10;
L¹ and L² are each independently a single bond, C₁-C₁₄ alkylene, C₂-C₁₄ alkenylene, MR^{a}M¹, or R^{a}MR^{b};
   P is a single bond, -(CH₂CH₂O)_{q}-, -(OCH₂CH₂)_{q}-, -CH₂O(CH₂CH₂O)_{q}CH₂-, or - CH₂CH₂O(CH₂CH₂O)_{q}CH₂-, wherein q is 0 or an integer from 1 to 120;
   A is CR^{d} or N;
R¹ and R² are each independently C₁-C₃₀ alkyl, C₂-C₃₀ alkenyl, or RcMRd, wherein C₁-C₃₀ alkyl and C₂-C₃₀ alkenyl are each independently unsubstituted or substituted with 1 to 3 C₁-C₁₆ alkyl or C₂-C₁₆ alkenyl;
M and M¹ are each independently C₁-C₁₄ alkylene, C₂-C₁₄ alkenylene, - NHC(=O)-, -C(=O)NH-, -C(=O)O-, -OC(=O)-, -C(=O)-, -NH-, -N⁺R₂-, -S-, -SS-, -O-, - S(O)₂-, -C(=O)S-, -SC(=O)-, -NHC(=O)NH-, -NHC(=O)O-, -OC(=O)NH-, - OP(=O)(OR)O-, or -OP(=O)(OR)O(CH₂)_{g}-, wherein g is an integer from 1 to 10;
   R is hydrogen, C₁-C₂₀ alkyl, C₂-C₂₀ alkenyl, C₂-C₂₀ alkynyl, or C₁-C₂₀ heteroalkyl,
R^{a} and R^{b} are each independently a single bond or C₁-C₁₀ alkylene;
R^{c} is a single bond, C₁-C₁₄ alkylene, or C₂-C₁₄ alkenylene;
R^{d} is hydrogen, C₁-C₁₈ alkyl, or C₂-C₁₈ alkenyl, wherein C₁-C₁₈ alkyl and C₂-C₁₈ alkenyl are each independently unsubstituted or substituted with 1 to 3 C₁-C₁₂ alkyl or C₂-C₁₂ alkenyl;
The heteroalkyl, the heteroalkylene, the heterocyclyl, and the heteroaryl each independently comprise 1 to 6 heteroatoms selected from N, O, and S;
The branched targeting ligand is represented by Formula 2 below,

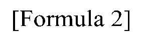

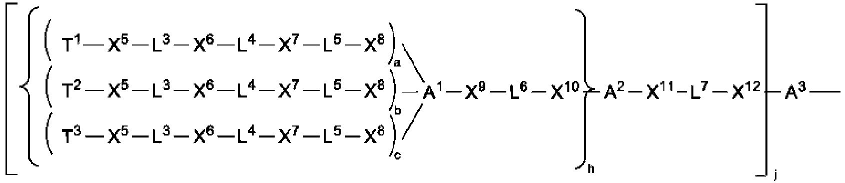

In Formula 2,
a, b, and c are each independently 0 or 1, wherein at least one of a, b, and c is 1, and h and j are 1 to 3;
T¹, T², and T³ are each independently one selected from the group consisting of a monosaccharide, a polysaccharide, folic acid, transferrin, an RGD peptide, a cyclic RGD peptide, a TAT peptide, an R9 peptide, a CADY peptide, an HA2 peptide, a monoclonal antibody, an antigen-binding fragment or an antibody fragment, a single-chain variable fragment (scFv), and an aptamer;
X⁵, X⁶, X⁷, X⁸, X⁹, X¹⁰, X¹¹, and X¹² are each independently one selected from the group consisting of a single bond, C₁-C₁₄ alkylene, C₁-C₁₄ heteroalkylene, C₂-C₁₄ alkenylene, C₁-C₆ alkyl-C(=O)-, C₂-C₆ alkenyl-C(=O)-, -(OCH₂CH₂)ₘ-, -(CH₂CH₂O)ₘ-, -O-, -S-, -S(=O)-, -S(=O)₂-, -S(=O)(=NR)-, -C(=O)-, -C(=O)O-, -OC(=O)-, -OC(=O)O-, -C(=O)C(=O)-, -C(=N-OR)-, -O-N=CR-, -OC=N-O-, -C(=O)NR-, -NRC(=O)-, - OC(=O)NR-, -NRC(=O)O-, -C(=O)NRC(=O)-, -S(=O)₂NR-, -NRS(=O)₂-, - NRC(=O)NR-, -NRC(=S)NR-, -C(=O)NH-N=CR-, -RC=N-NHC(=O)-, -NH-N=CR-, - RC=N-NH-, -NR-, -N(OR)-, -S-S-, 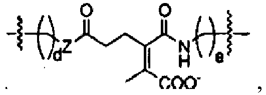 and functional groups bonded by a click chemistry, or a combination thereof,
wherein m is an integer from 0 to 10,
Z is -NH-, -O-, or -S-, and
d and e are each an integer from 1 to 10;
L³, L⁴, L⁵, L⁶, and L⁷ are each independently a single bond, C₁-C₁₄ alkylene, C₂-C₁₄ alkenylene, MR^{a}M¹, or R^{a}MR^{b};
A¹, A², and A³ are each independently a single bond, CR^{d}, or N; and
The heteroalkyl and the heteroalkylene each independently comprise 1 to 6 heteroatoms selected from N, O, and S.

In an embodiment, the T as a targeting ligand may be selected from a monosaccharide, a polysaccharide, folic acid, transferrin, an RGD peptide, a cyclic RGD peptide, a TAT peptide, an R9 peptide, a CADY peptide, an HA2 peptide, a monoclonal antibody, an antigen-binding fragment or an antibody fragment, a single-chain variable fragment (scFv), or an aptamer. Herein, the antigen-binding fragment or the antibody fragment refers to a fragment retaining an antigen-binding function, and may include Fab, F(ab'), F(ab')₂, Fv, and the like. As a specific example, an antibody that may be used as the targeting ligand includes, but is not limited to, anti-CD3, anti-CD19, anti-CD20, anti-CD22, anti-CD33, anti-CD38, anti-CD54, anti-CD74, anti-CD138, anti-CD166, anti-CD209, anti-cMET, anti-EGFR, anti-HER2, anti-HIV-gp120, anti-HLA-DR, an anti-transferrin receptor (TfRscFv), and the like.

The monosaccharide may be selected from the group consisting of glucose, *N-*acetylglucose, galactose, *N*-acetylgalactose, mannose, allose, altrose, arabinose, cladinose, erythrose, erythrulose, fructose, D-fucitol, L-fucitol, fucosamine, fucose, fuculose, galactosamine, D-galactosaminitol, *N*-acetylgalactosamine, glucosamine, *N-*acetylglucosamine, glucosaminitol, glucose-6-phosphate, glyceraldehyde, L-glycero-D-manno-heptose, glycerol, glycerone, gulose, idose, lyxose, mannosamine, mannose-6-phosphate, psicose, quinovose, quinovosamine, rhamnitol, rhamnosamine, rhamnose, ribose, ribulose, sedoheptulose, sorbose, tagatose, talose, tartaric acid, threose, xylose, xylulose, *N*,*N*,*N*-trimethylglucosamine, and *N*,*N*,*N*-trimethylgalactosamine.

In addition, the monosaccharide may be in a D- or L-configuration. The monosaccharide may include monosaccharide substituted with at least one substituent. For example, the monosaccharide may be a deoxy sugar (in which an alcoholic hydroxy group is replaced with hydrogen), an amino sugar (in which an alcoholic hydroxy group is replaced with an amino group), a thio sugar (in which an alcoholic hydroxy group is replaced with a thiol, C=O is replaced with C=S, or a ring oxygen in a cyclic form is replaced with sulfur), a seleno sugar, a telluro sugar, an aza sugar (in which a ring carbon is replaced with nitrogen), an imino sugar (in which a ring oxygen is replaced with nitrogen), a phosphano sugar (in which a ring oxygen is replaced with phosphorus), a phospha sugar (in which a ring carbon is replaced with phosphorus), a C-substituted monosaccharide (in which a hydrogen on a non-terminal carbon atom is replaced with carbon), an unsaturated monosaccharide, an alditol (in which a carbonyl group is replaced with a CHOH group), an aldonic acid (in which an aldehydic group is replaced with a carboxy group), a ketoaldonic acid, a uronic acid, an aldaric acid, or the like. The amino sugar may include an amino monosaccharide, preferably galactosamine, glucosamine, mannosamine, fucosamine, quinovosamine, neuraminic acid, muramic acid, lactosediamine, acosamine, bacillosamine, daunosamine, desosamine, forosamine, garosamine, kanosamine, mycaminose, myosamine, perosamine, pneumosamine, purpurosamine, and rhodosamine.

The polysaccharide includes a disaccharide, a trisaccharide, and an oligosaccharide, and may be selected from the group consisting of abequose, acarbose, amicetose, amylopectin, amylose, apiose, arcanose, ascarylose, ascorbic acid, boivinose, cellobiose, cellotriose, cellulose, chacotriose, chalcose, chitin, colitose, cyclodextrin, cymarose, dextrin, 2-deoxyribose, 2-deoxyglucose, diginose, digitalose, digitoxose, evalose, evernitrose, fructooligosaccharide, galactooligosaccharide, gentianose, gentiobiose, glucan, glucogen, glycogen, hamamelose, heparin, inulin, isolevoglucosenone, isomaltose, isomaltotriose, isopanose, kojibiose, lactose, lactosamine, lactosediamine, laminarabiose, levoglucosan, levoglucosenone, β-maltose, maltotriose, mannan oligosaccharide, manninotriose, melezitose, melibiose, muramic acid, mycarose, mycinose, neuraminic acid, nigerose, nojirimycin, noviose, oleandrose, panose, paratose, planteose, primeverose, raffinose, rhodinose, rutinose, sarmentose, sedoheptulose, solatriose, sophorose, stachyose, streptose, sucrose, α,α-trehalose, trehalosamine, turanose, tyvelose, xylobiose, and umbelliferose.

In addition, the polysaccharide may include polysaccharide substituted with at least one substituent. For example, the disaccharide may include an amino sugar and a derivative thereof, particularly mycaminose derivatized at a C-4' position or 4-deoxy-3-amino-glucose derivatized at a C-6' position.

In an embodiment, the functional groups bonded to each other by a click chemistry may be, for example, 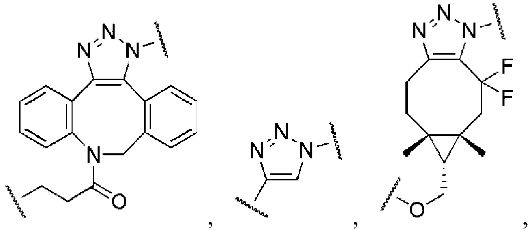 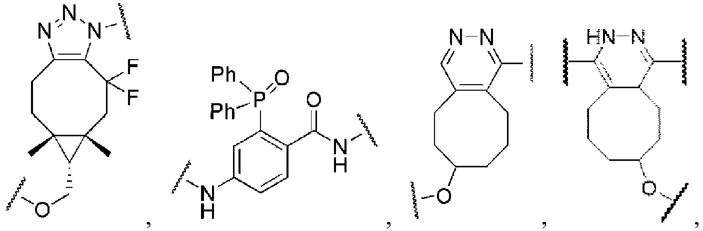 and the like, and any functional group bonded to each other by a click chemistry known in the art may be used without limitation.

T, T¹, T², and T³ may each independently be selected from the group consisting of glucose (Glu), N-acetylglucosamine (GlcNAc), galactose (Gal), *N-*acetylgalactosamine (GalNAc), and mannose (Man). Preferably, T may be selected as *N-*acetylgalactosamine (GalNAc) or mannose (Man).

Specific examples of the compound of Formula 1 according to the present disclosure are as follows, but are not limited thereto:
(1) Glu-DBCO-PEG2000-DSPE;
(2) GlcNAc-DBCO-PEG2000-DSPE;
(3) Gal-DBCO-PEG2000-DSPE;
(4) GalNAc-DBCO-PEG2000-DSPE;
(5) Man-DBCO-PEG2000-DSPE;
(6) Glu-succinamide-PEG2000-DMG;
(7) GlcNAc-succinamide-PEG2000-DMG;
(8) Gal-succinamide-PEG2000-DMG;
(9) GalNAc-succinamide-PEG2000-DMG;
(10) Man-succinamide-PEG2000-DMG;
(11) (R)-2,3-bis(stearoyloxy)propyl (2-(dimethyl(2-(((2R,3R,4S,5S,6R)-3,4,5-trihydroxy-6-(hydroxymethyl)tetrahydro-2H-pyran-2-yl)oxy)ethyl)ammonio)ethyl) phosphate;
(12) 2-((2-(((2R,3R,4S,5S,6R)-3-acetamido-4,5-dihydroxy-6-(hydroxymethyl)tetrahydro-2Hpyran-2-yl)oxy)ethyl)dimethylammonio)ethyl ((R)-2,3-bis(stearoyloxy)propyl) phosphate;
(13) (R)-2,3-bis(stearoyloxy)propyl (2-(dimethyl(2-(((2R,3R,4S,5R,6R)-3,4,5-trihydroxy-6-(hydroxymethyl)tetrahydro-2H-pyran-2-yl)oxy)ethyl)ammonio)ethyl) phosphate;
(14) 2-((2-(((2R,3R,4S,5R,6R)-3-acetamido-4,5-dihydroxy-6-(hydroxymethyl)tetrahydro-2H-pyran-2-yl)oxy)ethyl)dimethylammonio)ethyl ((R)-2,3-bis(stearoyloxy)propyl) phosphate;
(15) (R)-2,3-bis(stearoyloxy)propyl (2-(dimethyl(2-(((2S,3S,4S,5S,6R)-3,4,5-trihydroxy-6-(hydroxymethyl)tetrahydro-2H-pyran-2-yl)oxy)ethyl)ammonio)ethyl) phosphate;
(16) Man-1,2,3-triazole-PEG2000-DSPE;
(17) Man-DIFO-PEG2000-DSPE;
(18) Man-BCN-PEG2000-DSPE;
(19) Man-triarylphosphine-PEG2000-DSPE;
(20) Man-TCO-PEG2000-DSPE;
(21) Man-succinamide-PEG2000-carbamate-DSPE;
(22) Man-amide-PEG2000-amide-DSPE;
(23) Man-pentanoate-PEG2000-amide-DSPE;
(24) Man-succinamide-PEG2000-DMG;
(25) Man-amide-PEG2000-DMG;
(26) Man-pentanoate-PEG2000-DMG;
(27) (R)-2,3-bis(stearoyloxy)propyl (2-(dimethyl(2-(4-oxo-4-((2-(((2S,3S,4S,5S,6R)-3,4,5-trihydroxy-6-(hydroxymethyl)tetrahydro-2H-pyran-2-yl)oxy)ethyl)amino)butanamido)ethyl)ammonio)ethyl) phosphate;
(28) (R)-2,3-bis(stearoyloxy)propyl (3,3-dimethyl-10,13-dioxo-16-(((2S,3S,4S,5S,6R)-3,4,5-trihydroxy-6-(hydroxymethyl)tetrahydro-2H-pyran-2-yl)oxy)-6-oxa-3,9,14-triazahexadecan-3-ium-1-yl) phosphate;
(29) Man-succinamide-PEG2000-DSG;
(30) Man-succinamide-PEG2000-DOG;
(31) Man-succinamide-PEG2000-DLG;
(32) Man-PEG2000-(10Z,29Z)-tetraconta-10,29-dien-20-yl;
(33) Man-PEG2000-(7Z,10Z,29Z,32Z)-tetraconta-7,10,29,32-tetraen-20-yl;
(34) Tri-Man-DBCO-PEG2000-DMG;
(35) Tri-Man-PEG-DBCO-PEG2000-DMG;
(36) Tri-(PEG-Man)-PEG-DBCO-PEG2000-DMG;
(37) Tri-Man-PEG2000-DMG;
(38) Tri-Man-PEG2000-DSPE;
(39) (R)-2,3-bis(stearoyloxy)propyl (2-(4-((2-(((2R,3R,4R,5S,6R)-4,5-dihydroxy-6-(hydroxymethyl)-3-(trimethylammonio)tetrahydro-2H-pyran-2-yl)oxy)ethyl)amino)-4-oxobutanamido)ethyl) phosphate;
(40) (R)-2,3-bis(stearoyloxy)propyl (2-(4-((2-(((2R,3R,4R,5R,6R)-4,5-dihydroxy-6-(hydroxymethyl)-3-(trimethylammonio)tetrahydro-2H-pyran-2-yl)oxy)ethyl)amino)-4-oxobutanamido)ethyl) phosphate;
(41) (R)-2,3-bis(stearoyloxy)propyl (2-(4-((2-(((2R,3R,4R,5S,6R)-3-(dimethylammonio)-4,5-dihydroxy-6-(hydroxymethyl)tetrahydro-2H-pyran-2-yl)oxy)ethyl)amino)-4-oxobutanamido)ethyl) phosphate;
(42) (R)-2,3-bis(stearoyloxy)propyl (2-(4-((2-(((2R,3R,4R,5R,6R)-3-(dimethylammonio)-4,5-dihydroxy-6-(hydroxymethyl)tetrahydro-2H-pyran-2-yl)oxy)ethyl)amino)-4-oxobutanamido)ethyl) phosphate;
(43) Glu-carbamate-ethylethoxy-PEG2000-DMG;
(44) GlcNAc-carbamate-ethylethoxy-PEG2000-DMG;
(45) Gal-carbamate-ethylethoxy-PEG2000-DMG;
(46) GalNAc-carbamate-ethylethoxy-PEG2000-DMG;
(47) Man-carbamate-ethylethoxy-PEG2000-DMG;
(48) Di(tris(Man)methylamide)amidoamine-PEG2000-DMG;
(49) Tris(2-(tri-Man-amidoamine)ethoxy)methylamine-PEG2000-DMG;
(50) Di(tris(Man)methylamine)thioureaethylamine-PEG2000-DMG;
(51) G1-bis-MPA-(Man-succinamide)-PEG2000-DMG;
(52) Di((di(Man-ethylamidoamine))ethyl amidoamine)ethylamine-PEG2000-DMG;
(53) Di(di((di(Man-ethylamidoamine))ethyl amidoamine)ethylamidoamine)ethylamine-PEG2000-DMG;
(54) G1-polylysine-(Man-succinamide)-ethylamine-PEG2000-DMG;
(55) G2-polylysine-(Man-succinamide)-ethylamine-PEG2000-DMG.

The lipid compounds according to the present disclosure may be beneficially used in a lipid nanoparticle composition for delivering an active substance to vertebrate cells or organs. For example, a lipid nanoparticle composition comprising the lipid compound described herein exhibits excellent safety upon *in vivo* administration and long-term stability under refrigeration and at room temperature.

### Lipid Nanoparticle Composition

In another aspect, the present disclosure provides a lipid nanoparticle composition comprising the lipid compound of Formula 1, an isomer thereof, or a salt thereof. Specifically, the lipid nanoparticle composition may include: an ionizable lipid; phospholipid 1; phospholipid 2; a structural lipid (e.g., cholesterol); PEG-lipid 1; and PEG-lipid 2.

In the present disclosure, the term "phospholipid" refers to a lipid that may include a phosphate moiety and one or more carbon chains, such as an unsaturated fatty acid chain. The phospholipid may include one or more unsaturated (e.g., double or triple) bonds. Certain phospholipids may promote membrane fusion.

The lipid nanoparticle composition according to the present disclosure may include one or more phospholipids, for example, one or more (poly)unsaturated lipids. The phospholipids may assemble into one or more lipid bilayers. Generally, the phospholipid may comprise a phospholipid moiety and one or more fatty acid moieties. The phospholipid moiety may be selected from, but is not limited to, the group consisting of, for example, phosphatidylcholine, phosphatidylethanolamine, phosphatidylglycerol, phosphatidylserine, phosphatidic acid, 2-lysophosphatidylcholine, and sphingomyelin. The fatty acid moiety may be selected from, but is not limited to, the group consisting of, for example, lauric acid, myristic acid, myristoleic acid, palmitic acid, palmitoleic acid, stearic acid, oleic acid, linoleic acid, alpha-linolenic acid, erucic acid, phytanic acid, arachidic acid, arachidonic acid, eicosapentaenoic acid, behenic acid, docosapentaenoic acid, and docosahexaenoic acid.

The present disclosure may provide a lipid nanoparticle composition comprising: ionizable lipids; the lipid compound represented by Formula 1, an isomer thereof, or a pharmaceutically acceptable salt thereof as phospholipid 1; phospholipid 2; structural lipids; the lipid compound represented by Formula 1, an isomer thereof, or a pharmaceutically acceptable salt thereof as PEG-lipid 1; and PEG-lipid 2.

The ionizable lipid plays a role in promoting formation of a core structure with an active substance, and enhancing intracellular delivery efficiency and endosomal escape after endocytosis. For example, the ionizable lipid may be selected from a group consist of (6*Z*,9*Z*,28*Z*,31*Z*)-heptatriaconta-6,9,28,31-tetraen-19-yl 4-(dimethylamino)butanoate (DLin-MC3-DMA), [(4-hydroxybutyl)azanediyl]di(hexane-6,1-diyl) bis(2-hexyldecanoate) (ALC-0315), 8-[(2-hydroxyethyl)[6-oxo-6-(undecyloxy)hexyl]amino]octanoic acid (SM-102), 1-linoleoyl-2-linoleyloxy-3-dimethylaminopropane (DLin-2-DMAP), 1,2-dilinoleylcarbamoyloxy-3-dimethylaminopropane (DLin-C-DAP), 1,2-dilinoleoyl-3-dimethylaminopropane (DLin-DAP), 1,2-dilinoleyloxy-*N*,*N*-dimethylaminopropane (DLin-DMA), 2,2-dilinoleyl-4-dimethylaminomethyl-[1,3]-dioxolane (DLin-K-DMA), 2,2-dilinoleyl-4-(2-(dimethylamino)ethyl)-1,3-dioxolane (DLin-KC2-DMA), 1,2-dioleoyl-3-dimethylammonium propane (DODAP), *N*,*N*-dimethyl-(2,3-dioleyloxy)propylamine (DODMA), dioctadecylamidoglycylcarboxyspermine (DOGS), spermine cholesteryl carbamate (GL-67), bis-guanidinium-spermidine-cholesterol (BGTC), 3β-(*N*-(*N*',*N*'-dimethylaminoethane)carbamoyl)cholesterol (DC-Chol), 1,1'-(2-(4-(2-((2-(bis(2-hydroxydecyl)amino)ethyl)(2-hydroxydecyl)amino)ethyl)piperazin-1-yl)ethylazanediyl)didodecan-2-ol (C12-200), *N*-*tert*-butyl-*N*'-tetradecylaminopropionamidine (diC14-amidine), dimethyldioctadecylammonium bromide (DDAB), *N*-(1,2-dimyristyloxyprop-3-yl)-*N*,*N*-dimethyl-*N-*hydroxyethylammonium bromide (DMRIE), *N*,*N*-dioleyl-*N*,*N*-dimethylammonium chloride (DODAC), dioleyloxypropyl-3-dimethylhydroxyethylammonium bromide (DORIE), *N*-(1-(2,3-dioleyloxy)propyl)-*N*-(2-(sperminecarboxamido)ethyl)-*N*,*N-*dimethylammonium trifluoroacetate (DOSPA), 1,2-dioleoyltrimethylammonium propane chloride (DOTAP), *N*-(1-(2,3-dioleyloxy)propyl)-*N*,*N*,*N*-trimethylammonium chloride (DOTMA), aminopropyl-dimethyl-bis(dodecyloxy)propanaminium bromide (GAP-DLRIE), and the like, and may be used alone or in combination of two or more thereof.

The structural lipid may be selected from, but is not limited to, the group consisting of, for example, cholesterol, fecosterol, sitosterol, ergosterol, campesterol, stigmasterol, brassicasterol, tomatidine, tomatine, ursolic acid, alpha-tocopherol, and mixtures thereof. In some embodiments, the structural lipids are cholesterol. In some embodiments, the structural lipid includes, but is not limited to, cholesterol and corticosteroids (e.g., prednisolone, dexamethasone, prednisone, and hydrocortisone), or a combination thereof.

Phospholipid 1 may comprise the lipid compound represented by Formula 1, an isomer thereof, or a pharmaceutically acceptable salt thereof. Phospholipid 1 serves a tissue-specific delivery function by introducing targeting ligands at a terminal thereof.

As phospholipid 2, a phospholipid known in the art may be used. For example, a known phospholipid such as 1,2-dioleoyl-sn-glycero-3-phosphoethanolamine (DOPE), 1,2-distearoyl-sn-glycero-3-phosphocholine (DSPC), 1-palmitoyl-2-oleoyl-sn-glycero-3-phosphocholine (POPC), egg phosphatidylcholine (EPC), 1,2-dioleoyl-sn-glycero-3-phosphocholine (DOPC), 1,2-dipalmitoyl-sn-glycero-3-phosphocholine (DPPC), 1,2-dioleoyl-sn-glycero-3-phosphorylglycerol (DOPG), 1,2-dipalmitoyl-sn-glycero-3-phosphorylglycerol (DPPG), 1,2-distearoyl-sn-glycero-3-phosphoethanolamine (DSPE), phosphatidylethanolamine (PE), 1,2-dipalmitoyl-sn-glycero-3-phosphoethanolamine (DPPE), 1,2-dioleoyl-sn-glycero-3-phosphoethanolamine (DOPE), 1-palmitoyl-2-oleoyl-sn-glycero-3-phosphoethanolamine (POPE), 1-palmitoyl-2-oleoyl-sn-glycero-3-phosphocholine (POPC), 1,2-dioleoyl-sn-glycero-3-(phospho-L-serine) (DOPS), ceramide, sphingomyelin, and the like may be used. In the case of the phospholipid, a modified phospholipid such as Compounds **11** to **15**, **27**, **28**, and **39** to **42** of Table 1 may be used alone, or in combination with two or more additional phospholipids.

PEG-lipid 1 may comprise the lipid compound represented by Formula 1, an isomer thereof, or a pharmaceutically acceptable salt thereof. PEG-lipid 1 serves a tissue-specific delivery function by introducing targeting ligands at a terminal thereof.

As PEG-lipid 2, a PEG-lipid known in the art may be used. PEG-lipid 2 plays a role in protecting the nanoparticle by reducing recognition by the immune system and improving *in vivo* distribution. For example, the PEG-lipid may be selected from the group consisting of PEG-dilauroylglycerol, PEG-dimyristoylglycerol (PEG-DMG), PEG-dipalmitoylglycerol, PEG-distearoylglycerol (PEG-DSG), PEG-dilaurylglycamide, PEG-dimyristylglycamide, PEG-dipalmitoylglycamide, PEG-distearoylglycamide, PEG-cholesterol, PEG-DMB (3,4-ditetradecoxylbenzyl-omega-methyl-poly(ethylene glycol) ether), 1,2-distearoyl-*sn*-glycero-3-phosphoethanolamine-*N*-(methoxy(polyethylene glycol)-2000) (PEG-DSPE), 1,2-dimyristoyl-*sn*-glycero-3-phosphoethanolamine-*N-*(methoxy(polyethylene glycol)-2000) (PEG-DMPE), and the like may be used. In the case of the PEG-lipid, modified PEG-lipids such as Compounds 1 to 10, 16 to 26, 29 to 38, and 43 to 55 of Table 1 may be used alone or in combination with two or more additional PEG-lipids.

Additionally, a known stealth lipid may be further included. The stealth lipid may refer to a lipid that modulates the circulation time of the nanoparticle *in vivo* (e.g., in the blood). The stealth lipid may facilitate the formulation by reducing particle aggregation and controlling particle size. The stealth lipid used herein may adjust the pharmacokinetic properties of the LNP. Stealth lipids suitable for use in the lipid composition of the present disclosure may include, but are not limited to, lipids comprising a hydrophilic head group linked to a lipid moiety.

The lipid nanoparticle according to the present disclosure may encapsulate, within its core, an active substance such as RNA, siRNA, shRNA, rRNA, tRNA, mRNA, miRNA, saRNA, circRNA, DNA, cDNA, plasmid, DNAzyme, ribozyme, PNA, an aptamer, an antisense oligonucleotide (ASO), CRISPR, or the like.
In use as a drug delivery system for the treatment of retinal diseases, when administered via intravitreal or subretinal injection, T, T¹, T², and T³ may be preferably used as mannose (Man) or *N*-acetylglucosamine (GlcNAc).

In use as a drug delivery system for the prevention of infectious diseases, when administered via intramuscular or intranasal injection, T, T¹, T², and T³ may be preferably used as mannose (Man).
In use as a drug delivery system for the treatment of cancer and brain diseases, when administered via intravenous injection or the like may be used.

In use as a drug delivery system for the treatment of liver diseases, when administered via intravenous injection, T, T¹, T², and T³ may be preferably used as *N-*acetylgalactosamine (GalNAc).

### Method of Preparing Active Substance-LNP

In another aspect, the present disclosure provides a method for preparing the active substance-lipid nanoparticle formulation comprising the lipid compound of Formula 1. Specifically, the present disclosure provides a method of preparing the active substance-lipid nanoparticle formulation that includes mixing the organic phase comprising the lipid compound of Formula 1, an isomer thereof, or a pharmaceutically acceptable salt thereof, with the aqueous phase in which the active substances are dissolved.

The present disclosure may provide a method of preparing the active substance-lipid nanoparticle formulation that includes mixing in the organic phase in which ionizable lipids, phospholipids, cholesterol, and PEG-lipids, at a molar ratio of (20-60):(0-25):(30-60):(0-10); and preparing the aqueous phase in which the active substances are dissolved; wherein the phospholipids comprise phospholipid 1 and phospholipid 2 mixed at a molar ratio of (0.005-100):(0-99.995), the PEG-lipids comprise PEG-lipid 1 and PEG-lipid 2 mixed at a molar ratio of (0.005-100):(0-99.995), and the mass ratio of the ionizable lipid to the active substance is (2-80):1.

Specific examples of the ionizable lipids, the phospholipids, the cholesterol, the PEG-lipids, and the active substances are as described above.

### Composition for Nucleic Acid Delivery

In another aspect, the present disclosure may provide a composition for nucleic acid delivery comprising the lipid compound of Formula 1, an isomer thereof, or a pharmaceutically acceptable salt thereof.

### Drug Delivery System

In another aspect, the present disclosure provides a method for delivering the active substances (e.g., mRNA) to cells (e.g., vertebrate cells). The method of the present disclosure includes administering a lipid nanoparticle composition to a subject, wherein the administration involves contacting cells in the subject with the lipid nanoparticle composition, such that the active substance can be delivered to targeted tissue cells.

According to an embodiment, the present disclosure may provide a tissue-specific drug delivery system comprising the lipid nanoparticle composition. The tissue-specific drug delivery system according to the present disclosure enables selection of targeting tissues depending on the type of targeting ligand in Formulas 1 and 2.

In addition, the present disclosure may provide a drug delivery system for the treatment of retinal diseases, comprising the lipid nanoparticle composition. The drug delivery system for the treatment of retinal diseases may be administered via subretinal injection or intravitreal injection.

Furthermore, the present disclosure may provide a drug delivery system for the prevention of infectious diseases, comprising the lipid nanoparticle composition. The drug delivery system for the prevention of infectious diseases may be administered via intramuscular injection or intranasal injection.

In addition, the present disclosure may provide a drug delivery system for the treatment of cancer, brain diseases or liver diseases, comprising the lipid nanoparticle composition. The drug delivery system for the treatment of cancer, brain diseases or liver diseases enables the selection of the type of targeting ligand depending on the type of cancer, brain diseases, or liver diseases.

According to an embodiment, the drug delivery system for the treatment of cancer, brain diseases or liver diseases may be administered via intravenous injection, intratumoral injection, or intracerebral injection, or the like. Preferably, the drug delivery system for the treatment of cancer, brain diseases or liver diseases may be administered via intravenous injection.

### Pharmaceutically Acceptable Salt

The active substance of the present disclosure may be used in the form of a pharmaceutically acceptable salt, and as the salt, an acid addition salt formed by a pharmaceutically acceptable free acid is useful. The term "pharmaceutically acceptable salt" refers to any organic or inorganic addition salt of a base compound of the active substance that is relatively non-toxic and harmless to a patient at a concentration and does not adversely affect the beneficial efficacy of the base compound of the active substance. As the free acid for these salts, an inorganic acid and an organic acid may be used. As the inorganic acid, hydrochloric acid, bromic acid, nitric acid, sulfuric acid, perchloric acid, phosphoric acid, and the like may be used; and as the organic acid, citric acid, acetic acid, lactic acid, maleic acid, fumaric acid, gluconic acid, methanesulfonic acid, glycolic acid, succinic acid, tartaric acid, galacturonic acid, embonic acid, glutamic acid, aspartic acid, oxalic acid, (D)- or (L)-malic acid, ethanesulfonic acid, 4-toluenesulfonic acid, salicylic acid, benzoic acid, or malonic acid, and the like may be used. In addition, these salts include an alkali metal salt (e.g., a sodium salt, a potassium salt, etc.) and an alkaline earth metal salt (e.g., a calcium salt, a magnesium salt, etc.), and the like. For example, the acid addition salt may include acetate, aspartate, benzoate, besylate, bicarbonate/carbonate, bisulfate/sulfate, borate, camsylate, citrate, edisylate, esylate, formate, fumarate, gluceptate, gluconate, glucuronate, hexafluorophosphate, hibenzate, hydrochloride/chloride, hydrobromide/bromide, hydroiodide/iodide, isethionate, lactate, malate, maleate, malonate, mesylate, methyl sulfate, naphthylate, 2-napsylate, nicotinate, nitrate, orotate, oxalate, palmitate, pamoate, phosphate/hydrogen phosphate/dihydrogen phosphate, saccharate, stearate, succinate, tartrate, tosylate, trifluoroacetate, aluminum, arginine, benzathine, calcium, choline, diethylamine, diolamine, glycine, lysine, magnesium, meglumine, olamine, potassium, sodium, tromethamine, a zinc salt, and the like, among which a hydrochloride or a trifluoroacetate is preferable.

The acid addition salt according to the present disclosure may be prepared by conventional methods, for example, by dissolving the active substance in an organic solvent such as methanol, ethanol, acetone, methylene chloride, acetonitrile, or the like, followed by addition of an organic acid or an inorganic acid, and isolating the precipitate by filtration and drying. The salts may be prepared by removing the solvent and excess acid under reduced pressure and then drying, or by crystallization in the organic solvent.

In addition, a pharmaceutically acceptable metal salt may be prepared using bases. The alkali metal or alkaline earth metal salt is obtained, for example, by dissolving the compound in an excess amount of an alkali metal hydroxide or alkaline earth metal hydroxide solution, filtering the insoluble salt, and evaporating and drying the filtrate. Here, it is pharmaceutically suitable to prepare sodium, potassium, or calcium salts as the metal salt. In addition, corresponding silver salts may be obtained by reacting the alkali metal or alkaline earth metal salt with a suitable silver salt (e.g., silver nitrate).

Furthermore, the present disclosure encompasses not only the active substance and the pharmaceutically acceptable salt thereof, but also all possible solvates, hydrates, isomers, optical isomers, and the like that may be derived therefrom.

### Routes and Methods of Administration

The pharmaceutical formulations described herein may be administered to a subject via various routes of administration, including, but not limited to, oral, parenteral (e.g., intravenous, subcutaneous, intramuscular, intraspinal injection, intrathecal, direct intraventricular, intraperitoneal, intralymphatic, and intranasal injection), buccal, topical, or transdermal routes. The pharmaceutical formulations described herein include, but are not limited to, an aqueous liquid dispersion, a self-emulsifying dispersion, a solid solution, a liposomal dispersion, an aerosol, a solid dosage form, a powder, an immediate-release formulation, a controlled-release formulation, a fast-melting formulation, a tablet, a capsule, a pill, a delayed-release formulation, an extended-release formulation, a pulsatile-release formulation, a multiparticulate formulation, and a mixed immediate-release and controlled-release formulation.

In one aspect, disclosed herein is a method for the treatment a disease or condition in a vertebrate, the method including administering a therapeutically effective dose of the pharmaceutical composition described herein. In an embodiment, the disease or condition may be treated by administering a payload.

An appropriate or effective dose for administration depends on individual subject parameters including the specific condition being treated, the severity of the condition, age, physical condition, size, sex, and weight, the duration of the treatment, the nature of concurrent therapy (if any), the specific route of administration, and similar factors within the knowledge and expertise of a healthcare practitioner, as will be recognized by those skilled in the art. Factors involved in determining the dosage are known to those skilled in the art without undue experimentation other than routine testing. Generally, it is desirable to use a maximum dose of the individual components or combinations thereof, that is, the safest dose according to sound medical judgment. Empirical considerations, such as half-life, will generally contribute to determining the dosage.

The frequency of administration may be determined and adjusted over the course of the therapy, and is generally, but not necessarily, based on the treatment and/or suppression and/or amelioration and/or delay of the disease. In some embodiments, the dosage is daily, every other day, every 3 days, every 4 days, every 5 days, or every 6 days. In some embodiments, the frequency of administration is once every 1 week, every 2 weeks, every 4 weeks, every 5 weeks, every 6 weeks, every 7 weeks, every 8 weeks, every 9 weeks, or every 10 weeks; or once every 1 month, every 2 months, or every 3 months or more. The progress of such therapy may be easily monitored by conventional techniques and assays.

A dosing regimen may vary over time. In some embodiments, for an adult subject of normal weight, a dose ranging from about 0.01 to 1000 mg/kg may be administered. In some embodiments, the dose is 1 to 200 mg. In some embodiments, the dose may range from about 0.01 to 0.05 mg/kg, about 0.01 to 0.1 mg/kg, about 0.01 to 1 mg/kg, about 0.01 to 10 mg/kg, about 0.01 to 100 mg/kg, 0.01 to 500 mg/kg, about 0.1 to 1 mg/kg, about 0.1 to 5 mg/kg, about 0.1 to 10 mg/kg, about 0.1 to 100 mg/kg, about 0.1 to 500 mg/kg, about 0.1 to 1000 mg/kg, about 1 to 5 mg/kg, about 1 to 10 mg/kg, about 1 to 100 mg/kg, about 1 to 500 mg/kg, about 1 to 1000 mg/kg, about 10 to 100 mg/kg, about 10 to 500 mg/kg, about 10 to 1000 mg/kg, or about 100 to 1000 mg/kg.

As will be apparent to those skilled in the art, an appropriate dosage of the therapeutic agent described herein will depend on the specific agent (or a composition thereof) used, the formulation and route of administration, the type and severity of the disease, whether it is administered for prophylactic or therapeutic purposes, prior therapy, the clinical history of the subject and response to an antagonist, and the judgment of an attending physician.

The present disclosure provides a method for preparing the compounds.

Specifically, the compound of Formula 1 may be prepared by a method according to the following examples, but is not limited to being prepared by such a method. In particular, those skilled in the art will fully understand that the compound of Formula 1 of the present disclosure may be prepared by various methods using well-known techniques in the art. The following examples show methods for preparing representative compounds according to the present disclosure step by step, and various compounds of the present disclosure may be prepared by altering reagents and solvents used in the following preparation steps or by changing a reaction sequence.

Hereinafter, the present disclosure will be described in more detail by the following examples. However, the following examples are merely to illustrate the present disclosure, and the scope of the present disclosure is not limited by the following examples.

### Example 1. Preparation of Glu-DBCO-PEG2000-DSPE (Compound 1)

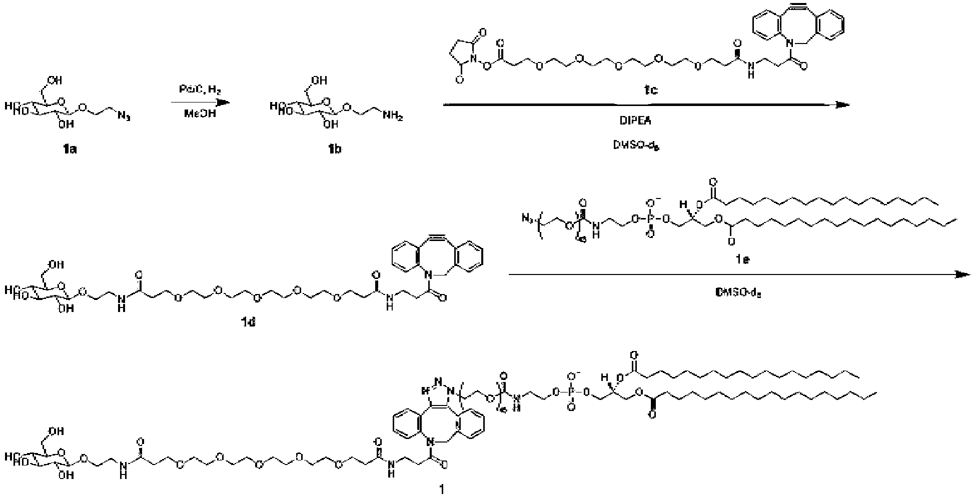

### Step 1: Synthesis of 2-aminoethyl-beta-D-glucopyranoside (Compound 1b)

2-Azidoethyl-beta-D-glucopyranoside (purchased from Synthose, 46.5 mg, 0.177 mmol) was dissolved in CH₃OH (1.00 mL) in an oven-dried 10 mL flask, and Pd/C was added. The reaction mixture was stirred at room temperature for 2 h under an H₂ atmosphere. The reaction mixture was then filtered through a Celite pad, and the filtrate was concentrated under reduced pressure. The resulting product was dried under high vacuum to give 2-aminoethyl-beta-D-glucopyranoside (Compound **1b**, 45.5 mg, 0.203 mmol, 100%) as a white foam. Compound **1b** was used in the next step without further purification.

¹H NMR (600 MHz, D₂O): δ 4.88 (d, *J* = 8.0 Hz, 1H), 3.98 (ddd, *J* = 10.9, 5.9, 4.3 Hz, 1H), 3.92 (dd, *J* = 12.3, 2.3 Hz, 1H), 3.76 (ddd, *J* = 10.7, 6.4, 4.5 Hz, 1H), 3.72 (dd, *J* = 12.3, 6.0 Hz, 1H), 3.50 (dd, *J* = 10.3, 9.3 Hz, 1H), 3.46 (dt, *J* = 5.7, 2.3 Hz, 1H), 3.38 (dd, *J* = 10.4, 9.5 Hz, 1H), 3.30 (dd, *J* = 9.4, 8.0 Hz, 1H), 2.93 (td, *J* = 5.7, 4.3 Hz, 2H); ¹³C NMR (151 MHz, D₂O): δ 102.28, 75.90, 75.62, 73.09, 69.59, 60.66, 39.88; HRMS (ESI) m/z: [M + H]⁺ calcd for C₈H₁₈NO₆ 224.1134; found 224.1132.

### Step 2: Synthesis of Glu-PEG5-DBCO (Compound 1d)

2-Aminoethyl-beta-D-glucopyranoside (Compound **1b**, 11.0 mg, 46.9 µmol) and DBCO-PEG5-N-hydroxysuccinimidyl (NHS) ester (purchased from Click Chemistry Tools, Compound **1c**, 29.9 mg, 42.3 µmol) were dissolved in DMSO-*d*₆ (0.50 mL) in an oven-dried 10 mL flask, and DIPEA (0.06 mL) was then added. The reaction mixture was stirred at room temperature for 5 h and concentrated under reduced pressure. The residue was purified by reverse-phase C18-silica gel chromatography (CH₃OH/H₂O, 65:35) to give Glu-PEG5-DBCO (Compound **1d**, 11.3 mg, 14.0 µmol, 33%) as a pale orange liquid.

¹H NMR (600 MHz, DMSO-*d*₆): δ 7.84 (t, *J* = 5.6 Hz, 1H), 7.68 (t, *J* = 5.7 Hz, 1H), 7.63 (d, *J* = 7.5 Hz, 1H), 7.56 (m, 1H), 7.54-7.43 (m, 3H), 5.04 (d, *J* = 14.1 Hz, 1H), 4.99 (d, *J* = 4.3 Hz, 1H), 4.96 (d, *J* = 4.9 Hz, 1H), 4.92 (d, *J* = 5.3 Hz, 1H), 4.54 (t, *J* = 5.8 Hz, 1H), 4.12 (d, *J* = 7.8 Hz, 1H), 3.77-3.65 (m, 2H), 3.63 (d, *J* = 14.1 Hz, 1H), 3.58 (t, *J* = 6.5 Hz, 1H), 3.54-3.37 (m, 19H), 3.30-3.18 (m, 1H), 3.18-3.06 (m, 2H), 3.06-3.00 (m, 1H), 3.00-2.86 (m, 2H), 2.44 (ddd, *J* = 15.8, 8.5, 5.8 Hz, 1H), 2.32 (t, *J* = 6.5 Hz, 2H), 2.17 (t, *J* = 6.5 Hz, 2H), 1.81 (ddd, *J* = 15.1, 8.5, 5.8 Hz, 1H); ¹³C NMR (151 MHz, DMSO-*d*₆): δ 170.62, 170.35, 151.87, 148.84, 132.86, 130.03, 129.44, 128.70, 128.54, 128.21, 127.28, 125.69, 122.94, 121.88, 114.76, 108.55, 103.62, 77.36, 77.00, 73.92, 70.51, 70.22, 70.13, 70.09, 69.99, 69.92, 68.39, 67.25, 67.14, 61.54, 55.30, 39.56, 39.20, 36.52, 36.41, 35.41, 34.64; HRMS (ESI) m/z: [M + H]⁺ calcd for C₄₀H₅₆N₃O₁₄ 802.3762; found 802.3752.

### Step 3: Synthesis of Glu-DBCO-PEG2000-DSPE (Compound 1)

DSPE-PEG2000-azide (purchased from Avanti Polar Lipids, Compound **1e**, 5.32 mg, 1.85 µmol) was dissolved in DMSO-*d*₆ (0.15 mL) in an oven-dried 10 mL flask. Glu-PEG5-DBCO (Compound **1d**, 6.28 mg, 7.44 µmol) was separately dissolved in DMSO-*d*₆ (0.10 mL) and added to the reaction mixture. The reaction mixture was stirred at room temperature for 5 h, and then an additional portion of Glu-PEG5-DBCO (Compound **1d**, 2.36 mg, 2.80 µmol) in DMSO-*d*₆ (0.10 mL) was added. The reaction mixture was stirred at room temperature for 18 h and concentrated under reduced pressure. The residue was purified by preparative size-exclusion chromatography (SEC) (Sephadex LH-20, GE Healthcare, H 26 cm × O.D. 1 cm) using CH₃OH as the eluent to give Glu-DBCO-PEG2000-DSPE (Compound **1**, 5.71 mg, 1.57 µmol, 85%) as a white solid.

¹H NMR (600 MHz, DMSO-*d*₆): δ 7.91-7.80 (m, 2H), 7.72-7.19 (m, 10H), 5.91 (d, *J* = 17.3 Hz, 1H), 5.84 (d, *J* = 16.6 Hz, 1H), 5.05 (m, 1H), 4.85 (d, *J* = 3.9 Hz, 1H), 4.72 (d, *J* = 5.3 Hz, 1H), 4.60 (q, *J* = 7.0 Hz, 1H), 4.64-4.39 (m, 4H), 4.16-3.96 (m, 5H), 3.91-2.77 (m, 318H), 2.37-2.13 (m, 6H), 1.97-1.79 (m, 1H), 1.49 (m, 5H), 1.23 (s, 56H), 0.85 (t, *J* = 6.9 Hz, 6H); MS (MALDI-TOF, DHB matrix) m/z: [M]⁻ calcd for C₁₇₂H₃₁₅N₇O₆₈P 3598.1; found 3598.1.

### Example 2. Preparation of GlcNAc-DBCO-PEG2000-DSPE (Compound 2)

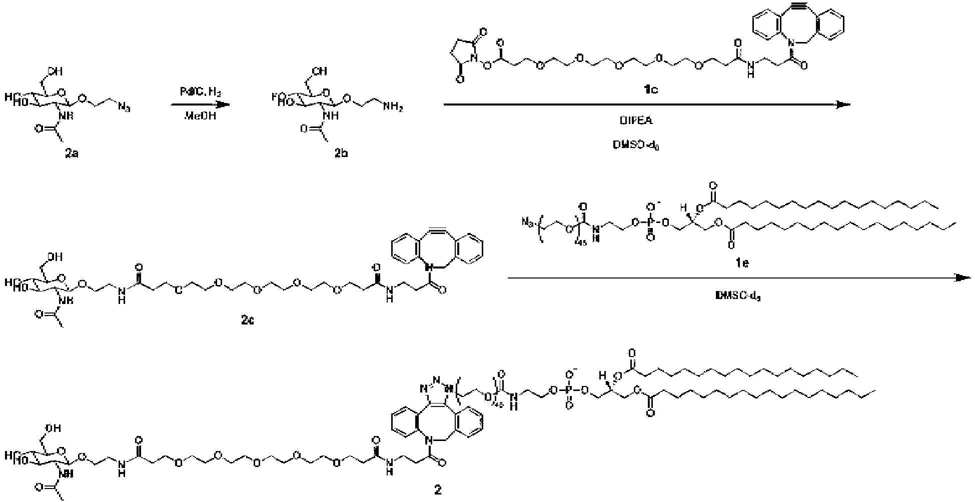

### Step 1: Synthesis of 2-aminoethyl 2-acetamido-2-deoxy-beta-D-glucopyranoside (Compound 2b)

2-Azidoethyl 2-acetamido-2-deoxy-beta-D-glucopyranoside (purchased from Synthose, 39.2 mg, 0.128 mmol) was dissolved in CH₃OH (1.00 mL) in an oven-dried 10 mL flask, and Pd/C was added. The reaction mixture was stirred at room temperature for 2 h under an H₂ atmosphere. The reaction mixture was then filtered through a Celite pad, and the filtrate was concentrated under reduced pressure. The resulting product was dried under high vacuum to give 2-aminoethyl 2-acetamido-2-deoxy-beta-D-glucopyranoside (Compound **2b**, 37.4 mg, 0.141 mmol, 100%) as a white foam. Compound **2b** was used in the next step without further purification.

¹H NMR (600 MHz, D₂O): δ 4.32 (d, *J* = 8.0 Hz, 1H), 3.82-3.77 (m, 2H), 3.63-3.56 (m, 2H), 3.50 (dd, *J* = 10.3, 9.3 Hz, 1H), 3.38 (dd, *J* = 9.4, 8.0 Hz, 1H), 3.26-3.18 (m, 2H), 2.70 (td, *J* = 5.7, 4.3 Hz, 2H), 1.91 (s, 3H); ¹³C NMR (151 MHz, D₂O): δ 174.76, 101.29, 75.82, 74.74, 73.69, 69.85, 60.66, 55.54, 39.88, 22.10; HRMS (ESI) m/z: [M + H]⁺ calcd for C₁₀H₂₁N₂O₆ 265.1400; found 265.1398.

### Step 2: Synthesis of GlcNAc-PEG5-DBCO (Compound 2c)

2-Aminoethyl 2-acetamido-2-deoxy-beta-D-glucopyranoside (Compound **2b**, 14.6 mg, 52.5 µmol) and DBCO-PEG5-N-hydroxysuccinimidyl (NHS) ester (Compound **1c**, 30.2 mg, 42.7 µmol) were dissolved in DMSO-*d*₆ (0.50 mL) in an oven-dried 10 mL flask, and then DIPEA (0.06 mL) was added. The reaction mixture was stirred at room temperature for 5 h and concentrated under reduced pressure. The residue was purified by reverse-phase C18 silica gel chromatography (CH₃OH/H₂O, 65:35) to give GlcNAc-PEG5-DBCO (Compound **2c**, 14.6 mg, 17.5 µmol, 41%) as a pale orange liquid.

¹H NMR (600 MHz, DMSO-*d*₆): δ 7.73 (t, *J* = 5.6 Hz, 1H), 7.68 (d, *J* = 8.4 Hz, 2H), 7.63 (d, *J* = 7.5 Hz, 1H), 7.61-7.56 (m, 1H), 7.53-7.28 (m, 7H), 5.04 (d, *J* = 14.1 Hz, 1H), 5.03 (br s, 1H), 4.98 (br s, 1H), 4.59 (br s, 1H), 4.29 (d, *J* = 8.4 Hz, 1H), 3.75-3.53 (m, 6H), 3.52-3.01 (m, 36H), 2.93 (m, 1H), 2.43 (m, 1H), 2.31 (t, *J* = 6.5 Hz, 2H), 2.16 (t, *J* = 6.5 Hz, 2H), 1.82 (m, 1H), 1.81 (s, 3H); ¹³C NMR (151 MHz, DMSO-*d*₆): δ 170.61, 170.34, 169.76, 151.87, 148.84, 132.85, 130.02, 129.43, 128.69, 128.53, 128.20, 127.27, 125.68, 122.93, 121.88, 114.75, 108.54, 101.55, 77.52, 74.86, 71.08, 70.21, 70.11, 70.08, 69.97, 69.91, 67.58, 67.21, 67.14, 61.55, 55.85, 55.30, 40.54, 39.63, 39.15, 36.47, 36.41, 35.41, 34.63, 23.54; HRMS (ESI) m/z: [M + H]⁺ calcd for C₄₂H₅₉N₄O₁₄ 843.4028; found 843.4005.

### Step 3: Synthesis of GlcNAc-DBCO-PEG2000-DSPE (Compound 2)

DSPE-PEG2000-azide (purchased from Avanti Polar Lipids, Compound **1e**, 5.32 mg, 1.85 µmol) was dissolved in DMSO-*d*₆ (0.15 mL) in an oven-dried 10 mL flask. GlcNAc-PEG5-DBCO (Compound **2c**, 9.36 mg, 10.5 µmol) was separately dissolved in DMSO-*d*₆ (0.10 mL) and added to the reaction mixture. The reaction mixture was stirred at room temperature for 5 h, and then an additional portion of GlcNAc-PEG5-DBCO (Compound **2c**, 1.76 mg, 2.03 µmol) was added. The reaction mixture was stirred at room temperature for 18 h and concentrated under reduced pressure. The residue was purified by preparative SEC (Sephadex LH-20, GE Healthcare, H 26 cm × O.D. 1 cm) using CH₃OH as the eluent to give GlcNAc-DBCO-PEG2000-DSPE (Compound **2**, 6.78 mg, 1.85 µmol, 79%) as a white solid.

¹H NMR (600 MHz, DMSO-*d*₆): δ 7.98-7.11 (m, 12H), 5.91 (d, *J* = 17.3 Hz, 1H), 5.84 (d, *J* = 16.6 Hz, 1H), 5.04 (m, 2H), 4.98 (m, 1H), 4.59 (m, 1H), 4.48 (td, *J* = 16.2, 7.2 Hz, 2H), 4.41-4.22 (m, 3H), 4.14-3.96 (m, 4H), 3.87-2.82 (m, 253H), 2.26 (m, 8H), 2.01-1.80 (m, 1H), 1.81 (s, 3H), 1.50 (m, 5H), 1.38-1.04 (s, 60H), 0.85 (t, *J* = 6.9 Hz, 6H); MS (MALDI-TOF, DHB matrix) m/z: [M]⁻ calcd for C₁₇₄H₃₁₈N₈O₆₈P 3639.1; found 3639.0.

### Example 3. Preparation of Gal-DBCO-PEG2000-DSPE (Compound 3)

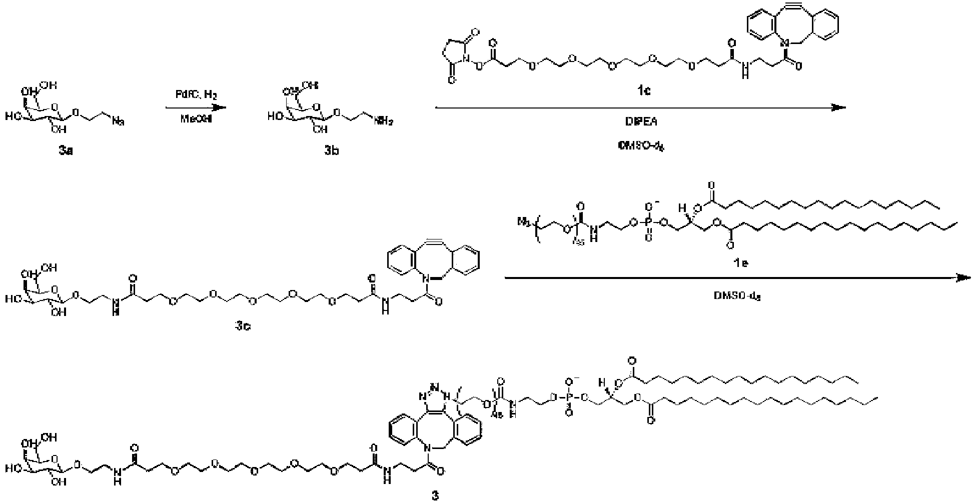

### Step 1: Synthesis of 2-aminoethyl-beta-D-galactopyranoside (Compound 3b)

2-Azidoethyl-beta-D-galactopyranoside (purchased from Synthose, 22.3 mg, 85.1 µmol) was dissolved in CH₃OH (1.00 mL) in an oven-dried 10 mL flask, and Pd/C was added. The reaction mixture was stirred at room temperature for 2 h under an H₂ atmosphere. The reaction mixture was then filtered through a Celite pad, and the filtrate was concentrated under reduced pressure. The resulting product was dried under high vacuum to give 2-aminoethyl-beta-D-galactopyranoside (Compound **3b**, 20.1 mg, 90.1 µmol, 100%) as a white foam. Compound **3b** was used in the next step without further purification.

¹H NMR (600 MHz, D₂O): δ 4.32 (d, *J* = 7.8 Hz, 1H), 3.89 (ddd, *J* = 10.9, 5.9, 4.3 Hz, 1H), 3.84 (d, *J* = 3.4 Hz, 1H), 3.74-3.63 (m, 3H), 3.61 (dt, *J* = 7.4, 4.3 Hz, 1H), 3.57 (dd, *J* = 10.0, 3.4 Hz, 1H), 3.45 (dd, *J* = 9.9, 7.8 Hz, 1H), 2.84 (m, 2H); ¹³C NMR (151 MHz, D₂O): δ 102.89, 75.15, 72.66, 70.79, 70.28, 68.61, 60.97, 40.01; HRMS (ESI) m/z: [M + H]⁺ calcd for C₈H₁₈NO₆ 224.1134; found 224.1131.

### Step 2: Synthesis of Gal-PEG5-DBCO (Compound 3c)

2-Aminoethyl-beta-D-galactopyranoside (Compound **3b**, 11.8 mg, 50.1 µmol) and DBCO-PEG5-N-hydroxysuccinimidyl (NHS) ester (purchased from Click Chemistry Tools, Compound **1c**, 29.9 mg, 42.3 µmol) were dissolved in DMSO-*d*₆ (0.50 mL) in an oven-dried 10 mL flask, and then DIPEA (0.06 mL) was added. The reaction mixture was stirred at room temperature for 5 h and concentrated under reduced pressure. The residue was purified by reverse-phase C18 silica gel chromatography (CH₃OH/H₂O, 65:35) to give Gal-PEG5-DBCO (Compound **3c**, 13.0 mg, 16.1 µmol, 39%) as a pale orange liquid.

¹H NMR (600 MHz, DMSO-*d*₆): δ 7.84 (t, *J* = 5.6 Hz, 1H), 7.68 (t, *J* = 5.7 Hz, 1H), 7.63 (d, *J* = 7.5 Hz, 1H), 7.56 (m, 1H), 7.54-7.43 (m, 6H), 5.04 (d, *J* = 14.1 Hz, 1H), 4.85 (d, *J* = 4.3 Hz, 1H), 4.73 (d, *J* = 4.9 Hz, 1H), 4.60 (t, *J* = 5.8 Hz, 1H), 4.37 (d, *J* = 5.3 Hz, 1H), 4.07 (d, *J* = 7.8 Hz, 1H), 3.70 (dt, J = 10.1, 5.9 Hz, 2H), 3.66-3.55 (m, 4H), 3.55-3.37 (m, 21H), 3.37-3.23 (m, 3H), 3.19 (m, 1H), 3.10 (m, 1H), 2.93 (m, 1H), 2.43 (m, 1H), 2.32 (t, *J* = 6.5 Hz, 2H), 2.17 (t, *J* = 6.5 Hz, 2H), 1.81 (m, 1H); ¹³C NMR (151 MHz, DMSO-*d*₆): δ 170.62, 170.57, 170.34, 151.87, 148.84, 132.85, 130.02, 129.43, 128.69, 128.53, 128.20, 127.27, 125.68, 122.93, 121.88, 114.75, 108.54, 104.21, 75.72, 73.73, 71.04, 70.22, 70.12, 70.08, 69.99, 69.91, 68.59, 68.22, 67.24, 67.14, 60.94, 55.30, 40.54, 39.64, 39.20, 36.53, 36.41, 35.41, 34.63; HRMS (ESI) m/z: [M + H]⁺ calcd for C₄₀H₅₆N₃O₁₄ 802.3762; found 802.3749.

### Step 3: Synthesis of Gal-DBCO-PEG2000-DSPE (Compound 3)

DSPE-PEG2000-azide (purchased from Avanti Polar Lipids, Compound **1e**, 6.51 mg, 2.65 µmol) was dissolved in DMSO-*d*₆ (0.15 mL) in an oven-dried 10 mL flask. Gal-PEG5-DBCO (Compound **3c**, 8.60 mg, 10.2 µmol) was separately dissolved in DMSO-*d*₆ (0.10 mL) and added to the reaction mixture. The reaction mixture was stirred at room temperature for 5 h, and then an additional portion of Gal-PEG5-DBCO (Compound **3c**, 2.70 mg, 3.19 µmol) was added. The reaction mixture was stirred at room temperature for 18 h and concentrated under reduced pressure. The residue was purified by preparative SEC (Sephadex LH-20, GE Healthcare, H 26 cm × O.D. 1 cm) using CH₃OH as the eluent to give Gal-DBCO-PEG2000-DSPE (Compound **3**, 7.01 mg, 2.25 µmol, 85%) as a white solid.

¹H NMR (600 MHz, DMSO-*d*₆): δ 8.10-7.75 (m, 1H), 7.76-7.03 (m, 11H), 5.91 (d, *J* = 16.9 Hz, 1H), 5.84 (d, *J* = 16.9 Hz, 1H), 5.03 (m, 1H), 4.85 (d, *J* = 3.9 Hz, 1H), 4.72 (d, *J* = 5.3 Hz, 1H), 4.60 (q, *J* = 7.0 Hz, 1H), 4.48 (td, *J* = 16.5, 7.3 Hz, 1H), 4.37 (d, *J* = 4.5 Hz, 1H), 4.29 (td, *J* = 12.7, 3.5 Hz, 1H), 4.20-3.97 (m, 5H), 3.96-2.86 (m, 298H), 2.38-2.09 (m, 8H), 2.01-1.77 (m, 1H), 1.49 (m, 5H), 1.40-1.01 (m, 60H), 0.85 (t, *J* = 6.9 Hz, 6H); MS (MALDI-TOF, DHB matrix) m/z: [M]⁻ calcd for C₁₇₂H₃₁₅N₇O₆₈P 3598.1; found 3598.0.

### Example 4. Preparation of GalNAc-DBCO-PEG2000-DSPE (Compound 4)

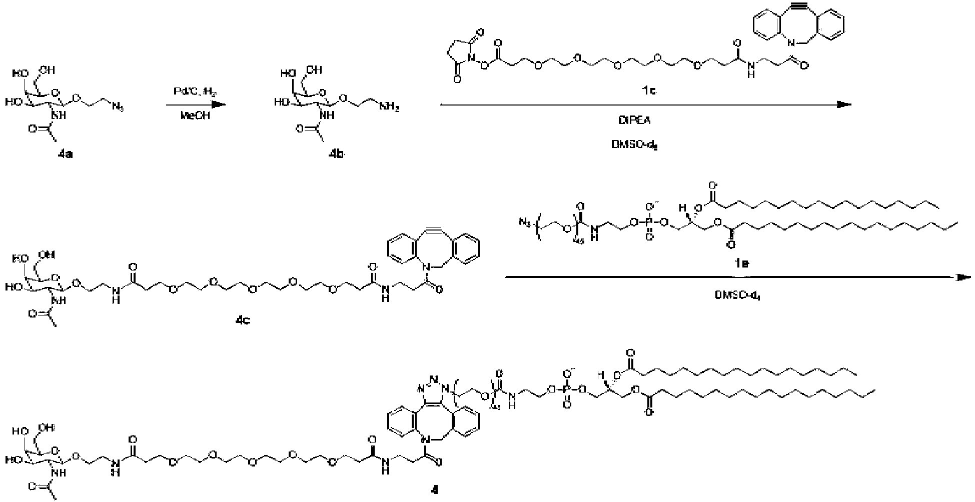

### Step 1: Synthesis of 2-aminoethyl 2-acetamido-2-deoxy-beta-D-galactopyranoside (Compound 4b)

2-Azidoethyl 2-acetamido-2-deoxy-beta-D-galactopyranoside (purchased from Synthose, 21.2 mg, 69.1 µmol) was dissolved in CH₃OH (1.00 mL) in an oven-dried 10 mL flask, and Pd/C was added. The reaction mixture was stirred at room temperature for 2 h under an H₂ atmosphere. The reaction mixture was then filtered through a Celite pad, and the filtrate was concentrated under reduced pressure. The resulting product was dried under high vacuum to give 2-aminoethyl 2-acetamido-2-deoxy-beta-D-galactopyranoside (Compound **4b**, 20.5 mg, 77.6 µmol, 100%) as a white foam. Compound **4b** was used in the next step without further purification.

¹H NMR (600 MHz, D₂O): δ 4.46 (d, *J* = 8.0 Hz, 1H), 3.94-3.90 (m, 3H), 3.84-3.65 (m, 5H), 2.91-2.70 (td, *J* = 5.7, 4.3 Hz, 2H), 2.04 (s, 3H); ¹³C NMR (151 MHz, D₂O): δ 174.91, 101.83, 75.08, 70.91, 70.62, 67.76, 60.99, 52.47, 40.00, 22.16; HRMS (ESI) m/z: [M - H]⁻ calcd for C₁₀H₁₉N₂O₆ 263.1243; found 263.1242.

### Step 2: Synthesis of GalNAc-PEG5-DBCO (Compound 4c)

2-Aminoethyl 2-acetamido-2-deoxy-beta-D-galactopyranoside (Compound **4b**, 11.5 mg, 47.8 µmol) and DBCO-PEG5-N-hydroxysuccinimidyl (NHS) ester (purchased from Click Chemistry Tools, Compound **1c**, 26.2 mg, 37.0 µmol) were dissolved in DMSO-*d*₆ (0.50 mL) in an oven-dried 10 mL flask, and DIPEA (0.06 mL) was then added. The reaction mixture was stirred at room temperature for 5 h and concentrated under reduced pressure. The residue was purified by reverse-phase C18 silica gel chromatography (CH₃OH/H₂O, 65:35) to give GalNAc-PEG5-DBCO (Compound **4c**, 8.32 mg, 7.77 µmol, 21%) as a pale orange liquid.

¹H NMR (600 MHz, DMSO-*d*₆): δ 7.75 (t, *J* = 5.6 Hz, 1H), 7.72-7.57 (m, 4H), 7.55-7.43 (m, 3H), 7.43-7.28 (m, 3H), 5.05 (d, *J* = 14.1 Hz, 1H), 4.63 (d, *J* = 4.73 Hz, 3H), 4.27 (d, *J* = 8.4 Hz, 1H), 3.71 (dt, *J* = 10.5, 8.6 Hz, 1H), 3.68-3.27 (m, 35H), 3.27-3.03 (m, 3H), 2.94 (ddt, *J* = 13.9, 8.6, 5.8 Hz, 1H), 2.44 (ddd, *J* = 15.4, 8.5, 6.3 Hz, 1H), 2.32 (t, *J* = 6.5 Hz, 2H), 2.17 (t, *J* = 6.5 Hz, 2H), 1.89-1.74 (m, 4H); ¹³C NMR (151 MHz, DMSO-*d*₆): δ 170.61, 170.60, 170.34, 170.26, 151.87, 148.84, 132.85, 130.02, 129.43, 128.69, 128.53, 128.20, 127.27, 125.68, 122.93, 121.88, 114.75, 108.54, 101.78, 75.86, 72.22, 70.22, 70.11, 70.08, 69.97, 69.91, 67.91, 67.22, 67.14, 60.96, 55.30, 52.47, 40.53, 39.80, 39.16, 36.48, 36.41, 35.41, 34.63, 23.53; HRMS (ESI) m/z: [M + H]⁺ calcd for C₄₂H₅₉N₄O₁₄ 843.4028; found 843.3998.

### Step 3: Synthesis of GalNAc-DBCO-PEG2000-DSPE (Compound 4)

DSPE-PEG2000-azide (purchased from Avanti Polar Lipids, Compound **1e**, 6.43 mg, 2.24 µmol) was dissolved in DMSO-*d*₆ (0.15 mL) in an oven-dried 10 mL flask. GalNAc-PEG5-DBCO (Compound **4c**, 6.94 mg, 7.91 µmol) was separately dissolved in DMSO-*d*₆ (0.10 mL) and added to the reaction solution. The reaction mixture was stirred at room temperature for 5 h, and then an additional portion of GalNAc-PEG5-DBCO (Compound **4c**, 1.28 mg, 1.44 µmol) was added. The reaction mixture was stirred at room temperature for 18 h and concentrated under reduced pressure. The residue was purified by preparative SEC (Sephadex LH-20, GE Healthcare, H 26 cm × O.D. 1 cm) using CH₃OH as the eluent to give GalNAc-DBCO-PEG2000-DSPE (Compound **4**, 7.56 mg, 2.06 µmol, 92%) as a white solid.

¹H NMR (600 MHz, DMSO-*d*₆): δ 7.98-7.11 (m, 12H), 5.91 (d, *J* = 17.3 Hz, 1H), 5.84 (d, *J* = 16.6 Hz, 1H), 5.04 (m, 1H), 4.69-4.37 (m, 4H), 4.41-4.22 (m, 3H), 4.14-3.96 (m, 4H), 3.87-2.82 (m, 253H), 2.35-2.15 (m, 8H), 2.01-1.80 (m, 1H), 1.81 (s, 3H), 1.62-1.40 (m, 5H), 1.23 (s, 60H), 0.85 (t, *J* = 6.9 Hz, 6H); MS (MALDI-TOF, DHB matrix) m/z: [M]⁻ calcd for C₁₇₄H₃₁₈N₈O₆₈P⁻ 3639.1; found 3639.1.

### Example 5. Preparation of Man-DBCO-PEG2000-DSPE (Compound 5)

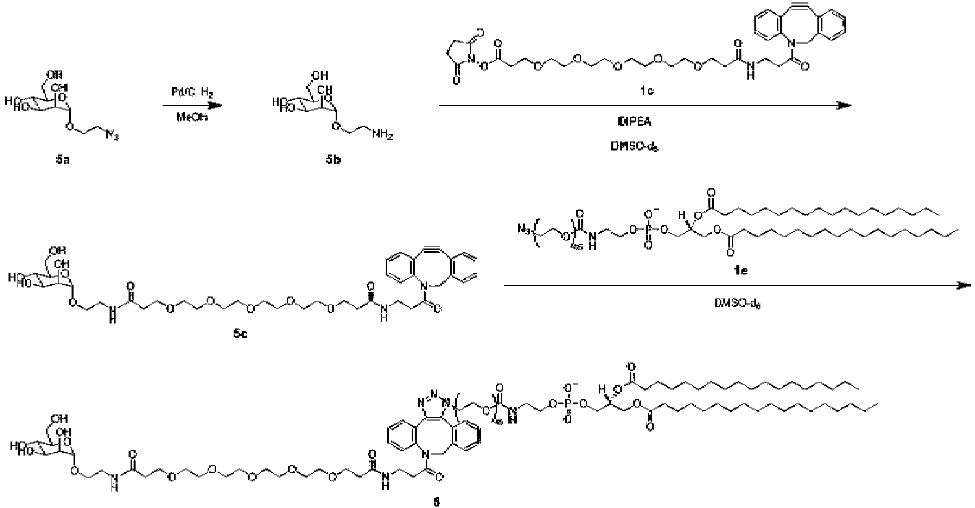

### Step 1: Synthesis of 2-aminoethyl-alpha-D-mannopyranoside (Compound 5b)

2-Azidoethyl-alpha-D-mannopyranoside (purchased from Synthose, 39.8 mg, 0.152 mmol) was dissolved in CH₃OH (1.00 mL) in an oven-dried 10 mL flask, and Pd/C was added. The reaction mixture was stirred at room temperature for 2 h under an H₂ atmosphere. The reaction mixture was then filtered through a Celite pad, and the filtrate was concentrated under reduced pressure. The resulting product was dried under high vacuum to give 2-aminoethyl-alpha-D-mannopyranoside (Compound **5b**, 37.4 mg, 0.167 mmol, 100%) as a transparent solid. Compound **5b** was used in the next step without further purification.

¹H NMR (600 MHz, D₂O): δ 4.88 (d, *J* = 1.1 Hz, 1H), 3.97 (m, 1H), 3.92-3.74 (m, 5H), 3.65 (m, 1H), 3.63 (m, 1H), 2.92-2.83 (m, 2H); ¹³C NMR (151 MHz, D₂O): δ 99.92, 72.80, 70.56, 69.97, 68.35, 66.77, 60.90, 39.83; HRMS (ESI) m/z: [M + H]⁺ calcd for C₈H₁₈NO₆ 224.1134; found 224.1136.

### Step 2: Synthesis of Man-PEG5-DBCO (Compound 5c)

2-Aminoethyl-alpha-D-mannopyranoside (Compound **5b**, 11.8 mg, 50.1 µmol) and DBCO-PEG5-N-hydroxysuccinimidyl (NHS) ester (purchased from Click Chemistry Tools, Compound **1c**, 29.2 mg, 41.2 µmol) were dissolved in DMSO-*d*₆ (0.50 mL) in an oven-dried 10 mL flask, and DIPEA (0.06 mL) was then added. The reaction mixture was stirred at room temperature for 5 h and concentrated under reduced pressure. The residue was purified by reverse-phase C18 silica gel chromatography (CH₃OH/H₂O, 65:35) to give Man-PEG5-DBCO (Compound **5c**, 13.0 mg, 16.1 µmol, 39%) as a pale orange liquid.

¹H NMR (600 MHz, DMSO-*d*₆): δ 7.89 (t, *J* = 5.6 Hz, 1H), 7.68 (t, *J* = 5.7 Hz, 1H), 7.65-7.56 (m, 2H), 7.55-7.27 (m, 6H), 5.04 (d, *J* = 14.0 Hz, 1H), 4.72 (d, *J* = 5.3 Hz, 1H), 4.70 (d, *J* = 4.2 Hz, 1H), 4.61 (d, *J* = 1.6 Hz, 1H), 4.57 (d, *J* = 5.9 Hz, 1H), 4.46 (t, *J* = 5.9 Hz, 1H), 3.79-3.52 (m, 6H), 3.52-3.14 (m, 26H), 3.10 (ddt, *J* = 14.0, 8.5, 6.1 Hz, 1H), 2.93 (ddt, *J* = 14.0, 8.5, 5.8 Hz, 1H), 2.43 (ddd, *J* = 15.3, 8.5, 6.3 Hz, 1H), 2.31 (t, *J* = 6.6 Hz, 2H), 2.17 (t, *J* = 6.5 Hz, 2H), 1.81 (ddd, *J* = 16.0, 8.5, 5.8 Hz, 1H); ¹³C NMR (151 MHz, DMSO-*d*₆): δ170.62, 170.59, 170.35, 151.87, 148.84, 132.86, 130.03, 129.44, 128.70, 128.54, 128.21, 127.28, 125.69, 122.94, 121.88, 114.76, 108.54, 100.42, 74.47, 71.37, 70.71, 70.22, 70.12, 70.09, 69.97, 69.92, 67.45, 67.27, 67.14, 65.86, 61.71, 55.30, 49.07, 38.87, 36.51, 36.41, 35.41, 34.64; HRMS (ESI) m/z: [M + H]⁺ calcd for C₄₀H₅₆N₃O₁₄ 802.3762; found 802.3747.

### Step 3: Synthesis of Man-DBCO-PEG2000-DSPE (Compound 5)

DSPE-PEG2000-azide (purchased from Avanti Polar Lipids, Compound **1e**, 6.31 mg, 2.20 µmol) was dissolved in DMSO-*d*₆ (0.15 mL) in an oven-dried 10 mL flask. Man-PEG5-DBCO (Compound **5c**, 8.33 mg, 9.87 µmol) was separately dissolved in DMSO-*d*₆ (0.10 mL) and added to the reaction mixture. The reaction mixture was stirred at room temperature for 5 h, and then an additional portion of Man-PEG5-DBCO (Compound **5c**, 2.58 mg, 3.05 µmol) was added. The reaction mixture was stirred at room temperature for 18 h and concentrated under reduced pressure. The residue was purified by preparative SEC (Sephadex LH-20, GE Healthcare, H 26 cm × O.D. 1 cm) using CH₃OH as the eluent to give Man-DBCO-PEG2000-DSPE (Compound **5**, 7.08 mg, 1.96 µmol, 89%) as a white solid.

¹H NMR (600 MHz, DMSO-*d*₆): δ 7.92-7.84 (m, 1H), 7.71-7.20 (m, 12H), 5.91 (d, *J* = 17.3 Hz, 1H), 5.84 (d, *J* = 16.6 Hz, 1H), 5.04 (m, 1H), 4.72 (d, *J* = 5.3 Hz, 1H), 4.69 (d, *J* = 4.4 Hz, 1H), 4.60 (d, *J* = 1.7 Hz, 1H), 4.57 (d, *J* = 5.9 Hz, 1H), 4.53-4.38 (m, 3H), 4.28 (dd, *J* = 12.0, 3.0 Hz, 1H), 4.13-3.98 (m, 4H), 3.90-3.11 (m, 268H), 3.10-2.83 (m, 4H), 2.35-2.10 (m, 8H), 1.98-1.80 (m, 1H), 1.57-1.42 (m, 5H), 1.41-1.05 (m, 60H), 0.85 (t, *J* = 7.0 Hz, 6H); MS (MALDI-TOF, DHB matrix) m/z: [M]⁻ calcd for C₁₇₂H₃₁₅N₇O₆₈P 3598.1; found 3598.0.

### Example 6. Preparation of 2-bromoethyl-beta-D-glucopyranoside (Compound 11b)

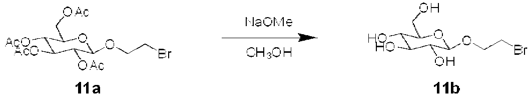

### Step 1: Synthesis of 2-bromoethyl-beta-D-glucopyranoside (Compound 11b)

2-Bromoethyl-2,3,4,6-tetra-O-acetyl-beta-D-glucopyranoside (purchased from Angene, 82.4 mg, 0.172 mmol) was dissolved in CH₃OH (1.60 mL) in an oven-dried 10 mL flask. 0.5 N sodium methoxide (NaOMe) solution (0.20 mL, 0.100 mmol) was added, and the reaction mixture was stirred for 20 min under an Ar atmosphere. The reaction mixture was passed through an Amberlite IR-120 (purchased from Sigma-Aldrich, H⁺ form) column and concentrated under reduced pressure to give 2-bromoethyl-beta-D-glucopyranoside (Compound **11b**, 48.2 mg, 0.168 mmol, 98%) as a transparent solid.

¹H NMR (400 MHz, MeOD): δ 4.33 (d, *J* = 7.7 Hz, 1H), 4.12 (ddd, *J* = 11.2, 7.0, 6.1 Hz, 1H), 3.91 (ddd, *J* = 11.2, 7.0, 6.1 Hz, 1H), 3.87 (d, *J* = 11.9 Hz, 1H), 3.66 (dd, *J* = 12.1, 3.9 Hz, 1H), 3.56 (m, 2H), 3.40-3.25 (m, 3H), 3.19 (dd, *J* = 9.0, 7.8 Hz, 1H); ¹³C NMR (101 MHz, MeOD): δ 103.12, 76.64, 76.57, 73.59, 70.14, 69.51, 61.31, 29.56; HRMS (ESI) m/z: [M + Na]⁺ calcd for C₈H₁₆BrO₆Na 308.9950; found 308.9944.

### Example 7. Preparation of Glu-DSPC (Compound 11)

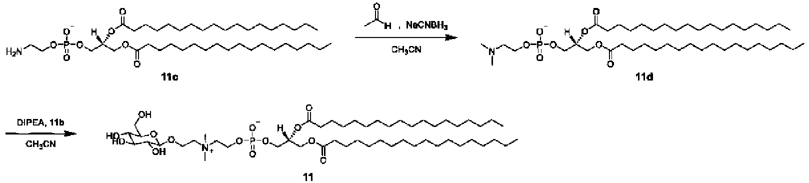

### Step 1: Synthesis of N,N-dimethylamino-DSPE (Compound 11d)

DSPE (purchased from Avanti Polar Lipids, 49.1 mg, 62.5 µmol) was dissolved in acetonitrile (0.50 mL) in an oven-dried 10 mL flask, and formaldehyde (purchased from Sigma-Aldrich, 0.45 mL) was added. The reaction mixture was stirred for 10 min at 50 °C under an Ar atmosphere. Sodium cyanoborohydride (NaCNBH₃, purchased from Sigma-Aldrich, 31.2 mg, 0.472 mmol) was added, and the reaction mixture was stirred at 50 °C for an additional 1h. The reaction mixture was filtered through a Celite pad, washed with chloroform, and concentrated under reduced pressure. The residue was recrystallized from acetone to give N,N-dimethylamino-DSPE (Compound **11d**, 34.0 mg, 43.9 µmol, 70%) as a white solid.

¹H NMR (400 MHz, CDCl₃): δ 5.21 (m, 1H), 4.37 (dd, *J* = 12.1, 3.0 Hz, 1H), 4.28-4.02 (m, 3H), 3.94 (t, *J* = 6.6 Hz, 2H), 3.01 (t, *J* = 6.6 Hz, 2H), 2.66 (s, 6H), 2.27 (t, *J* = 7.8 Hz, 4H), 1.57 (m, 4H), 1.24 (m, 56H), 0.87 (t, *J* = 6.8 Hz, 6H); ¹³C NMR (101 MHz, CDCl₃) δ 173.47, 173.17, 70.50, 63.67, 62.80, 58.45, 44.21, 34.31, 34.11, 31.93, 29.75, 29.73, 29.68, 29.61, 29.59, 29.42, 29.38, 29.23, 29.20, 24.97, 24.89, 22.69, 14.12; HRMS (ESI) m/z: [M]⁻ calcd for C₄₃H₈₅NO₈P 774.6013; found 774.6013.

### Step 2: Synthesis of Glu-DSPC (Compound 11)

Compound **11b** (21.3 mg, 70.6 µmol) and Compound **11d** (18.5 mg, 22.6 µmol) were dissolved in acetonitrile (0.50 mL) in an oven-dried 10 mL flask, and DIPEA (0.02 mL) was added. The reaction mixture was stirred at 65 °C for 42 h under an Ar atmosphere. The reaction mixture was concentrated, and the residue was recrystallized from acetone to give Glu-DSPC (Compound **11**, 4.21 mg, 4.29 µmol, 20%) as a white solid.

¹H NMR (500 MHz, CDCl₃/MeOD): δ 5.20 (m, 1H), 4.37 (dd, *J* = 12.1, 3.0 Hz, 1H), 4.32-3.79 (m, 10H), 3.68 (m, 2H), 3.43-3.01 (m, 12H), 2.29 (td, *J* = 9.2, 5.1 Hz, 4H), 1.57 (m, 4H), 1.24 (m, 56H), 0.85 (t, *J* = 6.8 Hz, 6H); HRMS (ESI) m/z: [M + H]⁺ calcd for C₅₁H₁₀₁NO₁₄P⁺ 982.6960; found 982.6962.

### Example 8. Preparation of 4-nitrophenyl carbonate-ethylethoxy-PEG2000-DMG (Compound 43g)

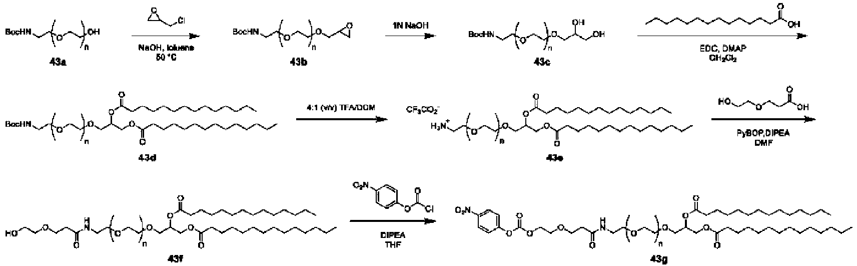

### Step 1: Synthesis of N-Boc-PEG2000-methoxy epoxide (Compound 43b)

*N*-Boc-PEG2000 (purchased from BroadPharm, 0.418 g, 0.188 mmol) was dissolved in toluene (4.00 mL) in an oven-dried 10 mL flask. Ground sodium hydroxide (NaOH) (0.230 g, 5.64 mmol) was added, followed by epichlorohydrin (0.30 mL) was added. The reaction mixture was stirred at 50 °C for 14 h under an Ar atmosphere. The reaction mixture was extracted with DCM, and then the organic layer was washed with brine, dried over anhydrous sodium sulfate, and concentrated. The residue was purified by silica gel chromatography (DCM/CH₃OH, 13:1) to give *N*-Boc-PEG2000-methoxy epoxide (Compound **43b**, 0.310 g, 0.140 mmol, 75%) as a white solid.

¹H NMR (600 MHz, CDCl₃): δ 5.08 (s, 1H), 3.91-3.04 (m, 193H), 2.82 (dd, *J* = 5.0, 4.1 Hz, 1H), 2.63 (dd, *J* = 5.1, 2.7 Hz, 1H), 1.47 (s, 9H); MS (MALDI-TOF, DHB matrix) m/z: [M + H]⁺ calcd for C₉₈H₁₉₅NO₄₈ 2154.3; found 2154.3.

### Step 2: Synthesis of N-Boc-PEG2000-dihydroxypropoxy (Compound 43c)

*N*-Boc-PEG2000-methoxy epoxide (Compound **43b**, 0.286 g, 0.127 mmol) was dissolved in H₂O (2.00 mL) in an oven-dried 10 mL flask, and 1 N NaOH (4.00 mL) was added. The reaction mixture was stirred at room temperature for 14 h. The mixture was concentrated under reduced pressure, and the residue was dissolved in a small amount of DCM and precipitated with ether. The resulting solid was collected by filtration and washed several times with diethyl ether. The product solid was dried under high vacuum to give *N*-Boc-PEG2000-dihydroxypropoxy (Compound **43c**, 0.249 g, 0.112 mmol) as a white solid. Compound **43c** was used in the next step without further purification. MS (MALDI-TOF, DHB matrix) m/z: [M + H]⁺ calcd for C₉₈H₁₉₇NO₄₉ 2172.3; found 2172.2.

### Step 3: Synthesis of N-Boc-PEG2000-DMG (Compound 43d)

*N*-Boc-PEG2000-dihydroxypropoxy (Compound **43c**, 0.248 g, 0.108 mmol), tetradecanoic acid (myristic acid, 0.202 g, 0.865 mmol), and 4-dimethylaminopyridine (DMAP, 21.5 mg, 0.161 mmol) were dissolved in DCM (1.00 mL) in an oven-dried 10 mL flask. EDC (0.169 g, 0.865 mmol) was added at 0 °C, and the reaction mixture was stirred at room temperature for 14 h. The reaction mixture was concentrated under reduced pressure, and the residue was purified by silica gel chromatography (DCM/CH₃OH, 13:1), followed by SEC (Bio-Beads S-X1, Bio-Rad, H 42 cm × O.D. 3.0 cm) using DMF as the eluent to give *N*-Boc-PEG2000-DMG (Compound **43d**, 0.133 g, 49.7 µmol, 41%) as a white solid.

¹H NMR (600 MHz, CDCl₃): δ 5.19 (m, 1H), 4.35 (dd, *J* = 11.9, 3.4 Hz, 1H), 4.15 (dd, *J* = 11.9, 6.8 Hz, 1H), 3.92-3.09 (m, 204H), 2.31 (td, *J* = 7.4, 3.9 Hz, 4H), 1.59 (m, 4H), 1.41 (s, 9H), 1.31 (m, 40H), 0.88 (m, 6H); MS (MALDI-TOF, DHB matrix) m/z: [M + H]⁺ calcd for C₁₂₆H₂₄₉NO₅₁ 2592.7; found 2592.6.

### Step 4: Synthesis of Amino-PEG2000-DMG (Compound 43e)

*N*-Boc-PEG2000-DMG (Compound **43d**, 0.132 g, 51.0 µmol) was dissolved in DCM (0.20 mL) in an oven-dried 10 mL flask, and then TFA (0.80 mL) was added. The reaction mixture was stirred at room temperature for 1 h. The reaction mixture was concentrated and purified by silica gel chromatography (DCM/MeOH/NH₄OH, 7:1:0.5), followed by SEC (Bio-Beads S-X1, Bio-Rad, H 42 cm × O.D. 3.0 cm) using DMF as the eluent to give amino-PEG2000-DMG (Compound **43e**, 85.0 mg, 34.7 µmol, 69%) as a white solid.

¹H NMR (600 MHz, CDCl₃): δ 7.94 (s, 1H), 5.23 (m, 1H), 4.36 (dd, *J* = 11.9, 3.4 Hz, 1H), 4.17 (dd, *J* = 11.9, 6.8 Hz, 1H), 3.92-3.09 (m, 183H), 2.32 (td, *J* = 7.4, 3.9 Hz, 4H), 1.63 (m, 4H), 1.28 (m, 40H), 0.90 (m, 6H); MS (MALDI-TOF, DHB matrix) m/z: [M + H]⁺ calcd for C₁₂₃H₂₄₂F₃NO₅₁ 2606.6; found 2606.5.

### Step 5: Synthesis of Hydroxy-ethylethoxy-PEG2000-DMG (Compound 43f)

Amino-PEG2000-DMG (Compound **43e**, 36.1 mg, 14.1 µmol) and hydroxy-PEG1-acid (purchased from BroadPharm, 4.42 mg, 32.0 µmol) were dissolved in DMF (1.00 mL) in an oven-dried 10 mL flask, and DIPEA (0.04 mL) was added. PyBOP (11.1 mg, 20.8 µmol) was then added, and the reaction mixture was stirred at room temperature for 15 h under an Ar atmosphere. The reaction mixture was concentrated under reduced pressure and purified by SEC (Bio-Beads S-X1, H 42 cm × O.D. 3.0 cm) using DMF as the eluent to give hydroxy-ethylethoxy-PEG2000-DMG (Compound **43f**, 33.4 mg, 13.0 µmol, 92%) as a white solid.

¹H NMR (600 MHz, DMSO-*d*₆): δ 7.89 (s, 1H), 5.10 (m, 1H), 4.56 (t, *J* = 5.5 Hz, 1H), 4.26 (dd, *J* = 12.0, 3.1 Hz, 1H), 4.08 (dd, *J* = 12.0, 7.0 Hz, 1H), 3.78-3.05 (m, 206H), 2.31 (t, *J* = 6.2 Hz, 2H), 2.25 (td, *J* = 7.4, 3.9 Hz, 4H), 1.50 (m, 4H), 1.23 (m, 40H), 0.85 (m, 6H); MS (MALDI-TOF, DHB matrix) m/z: [M + H]⁺ calcd for C₁₂₆H₂₄₉NO₅₂ 2608.7; found 2608.7.

### Step 6: Synthesis of 4-nitrophenyl carbonate-ethylethoxy-PEG2000-DMG (Compound 43g)

Hydroxy-ethylethoxy-PEG2000-DMG (Compound **43f**, 32.5 mg, 12.0 µmol) and 4-nitrophenyl chloroformate (34.5 mg, 0.164 mmol) were dissolved in anhydrous THF (1.00 mL) in an oven-dried 10 mL flask, and DIPEA (0.03 mL) was then added. The reaction mixture was stirred at room temperature for 15 h under an Ar atmosphere. The reaction mixture was concentrated under reduced pressure, and the residue was purified by SEC (Bio-Beads S-X1, H 42 cm × O.D. 3.0 cm) using DMF as the eluent to give 4-nitrophenyl carbonate-ethylethoxy-PEG2000-DMG (Compound **43g**, 29.6 mg, 10.8 µmol, 68%) as a white solid. Compound **43g** was used in the next step without further purification. MS (MALDI-TOF, DHB matrix) m/z: [M + H]⁺ calcd for C₁₃₃H₂₅₂N₂O₅₆ 2773.7; found 2773.6.

### Example 9. Preparation of Glu-carbamate-ethylethoxy-PEG2000-DMG (Compound 43)

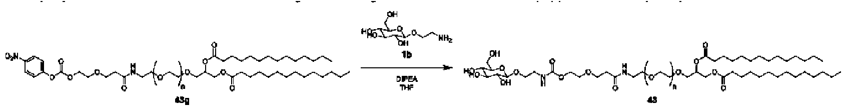

### Step 1: Synthesis of Glu-carbamate-ethylethoxy-PEG2000-DMG (Compound 43)

4-Nitrophenyl carbonate-ethylethoxy-PEG2000-DMG (Compound **43g**, 15.0 mg, 4.11 µmol) and Compound **1b** (4.06 mg, 17.3 µmol) were dissolved in DMSO-*d*₆ (0.30 mL) in an oven-dried 10 mL flask, and DIPEA (0.01 mL) was then added. The reaction mixture was stirred at room temperature for 15 h under an Ar atmosphere. The reaction mixture was concentrated under reduced pressure, and the residue was purified by silica gel chromatography (DCM/CH₃OH/H₂O, 6:1:0.1), followed by SEC (Bio-Beads S-X1, H 42 cm × O.D. 3.0 cm) using DMF as the eluent to give Glu-carbamate-ethylethoxy-PEG2000-DMG (Compound **43**, 6.28 mg, 2.23 µmol, 54%) as a white solid.

¹H NMR (600 MHz, DMSO-*d*₆): δ 7.90 (t, *J* = 5.6 Hz, 1H), 7.20 (t, *J* = 6.0 Hz, 1H), 5.10 (m, 1H), 4.99 (d, *J* = 4.1 Hz, 1H), 4.92 (d, *J* = 4.8 Hz, 1H), 4.90 (d, *J* = 5.1 Hz, 1H), 4.48 (t, *J* = 5.9 Hz, 1H), 4.26 (dd, *J* = 12.0, 3.2 Hz, 1H), 4.11 (d, *J* = 7.8 Hz, 1H), 4.08 (dd, *J* = 12.0, 7.0 Hz, 1H), 4.05-4.00 (m, 2H), 3.89-2.76 (m, 216H), 2.36-2.22 (m, 6H), 1.50 (m, 4H), 1.23 (m, 40H), 0.85 (m, 6H); MS (MALDI-TOF, DHB matrix) m/z: [M + H]⁺ calcd for C₁₃₅H₂₆₄N₂O₅₉ 2857.8; found 2857.7.

### Example 10. Preparation of GlcNAc-carbamate-ethylethoxy-PEG2000-DMG (Compound 44)

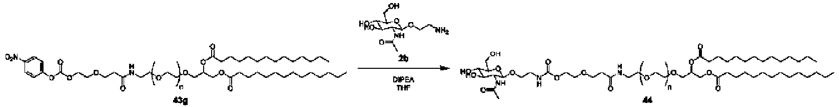

### Step 1: Synthesis of GlcNAc-carbamate-ethylethoxy-PEG2000-DMG (Compound 44)

4-Nitrophenyl carbonate-ethylethoxy-PEG2000-DMG (Compound **43g**, 14.1 mg, 3.88 µmol) and Compound **2b** (4.59 mg, 16.5 µmol) were dissolved in DMSO-*d*₆ (0.30 mL) in an oven-dried 10 mL flask, and DIPEA (0.01 mL) was then added. The reaction mixture was stirred at room temperature for 15 h under an Ar atmosphere. The reaction mixture was concentrated under reduced pressure, and the residue was purified by silica gel chromatography (DCM/CH₃OH/H₂O, 6:1:0.1), followed by SEC (Bio-Beads S-X1, H 42 cm × O.D. 3.0 cm) using DMF as the eluent to give GlcNAc-carbamate-ethylethoxy-PEG2000-DMG (Compound **44**, 4.11 mg, 1.44 µmol, 35%) as a white solid.

¹H NMR (600 MHz, DMSO-*d*₆): δ 7.91 (t, *J* = 5.6 Hz, 1H), 7.67 (d, *J* = 8.9 Hz, 1H), 6.99 (t, *J* = 6.0 Hz, 1H), 5.10 (m, 1H), 4.99 (d, *J* = 4.6 Hz, 1H), 4.92 (d, *J* = 5.3 Hz, 1H), 4.53 (t, *J* = 5.9 Hz, 1H), 4.30 (d, *J* = 8.4 Hz, 1H), 4.26 (dd, *J* = 12.0, 3.1 Hz, 1H), 4.08 (dd, *J* = 12.0, 7.0 Hz, 1H), 4.05-4.00 (m, 2H), 3.89-2.76 (m, 216H), 2.36-2.22 (m, 6H), 1.81 (s, 3H), 1.50 (m, 4H), 1.23 (m, 40H), 0.85 (m, 6H); MS (MALDI-TOF, DHB matrix) m/z: [M + H]⁺ calcd for C₁₃₇H₂₆₇N₃O₅₉ 2898.8; found 2898.7.

### Example 11. Preparation of Gal-carbamate-ethylethoxy-PEG2000-DMG (Compound 45)

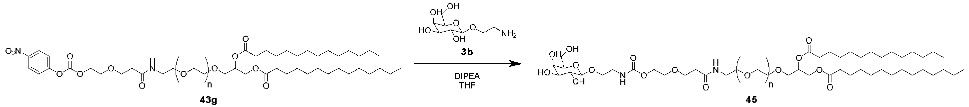

### Step 1: Synthesis of Gal-carbamate-ethylethoxy-PEG2000-DMG (Compound 45)

4-Nitrophenyl carbonate-ethylethoxy-PEG2000-DMG (Compound **43g**, 14.3 mg, 3.92 µmol) and Compound **3b** (4.41 mg, 18.7 µmol) were dissolved in DMSO-*d*₆ (0.30 mL) in an oven-dried 10 mL flask, and DIPEA (0.01 mL) was then added. The reaction mixture was stirred at room temperature for 15 h under an Ar atmosphere. The reaction mixture was concentrated under reduced pressure, and the residue was purified by silica gel chromatography (DCM/CH₃OH/H₂O, 6:1:0.1), followed by SEC (Bio-Beads S-X1, H 42 cm × O.D. 3.0 cm) using DMF as the eluent to give Gal-carbamate-ethylethoxy-PEG2000-DMG (Compound **45**, 6.15 mg, 2.19 µmol, 52%) as a white solid.

¹H NMR (600 MHz, DMSO-*d*₆): δ 7.90 (t, *J* = 5.6 Hz, 1H), 7.19 (t, *J* = 6.0 Hz, 1H), 5.10 (m, 1H), 4.86 (d, *J* = 4.1 Hz, 1H), 4.71 (d, *J* = 4.8 Hz, 1H), 4.57 (d, *J* = 5.1 Hz, 1H), 4.37 (t, *J* = 5.9 Hz, 1H), 4.26 (dd, *J* = 12.0, 3.2 Hz, 1H), 4.08 (dd, *J* = 12.0, 7.0 Hz, 1H), 4.06 (d, *J* = 7.8 Hz, 1H), 4.05-4.00 (m, 2H), 3.89-2.76 (m, 224H), 2.36-2.22 (m, 6H), 1.50 (m, 4H), 1.23 (m, 40H), 0.85 (m, 6H); MS (MALDI-TOF, DHB matrix) m/z: [M + H]⁺ calcd for C₁₃₅H₂₆₄N₂O₅₉ 2857.8; found 2857.7.

### Example 12. Preparation of GalNAc-carbamate-ethylethoxy-PEG2000-DMG (Compound 46)

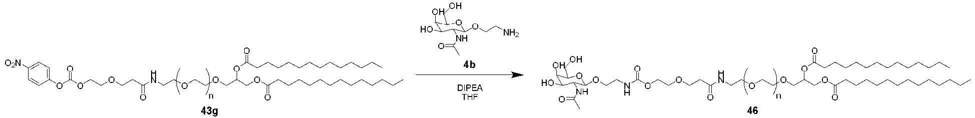

### Step 1: Synthesis of GalNAc-carbamate-ethylethoxy-PEG2000-DMG (Compound 46)

4-Nitrophenyl carbonate-ethylethoxy-PEG2000-DMG (Compound **43g**, 13.13 mg, 3.61 µmol) and Compound **4b** (4.40 mg, 15.8 µmol) were dissolved in DMSO-*d*₆ (0.30 mL) in an oven-dried 10 mL flask, and DIPEA (0.01 mL) was then added. The reaction mixture was stirred at room temperature for 15 h under an Ar atmosphere. The reaction mixture was concentrated under reduced pressure, and the residue was purified by silica gel chromatography (DCM/CH₃OH/H₂O, 6:1:0.1), followed by SEC (Bio-Beads S-X1, H 42 cm × O.D. 3.0 cm) using DMF as the eluent to give GalNAc-carbamate-ethylethoxy-PEG2000-DMG (Compound **46**, 5.39 mg, 1.89 µmol, 50%) as a white solid.

¹H NMR (600 MHz, DMSO-*d*₆): δ 7.90 (t, *J* = 5.6 Hz, 1H), 7.63 (d, *J* = 8.8 Hz, 1H), 6.98 (t, *J* = 6.0 Hz, 1H), 5.10 (m, 1H), 4.59 (s, 2H), 4.50 (s, 1H), 4.25 (d, *J* = 8.6 Hz, 1H), 4.25 (dd, *J* = 12.0, 3.1 Hz, 1H), 4.08 (dd, *J* = 12.0, 7.0 Hz, 1H), 4.05-4.00 (m, 2H), 3.89-2.76 (m, 210H), 2.36-2.22 (m, 6H), 1.81 (s, 3H), 1.50 (m, 4H), 1.23 (m, 40H), 0.85 (m, 6H); MS (MALDI-TOF, DHB matrix) m/z: [M + H]⁺ calcd for C₁₃₇H₂₆₇N₃O₅₉ 2898.8; found 2898.8.

### Example 13. Preparation of Man-carbamate-ethylethoxy-PEG2000-DMG (Compound 47)

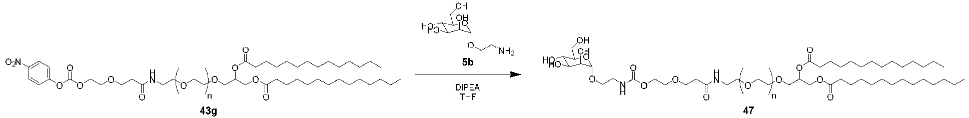

### Step 1: Synthesis of Man-carbamate-ethylethoxy-PEG2000-DMG (Compound 47)

4-Nitrophenyl carbonate-ethylethoxy-PEG2000-DMG (Compound **43g**, 14.0 mg, 3.85 µmol) and Compound **5b** (4.65 mg, 19.7 µmol) were dissolved in DMSO-*d*₆ (0.30 mL) in an oven-dried 10 mL flask, and DIPEA (0.01 mL) was then added. The reaction mixture was stirred at room temperature for 15 h under an Ar atmosphere. The reaction mixture was concentrated under reduced pressure, and the residue was purified by silica gel chromatography (DCM/CH₃OH/H₂O, 6:1:0.1), followed by SEC (Bio-Beads S-X1, H 42 cm × O.D. 3.0 cm) using DMF as the eluent to give Man-carbamate-ethylethoxy-PEG2000-DMG (Compound **47**, 6.43 mg, 2.28 µmol, 56%) as a white solid.

¹H NMR (600 MHz, DMSO-*d*₆): δ 7.90 (t, *J* = 5.6 Hz, 1H), 7.20 (t, *J* = 6.0 Hz, 1H), 5.10 (m, 1H), 4.99 (d, *J* = 4.1 Hz, 1H), 4.92 (d, *J* = 4.8 Hz, 1H), 4.90 (d, *J* = 5.1 Hz, 1H), 4.48 (t, *J* = 5.9 Hz, 1H), 4.26 (dd, *J* = 12.0, 3.2 Hz, 1H), 4.11 (d, *J* = 7.8 Hz, 1H), 4.08 (dd, *J* = 12.0, 7.0 Hz, 1H), 4.05-4.00 (m, 2H), 3.89-2.76 (m, 216H), 2.36-2.22 (m, 6H), 1.50 (m, 4H), 1.23 (m, 40H), 0.85 (m, 6H); MS (MALDI-TOF, DHB matrix) m/z: [M + H]⁺ calcd for C₁₃₅H₂₆₄N₂O₅₉ 2857.8; found 2857.8.

By applying the same method as in Example 5, Compounds **16**-**20** shown in Table 1 below were synthesized using various click chemistries.

By applying the same method as in Examples 6 and 7, Compounds **12**-**15** shown in Table 1 below were synthesized using other monosaccharide derivatives instead of compound **11a** in step 1 of Example 6.

By applying the same method as in Examples 6 and 7, Compounds **27** and **28** shown in Table 1 below were synthesized using various chemical linkages to monosaccharide derivatives.

By applying the same method as in Example 1, Compounds **39-42** shown in Table 1 below were synthesized using phospholipid 11c with a different chemical linkage instead of Compounds 1c and 1e in Example 1.

By applying the same method as in Examples 1 to 5 or 8 to 13, Compounds **6**-**10**, **21**-**26**, and **29**-**33** shown in Table 1 below were synthesized using various chemical linkages to monosaccharide derivatives and employing different PEG-lipid tail moieties.

By applying the same method as in Examples 1 to 5 or 8 to 13, Compounds **34-38** and **48**-**55** shown in Table 1 below were synthesized by employing branched targeting ligands linked via click chemistries and various chemical linkages.

In Compounds **1**-**55**, n may be an integer from 37 to 53, x may be 1 to 3, y may be 1 to 5, and z may be an integer from 1 to 12. Specifically, n is 45, x is 1, y is 2, and z is 3.

In addition, Compounds **11**-**15**, **27**, **28**, and **39**-**42** relate to modified phospholipids as the lipid compounds of Formula 1, and Compounds **1**-**10**, **16**-**26**, **29**-**38**, and **43**-**55** relate to modified PEG-lipids as the lipid compounds of Formula 1.

**[Table 1]**

| Cmpd. No. | Structure | MS (calcd.)/ MS (found) |
|---|---|---|
| | Common Name | |
| **1** | 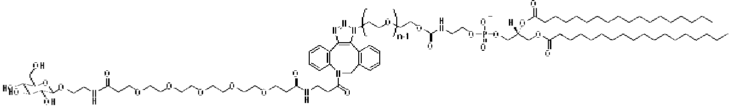 Glu-DBCO-PEG2000-DSPE | 3598.1/3598.1 |
| **2** | 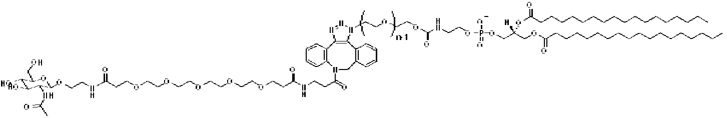 GlcNAc-DBCO-PEG2000-DSPE | 3639.1/3639.0 |
| **3** | 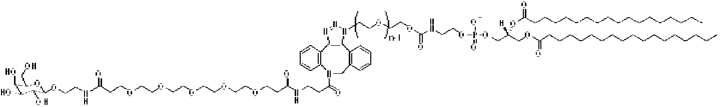 Gal-DBCO-PEG2000-DSPE | 3598.1/3598.0 |
| **4** | 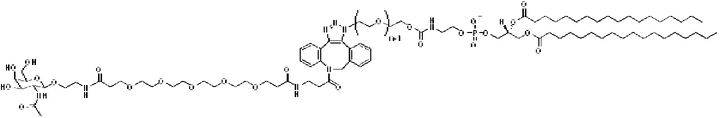 GalNAc-DBCO-PEG2000-DSPE | 3639.1/3639.1 |
| **5** | 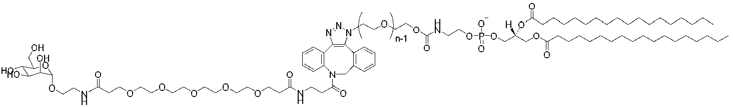 Man-DBCO-PEG2000-DSPE | 3598.1/3598.0 |
| **6** | 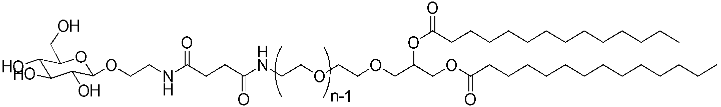 Glu-succinamide-PEG2000-DMG | 2797.8/2797.7 |
| **7** | 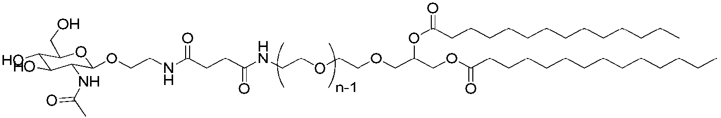 GlcNAc-succinamide-PEG2000-DMG | 2838.8/2838.8 |
| **8** | 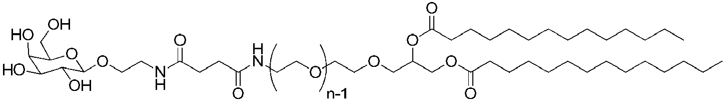 Gal-succinamide-PEG2000-DMG | 2797.8/2797.6 |
| **9** | 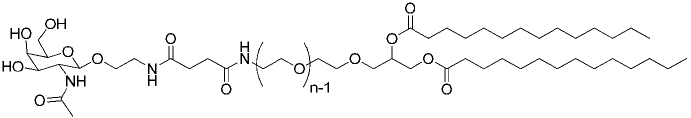 GalNAc-succinamide-PEG2000-DMG | 2838.8/2838.7 |
| **10** | 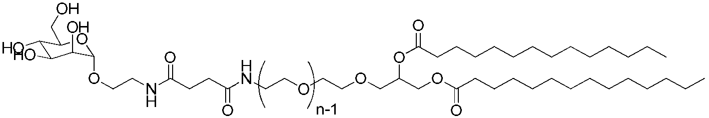 Man-succinamide-PEG2000-DMG | 2797.8/2797.6 |
| **11** | 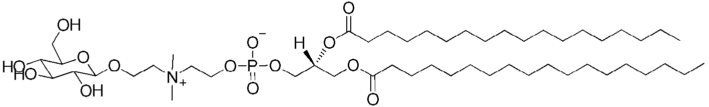 (R)-2,3-bis(stearoyloxy)propyl (2-(dimethyl(2-(((2R,3R,4S,5S,6R)-3,4,5-trihydroxy-6-(hydroxymethyl)tetrahydro-2H-pyran-2-yl)oxy)ethyl)ammonio)ethyl) phosphate | 981.7/981.6 |
| **12** | 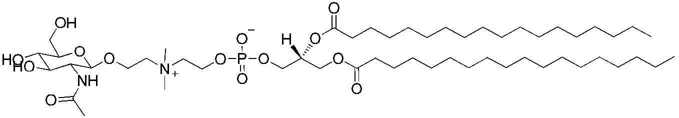 2-((2-(((2R,3R,4S,5S,6R)-3-acetamido-4,5-dihydroxy-6-(hydroxymethyl)tetrahydro-2H-pyran-2-yl)oxy)ethyl)dimethylammonio)ethyl ((R)-2,3-bis(stearoyloxy)propyl) phosphate | 1022.7/1022.8 |
| **13** | 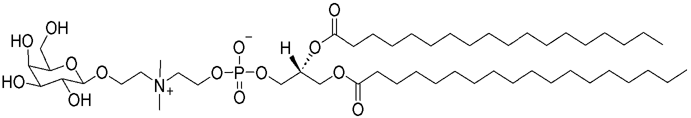 (R)-2,3-bis(stearoyloxy)propyl (2-(dimethyl(2-(((2R,3R,4S,5R,6R)-3,4,5-trihydroxy-6-(hydroxymethyl)tetrahydro-2H-pyran-2-yl)oxy)ethyl)ammonio)ethyl) phosphate | 981.7/981.6 |
| **14** | 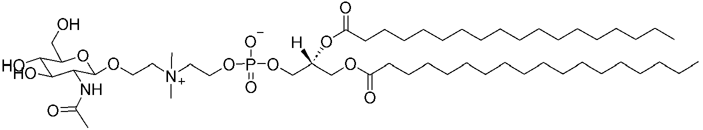 2-((2-(((2R,3R,4S,5R,6R)-3-acetamido-4,5-dihydroxy-6-(hydroxymethyl)tetrahydro-2H-pyran-2-yl)oxy)ethyl)dimethylammonio)ethyl ((R)-2,3-bis(stearoyloxy)propyl) phosphate | 1022.7/1022.8 |
| **15** | 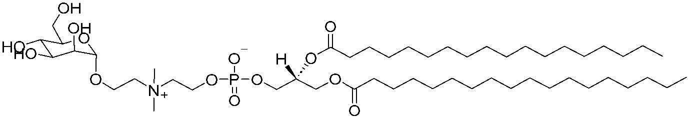 (R)-2,3-bis(stearoyloxy)propyl (2-(dimethyl(2-(((2S,3S,4S,5S,6R)-3,4,5-trihydroxy-6-(hydroxymethyl)tetrahydro-2H-pyran-2-yl)oxy)ethyl)ammonio)ethyl) phosphate | 981.7/981.6 |
| **16** | 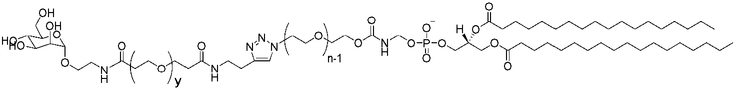 Man-1,2,3-triazole-PEG2000-DSPE | 3245.0/3245.0 |
| **17** | 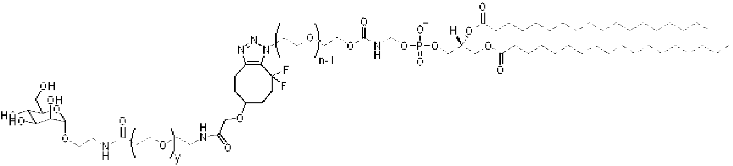 Man-DIFO-PEG2000-DSPE | 3365.0/3365.1 |
| **18** | 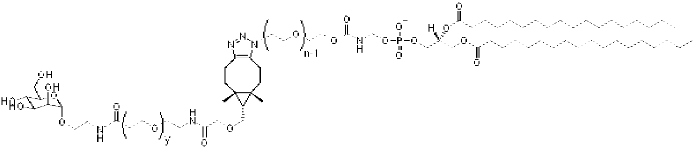 Man-BCN-PEG2000-DSPE | 3383.1/3383.0 |
| **19** | 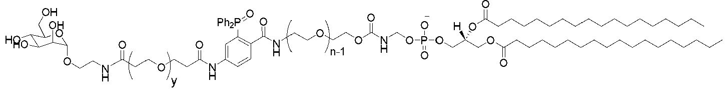 Man-triarylphosphine-PEG2000-DSPE | 3469.0/3469.1 |
| **20** | 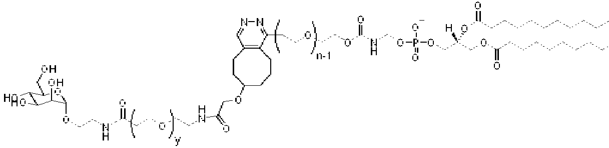 Man-TCO-PEG2000-DSPE | 3340.1/3340.0 |
| **21** | 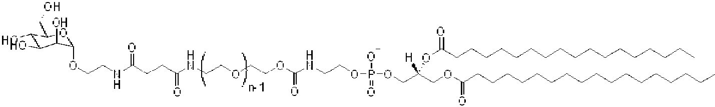 Man-succinamide-PEG2000-carbamate-DSPE | 3076.0/3075.9 |
| **22** | 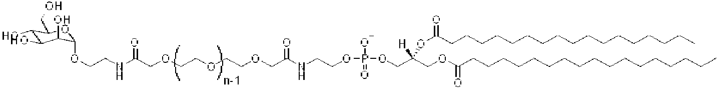 Man-amide-PEG2000-amide-DSPE | 3048.9/3048.8 |
| **23** | 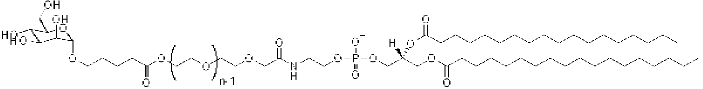 Man-pentanoate-PEG2000-amide-DSPE | 3047.9/3047.8 |
| **24** | 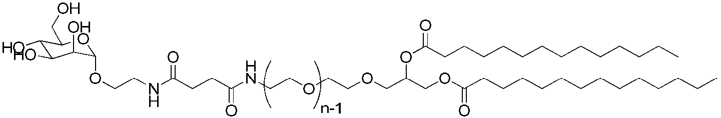 Man-succinamide-PEG2000-DMG | 2797.8/2797.7 |
| **25** | 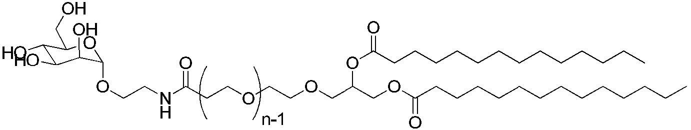 Man-amide-PEG2000-DMG | 2726.8/2726.7 |
| **26** | 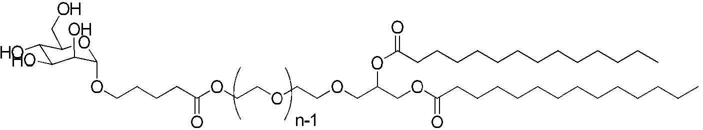 Man-pentanoate-PEG2000-DMG | 2755.8/2755.7 |
| **27** | 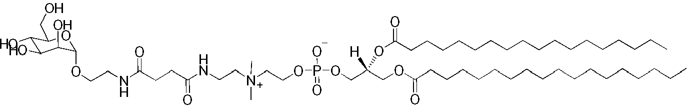 (R)-2,3-bis(stearoyloxy)propyl (2-(dimethyl(2-(4-oxo-4-((2-(((2S,3S,4S,5S,6R)-3,4,5-trihydroxy-6-(hydroxymethyl)tetrahydro-2H-pyran-2-yl)oxy)ethyl)amino)butanamido)ethyl)ammonio)ethyl) phosphate | 1123.8/1123.7 |
| **28** | 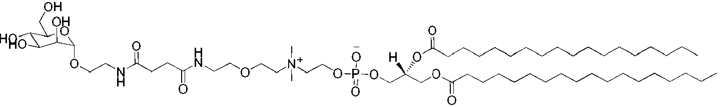 (R)-2,3-bis(stearoyloxy)propyl (3,3-dimethyl-10,13-dioxo-16-(((2S,3S,4S,5S,6R)-3,4,5-trihydroxy-6-(hydroxymethyl)tetrahydro-2H-pyran-2-yl)oxy)-6-oxa-3,9,14-triazahexadecan-3-ium-1-yl) phosphate | 1167.8/1167.7 |
| **29** | 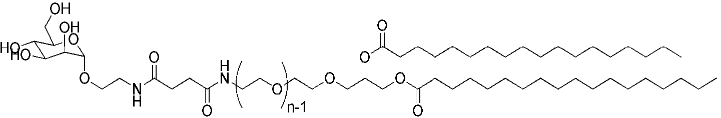 Man-succinamide-PEG2000-DSG | 2909.9/2909.8 |
| **30** | 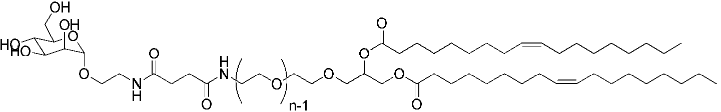 Man-succinamide-PEG2000-DOG | 2905.9/2905.8 |
| **31** | 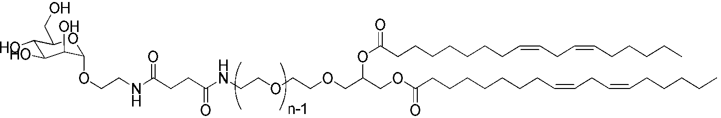 Man-succinamide-PEG2000-DLG | 2901.8/2901.7 |
| **32** | 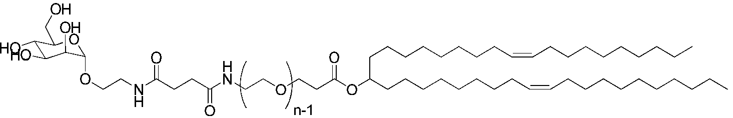 Man-PEG2000-(10Z,29Z7)-tetraconta-10,29-dien-20-yl | 2887.9/2887.8 |
| **33** | 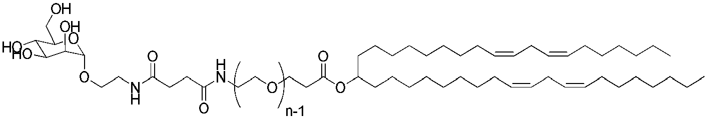 Man-PEG2000-(7Z,10Z,29Z,32Z)-tetraconta-7,10,29,32-tetraen-20-yl | 2883.9/2883.9 |
| **34** | 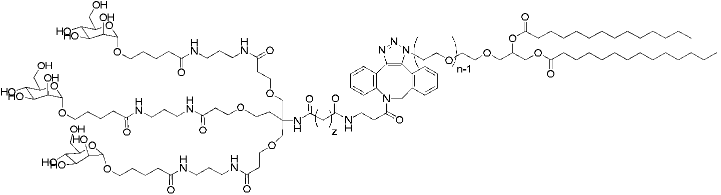 Tri-Man-DBCO-PEG2000-DMG | 4196.5/4196.4 |
| **35** | 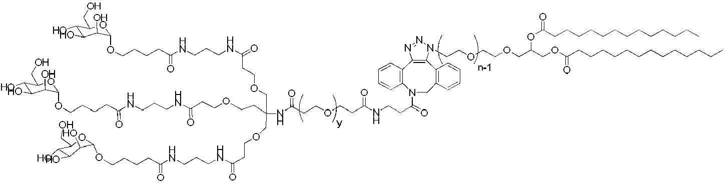 Tri-Man-PEG-DBCO-PEG2000-DMG | 4270.5/4270.6 |
| **36** | 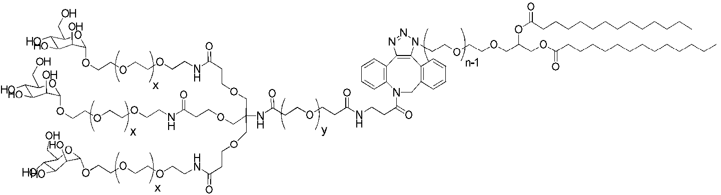 Tri-(PEG-Man)-PEG-DBCO-PEG2000-DMG | 4181.4/4181.5 |
| **37** | 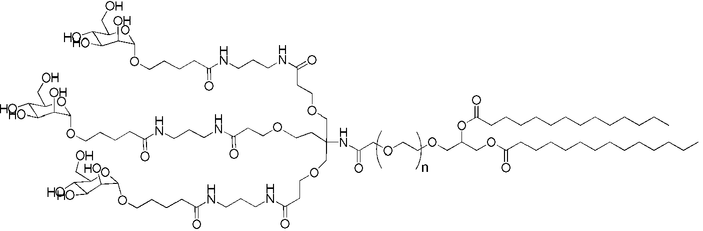 Tri-Man-PEG2000-DMG | 3839.3/3839.4 |
| **38** | 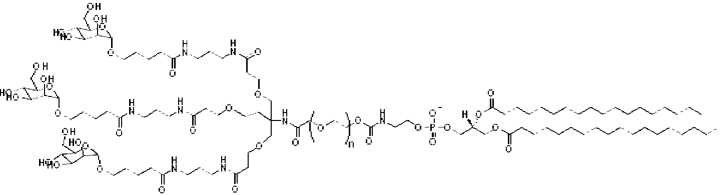 Tri-Man-PEG2000-DSPE | 4117.4/4117.3 |
| **39** | 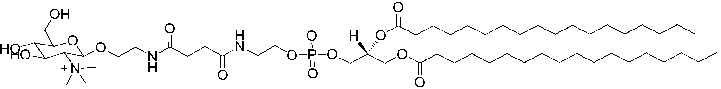 (R)-2,3-bis(stearoyloxy)propyl (2-(4-((2-(((2R,3R,4R,5S,6R)-4,5-dihydroxy-6-(hydroxymethyl)-3-(trimethylammonio)tetrahydro-2H-pyran-2-yl)oxy)ethyl)amino)-4-oxobutanamido)ethyl) phosphate | 1093.8/1093.7 |
| **40** | 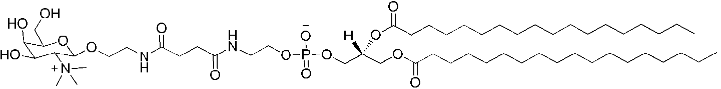 (R)-2,3-bis(stearoyloxy)propyl (2-(4-((2-(((2R,3R,4R,5R,6R)-4,5-dihydroxy-6-(hydroxymethyl)-3-(trimethylammonio)tetrahydro-2H-pyran-2-yl)oxy)ethyl)amino)-4-oxobutanamido)ethyl) phosphate | 1093.8/1093.9 |
| **41** | 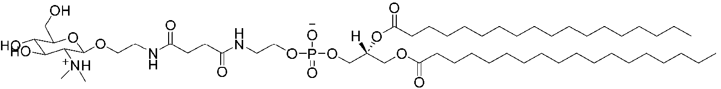 (R)-2,3-bis(stearoyloxy)propyl (2-(4-((2-(((2R,3R,4R,5S,6R)-3-(dimethylammonio)-4,5-dihydroxy-6-(hydroxymethyl)tetrahydro-2H-pyran-2-yl)oxy)ethyl)amino)-4-oxobutanamido)ethyl) phosphate | 1079.7/1079.6 |
| **42** | 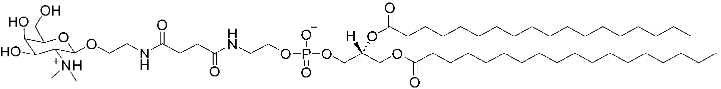 (R)-2,3-bis(stearoyloxy)propyl (2-(4-((2-(((2R,3R,4R,5R,6R)-3-(dimethylammonio)-4,5-dihydroxy-6-(hydroxymethyl)tetrahydro-2H-pyran-2-yl)oxy)ethyl)amino)-4-oxobutanamido)ethyl) phosphate | 1079.7/1079.8 |
| **43** | 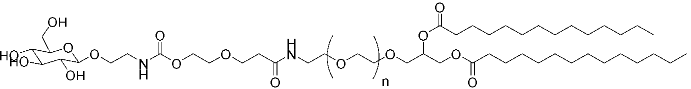 Glu-carbamate-ethylethoxy-PEG2000-DMG | 2857.8/2857.7 |
| **44** | 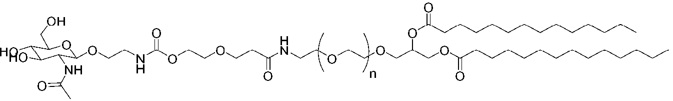 GleNAc-carbamate-ethylethoxy-PEG2000-DMG | 2898.8/2898.7 |
| **45** | 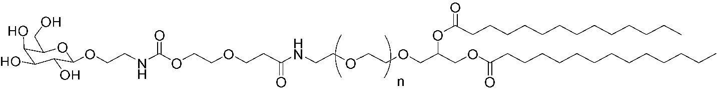 Gal-carbamate-ethylethoxy-PEG2000-DMG | 2857.8/2857.8 |
| **46** | 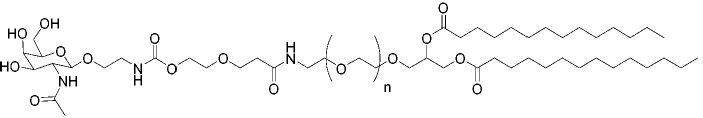 GalNAc-carbamate-ethylethoxy-PEG2000-DMG | 2898.8/2898.7 |
| **47** | 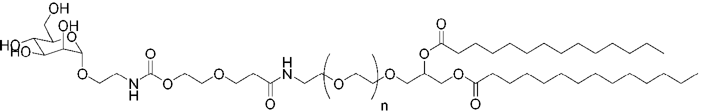 Man-carbamate-ethylethoxy-PEG2000-DMG | 2857.8/2857.6 |
| **48** | 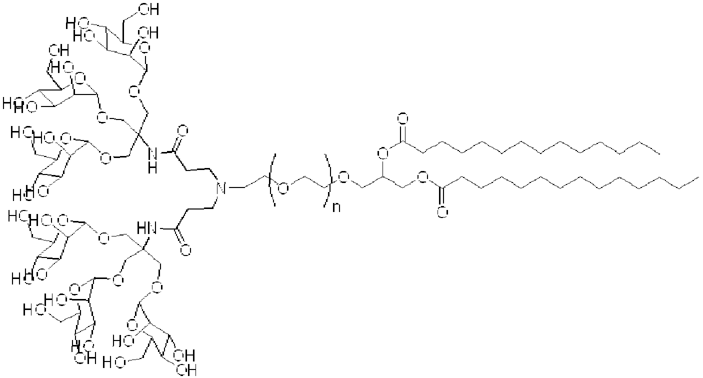 Di(tris(Man)methylamide)amidoamine-PEG2000-DMG | 3811.2/3811.3 |
| **49** | 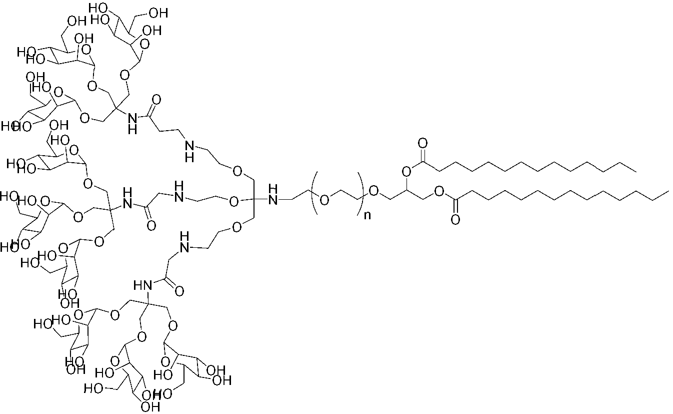 Tris(2-(tri-Man-amidoamine)ethoxy)methylamine-PEG2000-DMG | 4661.6/4661.5 |
| **50** | 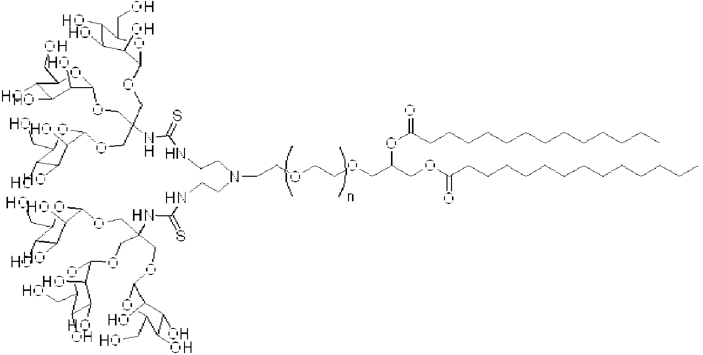 Di(tris(Man)methylamine)thioureaethylamine-PEG2000-DMG | 3873.1/3873.2 |
| **51** | 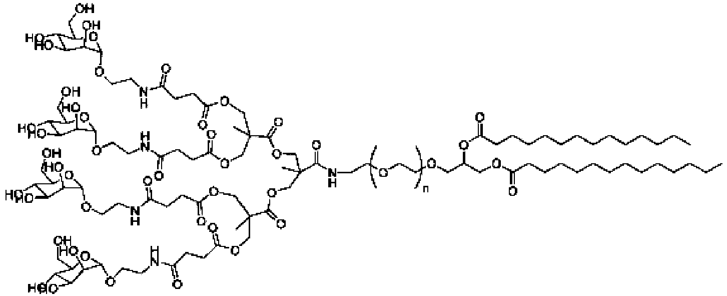 G1-bis-MPA-(Man-succinamide)-PEG2000-DMG | 4061.2/4061.3 |
| **52** | 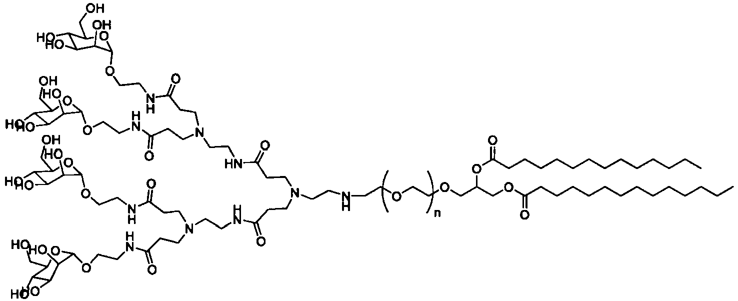 Di((di(Man-ethylamidoamine))ethylamidoamine)ethylamine-PEG2000-DMG | 3872.3/3872.2 |
| **53** | 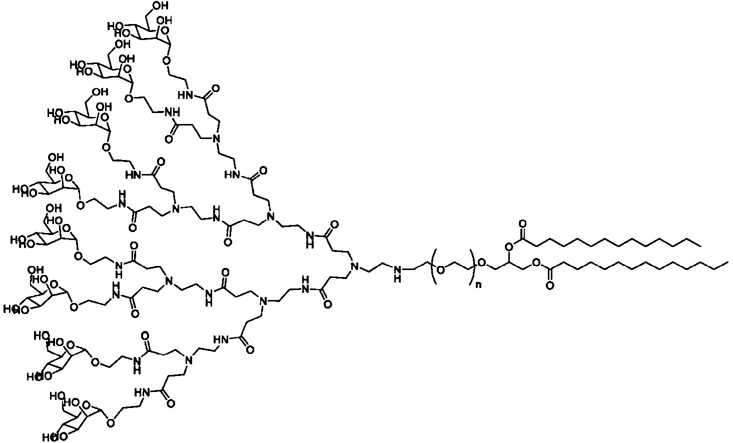 Di(di((di(Man-ethylamidoamine))ethyl amidoamine)ethylamidoamine)ethylamine-PEG2000-DMG | 5437.1/5437.0 |
| **54** | 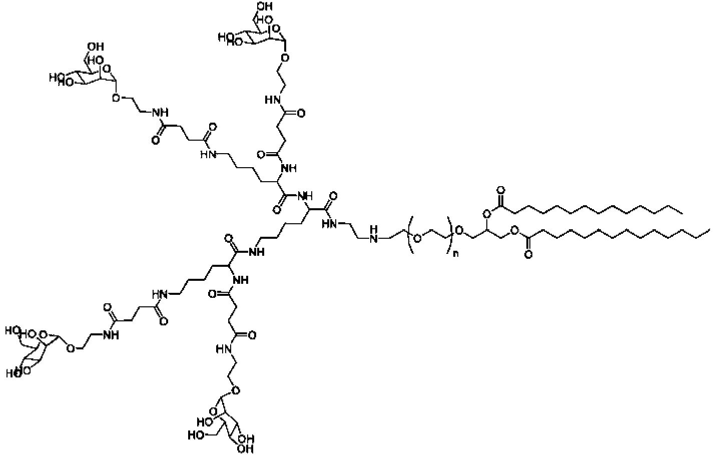 Gl-polylysine-(Man-succinamide)-ethylamine-PEG2000-DMG | 4140.4/4140.3 |
| **55** | 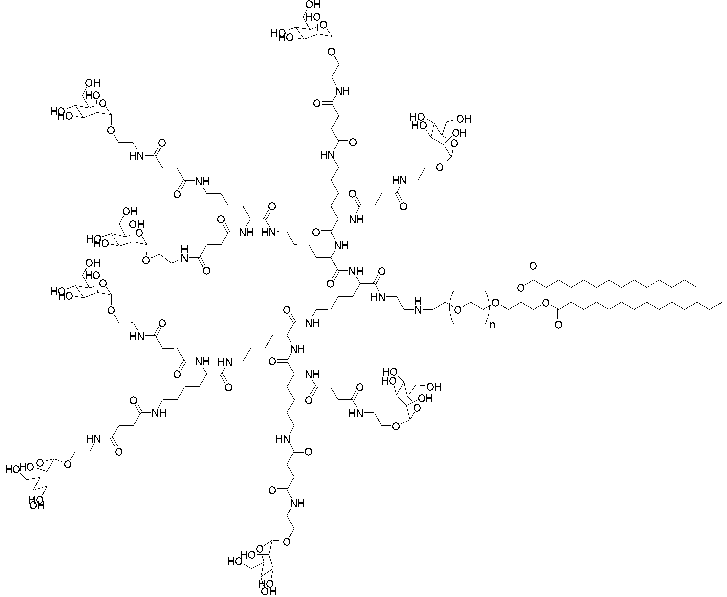 G2-polylysine-(Man-succinamide)-ethylamine-PEG2000-DMG | 5873.2/5873.1 |

In Table 1 above, n is 45, x is 1, y is 2, and z is 3.

### Example 14. Preparation of mRNA-lipid nanoparticles (LNPs)

Ionizable lipids (SM-102 (Broadpharm, USA; Patent Document 3 [PCT/US2016/052352 (BENENARO, K. E.; KUMARASINGHE, E. S.; CORNEBISE, M.), 2016.09.16]), ALC-0315 (Broadpharm, USA; Patent Document 4 [PCT/US2016/029572 (TAM, Y.; HOPE, M. J.; WEISSMAN, D.; PARDI, N.), 2016.04.27]), DLin-MC3-DMA (MC3; Broadpharm, USA), etc.), phospholipids (Compounds **11**-**15**, **27**-**28**, **39**-**42**, DSPC (Sigma-Aldrich, USA), etc.), cholesterol (Sigma-Aldrich, USA), and PEG-lipids (Compounds **1**-**10**, **16**-**26**, **29**-**38**, **43**-**55**, ALC-0159 (Broadpharm, USA; Patent Document 5 [PCT/US2015/034496 (ANSELL, S. M.; DU, X) 2015.06.05]), DMG-PEG (DMG-PEG2000; Sigma-Aldrich, USA), etc.), which were synthesized according to Examples 1 to 13 or purchased commercially, were dissolved in an organic phase (ethanol). The aqueous phase was prepared by diluting 5-100 µg of mRNA (firefly luciferase mRNA (fLuc mRNA), enhanced green fluorescent protein mRNA (EGFP mRNA), etc.; TriLink Biotechnologies, USA) in 0.2-2.0 mL of a 5-50 mM aqueous sodium acetate solution or a 5-50 mM aqueous sodium citrate solution.

The mRNA-lipid nanoparticles (LNPs) were prepared by mixing the organic phase (ethanol) containing ionizable lipids, the phospholipids, the cholesterol, and the PEG-lipidswith the aqueous phase (the aqueous sodium acetate or sodium citrate solution) containing the mRNA using a microfluidic mixing device (NanoAssemblr Spark; Precision Nanosystems, Canada) at an average flow rate of 25-250 µL/10 s. Specifically, the constituent lipid compounds were dissolved in ethanol at a molar ratio of (20-60):(0-25):(30-60):(0-10) for ionizable lipids: phospholipids: cholesterol: PEG-lipids, and phospholipid 1 and PEG-lipid 1 attached to the targeting ligands were mixed at 0.05-100 mol% of the total phospholipid and the total PEG-lipid, respectively (see Table 2), wherein the total molar ratio sums to 100. The lipid nanoparticles were prepared by mixing the organic phase and the aqueous phase such that the mass ratio of the ionizable lipids to the mRNA was (4-30):1. To remove the ethanol from the mRNA-LNPs and to adjust the pH of the LNP aqueous solution to a level similar to physiological pH (~ 7.4), dialysis was performed using a dialysis cassette (Slide-A-Lyzer^{™} Dialysis Cassette, 10K MWCO, Thermo Fisher, USA) against hosphate-buffered saline (PBS, pH 7.4, Invitrogen, USA) or 12-18 hours with multiple buffer exchanges. The expected structure of the prepared mRNA-LNPs is shown in FIG. 1.

FIG. 1 is a schematic diagram showing the structure of mRNA-LNPs for tissue-specific delivery of active substances according to an embodiment of the present disclosure. A is the LNP to which tissue-specific ligands are not attached; B is the LNP in which a portion of the surface is modified with PEG-lipids attached to tissue-specific ligands; C is the LNP in which most of the surface is modified with PEG-lipids attached to tissue-specific ligands; D is the LNP in which a portion of the surface is modified with phospholipids attached to tissue-specific ligands; and E is the LNP in which most of the surface is modified with phospholipids attached to tissue-specific ligands.

### [Experimental Examples]

### Experimental Example 1. Measurement of pKa of mRNA-LNPs

The apparent pKa of the mRNA-LNPs according to Example 14 was determined by analysis using 6-(*p*-toluidino)-2-naphthalenesulfonic acid sodium salt (TNS; Sigma-Aldrich, USA). Anionic TNS, an anionic compound, generally does not exhibit fluorescence in an aqueous solution. However, as the pH decreases, the proportion of positively charged ionizable lipids increases, allowing electrostatic interactions with TNS. As a result, TNS migrates into the hydrophobic domain (lipophilic) and exhibits progressively stronger fluorescence. Conversely, when the pH of the solution increases and the ionizable lipids are neutralized, the TNS can no longer engage in electrostatic interactions. Consequently, TNS loses its lipophilic character, exits the hydrophobic domain, and its fluorescence is quenched. Based on this principle, the pKa of the LNPs can be measured by plotting the change in TNS fluorescence intensity according to the variations in the surrounding pH as an S-shaped curve, and calculating the log value of the inflection point. Generally, LNPs with a pKa ranging from 6.0 to 7.0 exhibit near-neutral surface charge in plasma (pH ~ 7.4) upon intravenous injection, thereby reducing nonspecific interactions with tissues and increasing delivery efficiency to hepatocytes.Upon internalization into cells, the acidic environment of the endosome (pH 5.5) causes the LNPs to become highly positively charged, which facilitates endosome escape of the encapsulated active substances, such as nucleic acids, proteins, or synthetic drugs, thereby enhancing *in vivo* delivery efficiency. (Non-Patent Document 1 [Sabnis, S. et al., Mol. Ther. 2018, 26, 1509], Non-Patent Document 2 [Jayaraman, M. et al., Angew. Chem. Int. Ed. 2012, 51, 8529]).

Specifically, a master buffer containing 10 mM sodium phosphate, 10 mM sodium borate, 10 mM sodium citrate, and 150 mM sodium chloride (NaCl) was prepared, and the pH of this aqueous solution was then adjusted over a range from pH 2.5 to 12 using 1 M sodium hydroxide (NaOH) and 1 M hydrochloric acid (HCl). Then, 150-300 µM TNS standard stock solutions were prepared by dissolving TNS in DMSO. LNPs were prepared at mRNA concentrations of 0.02-0.08 mg/mL in either 1× PBS buffer or a 20 mM tris(hydroxymethyl)aminomethane (Tris) buffer containing 8 % sucrose, respectively. Solutions adjusted to each pH were dispensed into three wells (n = 3) of a black-bottomed 96-well plate, with 90-94 µL added per well. 3.26-4.00 µL of LNPs and 2 µL of the TNS solution were added to each well. After each well was carefully mixed, the fluorescence intensity of each well was measured with a multi-plate reader (Cary Eclipse, Agilent Technologies, USA) at an excitation wavelength of λₑₓ 330 nm and an emission wavelength of λₑₘ 435 nm.

### Experimental Example 2. Measurement of Size, Polydispersity, and Surface Charge of mRNA-LNP

Each mRNA-LNPs prepared in Example 14 was diluted with PBS to a final mRNA concentration of 1 µg/mL, and then the size, polydispersity index (PDI), and surface charge of the mRNA-LNPs were measured using Malvern Zetasizer Nano ZS90 (Malvern Instruments, UK). The results are shown in Table 2. For particles intended for systemic administration, such as via blood vessels, the particle size must be smaller than 200 nm in order to pass through the endothelial capillaries of the liver. In addition, the size distribution is considered uniform hen the PDI is 0.3 or less, which enables consistent and efficient therapeutic effects. (Non-Patent Document 3 [Danaei, M.; Dehghankhold, M.; Ataei, S.; Davarani, F. H.; Javanmard, R.; Dokhani, A.; Khorasani, S.; Mozafari, M. R., Pharmaceutics 2018, 10, 57]).

### Experimental Example 3. Measurement of mRNA Encapsulation Efficiency

The mRNA encapsulation efficiency (EE, %) of LNPs containing mRNA was measured using the RiboGreen RNA assay kit (Quant-iT^{™} RiboGreen RNA, Invitrogen). The results are shown in Table 2. The mRNA-LNPs prepared in Example 14 were diluted to a final volume of 50 µL using either 1X TE (Tris-EDTA) buffer or a 1X TE buffer containing 2 % Triton-X 100, such that the final concentration of mRNA was 20-30 ng/mL, and then added to each well of a 96-well plate. Group A (without Triton X-100) was supplemented with 50 µL of 1X TE buffer, while Group B (with Triton X-100) was supplemented with 50 µL of 1X TE buffer containing 2% Triton-X 100. To release the encapsulated mRNA, the LNPs were treated with Triton-X 100 and incubated at 37°C for 10 minutes. Then, 100 µL of a RiboGreen reagent was added to each well. The fluorescence intensity of Group A and Group B was measured using a multi-plate reader (*λ*ₑₓ 480 nm, *λ*ₑₘ 520 nm), and mRNA encapsulation efficiency (%) was calculated using Equation 1 below. The mRNA encapsulation efficiency (%) for each LNP was determined as the average of two independent measurements. 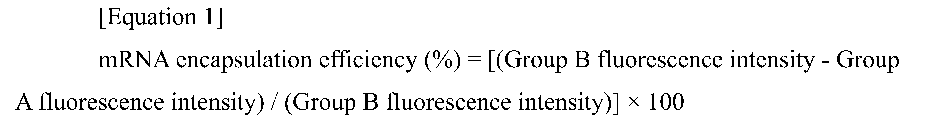 mRNA encapsulation efficiency (%) = [(Group B fluorescence intensity - Group A fluorescence intensity) / (Group B fluorescence intensity)] × 100

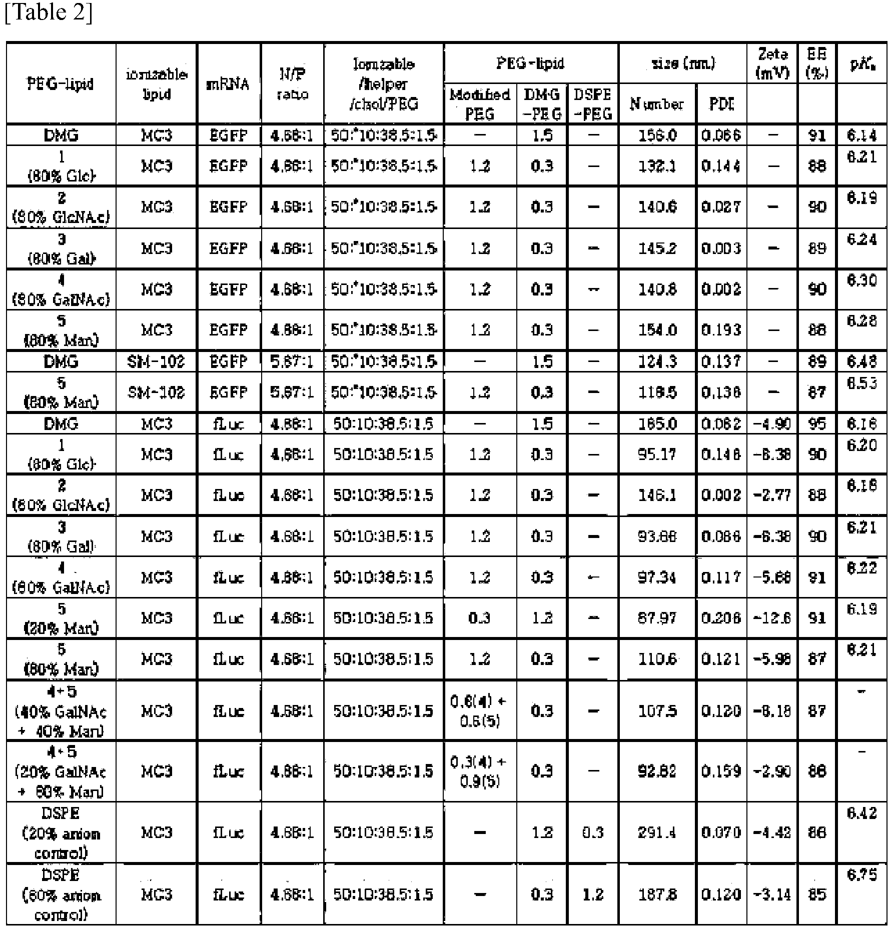

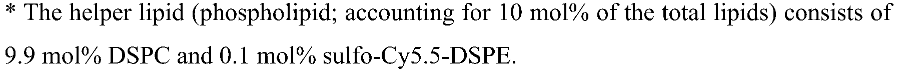

### Experimental Example 4. Evaluation of Protein Expression Efficiency of EGFP mRNA-LNPs Administered to Cells

In this experimental example, LNPs encapsulating EGFP mRNA prepared according to Example 14 were administered to three types of retinal cells (ARPE-19 human retinal pigment epithelial cells (ATCC, USA), 661W mouse cone photoreceptor cells (provided by Prof. Jin-Woo Kim's laboratory at KAIST), and rMC-1 rat Müller glial cells (Kerafast, USA)) to compare *in vitro* protein expression efficiency (related to transfection, endosomal escape, and mRNA stability) over time.

As lipid compounds constituting the LNPs, cholesterol was used as the structural lipid, and MC3 or SM-102 was used as the ionizable lipid. DSPC was used as the phospholipid, but 1 mol% of the total phospholipids was substituted with sulfo-Cy5.5-DSPE for tracking the LNPs under a fluorescence microscope. As shown in Table 1, as a control, EGFP mRNA-LNPs were formulated with neutral PEG-lipid DMG-PEG2000. In the experimental group, the formulation comprised a portion of DMG-PEG2000 and a portion of lipid compounds **1**-**5**, which are terminally modified anionic DSPE-PEG derivatives with targeting ligands.

Specifically, 18 hours before administration of the mRNA-LNPs, the ARPE-19, 661W, and rMC-1 cells were seeded in an 8-well plate (ibidi) at a density of 1 × 10⁴ to 2 × 10⁵ cells per well, and then cultured at 37°C in a humidified atmosphere containing 5% CO2. The concentration of the administered mRNA-LNPs was adjusted to 100 ng per well based on mRNA to treat the cells. At 5, 10, 15, and 30 minutes, and at 1, 3, 6, 24, and 48 hours after treatment, the culture medium was removed, the cells were fixed with 4 % PFA for 15 minutes, and then fluorescence images were acquired using a fluorescence microscope as shown in FIG. 7.

As shown in FIG. 7, in the ARPE-19 cells administered with the EGFP mRNA-LNPs, green fluorescence derived from GFP expression was detected as early as 5 min after treatment, and the fluorescence intensity gradually increased over time. In addition, when SM-102 was used as the ionizable lipid instead of MC3, the overall GFP expression level (green fluorescence) was significantly stronger, reaching a maximum at 24 h and 48 h. Meanwhile, when SM-102, which exhibited strong signal intensity, was used as the ionizable lipid, and 80 mol% of the total PEG-lipid in the LNPs was substituted with compound **5** for tissue-specific delivery, the fluorescence intensity (expression) of GFP was notably stronger, particularly at 24 h and 48 h, compared to LNPs containing only unmodified DMG-PEG.

Therefore, when the LNPs comprising terminally modified lipids according to an embodiment of the present disclosure are administered to cells for tissue-specific targeting, intracellular delivery is selectively enhanced, and efficient endosomal escape occurs at relatively early time points, resulting in high levels of protein expression in the cytoplasm.

### Experimental Example 5. Evaluation of Protein Expression Efficiency of fLuc mRNA-LNPs via Subretinal Injection

All animal experiments described below were conducted with prior approval from the Institutional Animal Care and Use Committee (IACUC) of the Korea Research Institute of Bioscience and Biotechnology (KRIBB), following ethical review. In this experimental example, fLuc mRNA-LNPs (100 ng to 1 mg of mRNA, 300 ng/µL) comprising the modified lipid compounds were administered to 6- to 8-week-old female or male C57BL/6J mice (Orient Bio) under inhalation anesthesia (3% isoflurane and 97% medical oxygen) via subretinal injection (pars plana approach; Non-Patent Document 4 [Huang, P. et al., Nature Protocols 2022, 17, 1468]) using a 33-gauge blunt-needle Hamilton syringe. Then, to assess protein expression levels at 24 h and 48 h post-administration, 200 µL of luciferin (15 mg/mL) was administered via intraperitoneal injection 10 min before imaging. Luminescence images were acquired using an *in vivo* imaging system (IVIS, Lumina III, PerkinElmer, Waltham, MA) under inhalation anesthesia with 3% isoflurane and 97% medical oxygen. The results are shown in FIGs. 2 and 4.

In the LNP formulations, cholesterol was used as the structural lipid, MC3 as the ionizable lipid, and DSPC as the phospholipid. As shown in Table 1, for the PEG-lipid, the control group employed neutral DMG-PEG2000 or terminally unmodified DSPE-PEG at the same molar ratios (20 mol% and 80 mol% of the total PEG-lipid) as the experimental group to evaluate the effect of the anionic charge. In the experimental group, the LNPs comprised a portion of DMG-PEG2000 and a portion of lipid compounds **1**-**5**, which are terminally modified anionic DSPE-PEG.

FIG. 2 shows IVIS images acquired at 24 h and 48 h after subretinal injection of fLuc mRNA-LNPs containing Compound **5**, a modified PEG-lipid according to an embodiment of the present disclosure, into the mouse eye. Compared to the control group containing the same level of anionic charge, LNPs containing Compound **5** at 20 mol% or 80 mol% of the total PEG-lipid exhibited substantially higher levels of protein expression. In addition, the protein expression signal at 24 h was higher than that at 48 h, and stronger protein expression was observed when a larger proportion of Compound **5** was contained in the total PEG-lipid (i.e., 80 mol% rather than 20 mol%). These results suggest that Compound **5** contributes to more efficient tissue-specific delivery to the retinal tissue at the injection site.

FIG. 4 shows IVIS images acquired at 24 h after subretinal administration of fLuc mRNA-LNPs containing 80 mol% of modified PEG-lipid compounds **1**-**5** out of the total PEG-lipid (with the remaining 20 mol% consisting of DMG-PEG2000) according to an embodiment of the present disclosure, into the mouse eyes. The results demonstrate that the efficiency of retinal tissue-specific delivery varies depending on the type of monosaccharide used for the terminal modification of the PEG-lipid. Specifically, relatively high protein expression levels were observed when glucose (Glu) or mannose (Man) was used. High expression was also observed when mannose (Man) and N-acetylgalactosamine (GalNAc) were used in combination at the same molar ratio. In contrast, relatively lower protein expression was observed when galactose (Gal) or GalNAc was used alone. Therefore, it is expected that by selecting and optimizing a ligand exhibiting tissue specificity, novel LNPs and compositions capable of delivering active substances with significantly enhanced efficiency compared to conventional systems can be developed.

### Experimental Example 6. Evaluation of Protein Expression Efficiency of fLuc mRNA-LNPs via Intravitreal Injection

In this experimental example, fLuc mRNA-LNPs (100 ng to 1 mg of mRNA, 300 ng/µL) comprising the modified lipid compounds were administered to 6- to 8-week-old female or male C57BL/6J mice (Orient Bio) under inhalation anesthesia (3% isoflurane and 97% medical oxygen) via intravitreal injection (Non-Patent Document 5 [You, S. et al., Biomaterials 2020, 255, 120188]) using a 33-gauge blunt-needle Hamilton syringe. Then, to assess protein expression levels at 24 h and 48 h post-administration, 200 µL of luciferin (15 mg/mL) was administered via intraperitoneal injection 10 min before imaging. Luminescence images were acquired using an *in vivo* imaging system (IVIS) Lumina III (PerkinElmer, Waltham, MA) under inhalation anesthesia with 3% isoflurane and 97% medical oxygen. The results are shown in FIGS. 3 and 5.

The lipid compounds constituting the LNPs are the same as those described in Experimental Example 5.

FIG. 3 shows IVIS images acquired at 24 h and 48 h after intravitreal injection of fLuc mRNA-LNPs containing Compound **5**, a modified PEG-lipid according to an embodiment of the present disclosure, into the mouse eyes. In the case of intravitreal injection, unlike the results observed with subretinal injection, no signal was detected in the eyes of mice treated with control LNPs consisting of unmodified PEG-lipids. In contrast, among the LNPs containing Compound **5**, terminally modified PEG-lipid, only a weak signal was observed when Compound **5** was included at 20 mol% out of the total PEG-lipid, with protein expression detected in one of the two mice tested. When the content was increased to 80 mol%, a relatively strong signal (protein expression) was observed. These results indicate that protein expression increases with increasing amount of Compound **5**, suggesting that mannose (Man), the ligand attached to the PEG terminal, contributes to enhanced delivery efficiency. In addition, the protein expression was stronger at 24 h than at 48 h post-administration.

FIG. 5 shows IVIS images acquired at 24 h after intravitreal administration of fLuc mRNA-LNPs containing 80 mol% of modified PEG-lipid compounds **1**-**5** (with the remaining 20 mol% consisting of DMG-PEG2000) according to an embodiment of the present disclosure. Unlike subretinal injection, most LNPs exhibited almost no protein expression. However, LNPs containing Compound **5** exhibited relatively distinct protein expression at 24 h, and the LNPs containing Compound **4** also exhibited weak but observable protein expression. Therefore, in the case of intravitreal injection, LNPs containing Compound **5** are considered to exhibit relatively excellent retinal tissue-specific delivery efficiency

Although intravitreal injection is a much safer and more convenient administration method compared to subretinal injection of Experimental Example 5, the delivery efficiency is generally significantly lower compared to subretinal injection, which is directly injected into the outer retinal layers (RPE and photoreceptors) where pathological phenomena predominantly occur. Upon intravitreal injection, most of the injected LNPs fail to penetrate the retina and are expelled to the outside, and even when partial penetration occurs, they rarely reach the deeper outer layers and mostly remain in inner layers such as the ganglion cell layer. Therefore, there is a demand for the development of LNPs that exhibit high outer retinal tissue-specific delivery efficiency, such that the LNPs have excellent retinal penetrability and enable efficient transfection into surrounding RPE and photoreceptor cells upon reaching the outer layers. Consequently, through the optimization of LNPs containing Compound **5** at 80 mol% out of the total PEG-lipid according to an embodiment of the present disclosure, it is expected that the active substances can be efficiently delivered to the outer retinal layer via intravitreal injection that allows for safe and repeated administration.

### Experimental Example 7. Evaluation of Protein Expression Efficiency of fLuc mRNA-LNPs via Intramuscular Injection (I.M.) and Intravenous Injection (I.V.)

In this experimental example, fLuc mRNA-LNPs comprising the modified lipid compounds prepared according to the method of Example 14 were administered *in vivo* via intramuscular injection (i.m.) or intravenous injection (i.v.) to compare the resulting protein expression efficiency (related to transfection, endosomal escape, and mRNA stability) *in vivo*.

The dose of mRNA-LNPs was adjusted to 0.1-0.5 mg/kg of body weight, and the LNPs were administered via i.m. or i.v. injection to female C57BL/6 mice (6-10 weeks old, Orient Bio, Korea). Subsequently, at 1, 3, 6, 24, 48, and 72 h post-administration, 200 µL of luciferin was administered via intraperitoneal injection, and then whole-body fluorescence images were acquired using an IVIS Lumina III (PerkinElmer, USA).

FIG. 6 shows luminescence images acquired at 1, 3, 6, and 24 h after administration via the mice tail veins injection of fLuc mRNA-LNPs containing 80 mol% of modified PEG-lipid compounds **1**-**5** (with the remaining 20 mol% consisting of DMG-PEG2000) according to an embodiment of the present disclosure. In the case of mRNA-LNPs containing Compound **4**, which utilizes GalNAc as a terminal ligand, a remarkably high protein expression efficiency was observed, particularly in the liver, and the signal was much stronger at 3 h than at 1 h post-administration. Next, protein expression in the liver was also observed for the mRNA-LNPs containing Man alone or GalNAc/Man mixture at a ratio of 1:3, but the signals were markedly lower than that of the case of GalNAc alone, and the signal was hardly detected at 1h, but was at a detectable level at 3 h. These results indicated that when mRNA-LNPs containing modified PEG-lipid compounds are administered via intravenous injection, those incorporating GalNAc as a ligand exhibit exceptionally strong specificity for liver tissue. It is expected that such LNPs capable of tissue-specific delivery can be utilized for selective and efficient treatment of liver-specific diseases.

The preferred embodiments of the present disclosure have been described above. Those of ordinary skill in the art to which the present disclosure pertains will understand that the present disclosure may be embodied in modified forms without departing from the essential characteristics thereof. Therefore, the disclosed embodiments should be considered from a descriptive standpoint rather than a limiting standpoint. The scope of the present disclosure is defined by the appended claims rather than the foregoing description, and all differences falling within the scope of equivalents thereof shall be construed as being included in the present disclosure.

## Claims

1. A lipid compound represented by Formula 1, an isomer thereof, or a pharmaceutically acceptable salt thereof: 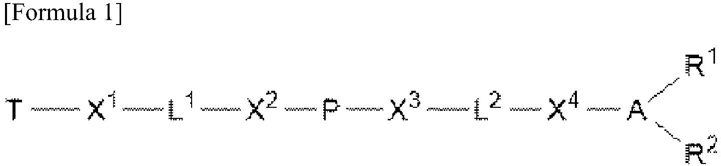 wherein, in Formula 1,
T is a targeting ligand, and is one selected from the group consisting of a monosaccharide, a polysaccharide, folic acid, transferrin, an RGD peptide, a cyclic RGD peptide, a TAT peptide, an R9 peptide, a CADY peptide, an HA2 peptide, a monoclonal antibody, an antigen-binding fragment or an antibody fragment, a single-chain variable fragment (scFv), and an aptamer, or a branched targeting ligand of the following Formula 2;
X¹, X², X³, and X⁴ are each independently one selected from the group consisting of a single bond, C₁-C₁₄ alkylene, C₁-C₁₄ heteroalkylene, C₂-C₁₄ alkenylene, C₁-C₆ alkyl-C(=O)-, C₂-C₆ alkenyl-C(=O)-, - (OCH₂CH₂)ₘ-, -(CH₂CH₂O)ₘ-, -O-, -S-, -S(=O)-, -S(=O)₂-, - S(=O)(=NR)-, -C(=O)-, -C(=O)O-, -OC(=O)-, -OC(=O)O-, - C(=O)C(=O)-, -C(=N-OR)-, -O-N=CR-, -OC=N-O-, -C(=O)NR-, - NRC(=O)-, -OC(=O)NR-, -NRC(=O)O-, -C(=O)NRC(=O)-, - S(=O)₂NR-, -NRS(=O)₂-, -NRC(=O)NR-, -NRC(=S)NR-, -C(=O)NH-N=CR-, -RC=N-NHC(=O)-, -NH-N=CR-, -RC=N-NH-, -NR-, -N(OR)-, -S-S-, 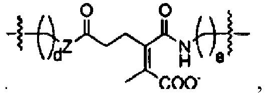 and functional groups bonded by a click chemistry, or a combination thereof,
wherein m is an integer from 0 to 10,
Z is -NH-, -O-, or -S-, and
d and e are each an integer from 1 to 10;
L¹ and L² are each independently a single bond, C₁-C₁₄ alkylene, C₂-C₁₄ alkenylene, MR^{a}M¹, or R^{a}MR^{b};
P is a single bond, -(CH₂CH₂O)_{q}-, -(OCH₂CH₂)_{q}-, - CH₂O(CH₂CH₂O)_{q}CH₂-, or -CH₂CH₂O(CH₂CH₂O)_{q}CH₂-, wherein q is 0 or an integer from 1 to 120;
A is CR^{d} or N;
R¹ and R² are each independently C₁-C₃₀ alkyl, C₂-C₃₀ alkenyl, or R^{c}MR^{d}, wherein the C₁-C₃₀ alkyl and C₂-C₃₀ alkenyl are each independently unsubstituted or substituted with 1 to 3 C₁-C₁₆ alkyl or C₂-C₁₆ alkenyl; M and M¹ are each independently C₁-C₁₄ alkylene, C₂-C₁₄ alkenylene, - NHC(=O)-, -C(=O)NH-, -C(=O)O-, -OC(=O)-, -C(=O)-, -NH-, -N⁺R₂-, -S-, -SS-, -O-, -S(O)₂-, -C(=O)S-, -SC(=O)-, -NHC(=O)NH-, - NHC(=O)O-, -OC(=O)NH-, -OP(=O)(OR)O-, or - OP(=O)(OR)O(CH₂)_{g}-, wherein g is an integer from 1 to 10;
R is hydrogen, C₁-C₂₀ alkyl, C₂-C₂₀ alkenyl, C₂-C₂₀ alkynyl, or C₁-C₂₀ heteroalkyl,
R^{a} and R^{b} are each independently a single bond or C₁-C₁₀ alkylene;
R^{c} is a single bond, C₁-C₁₄ alkylene, or C₂-C₁₄ alkenylene;
R^{d} is hydrogen, C₁-C₁₈ alkyl, or C₂-C₁₈ alkenyl, wherein the C₁-C₁₈ alkyl and C₂-C₁₈ alkenyl are each independently unsubstituted or substituted with 1 to 3 C₁-C₁₂ alkyl or C₂-C₁₂ alkenyl;
The heteroalkyl, the heteroalkylene, the heterocyclyl, and the heteroaryl each independently comprise 1 to 6 heteroatoms selected from N, O, and S;
The branched targeting ligand is represented by Formula 2, 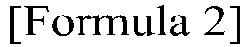 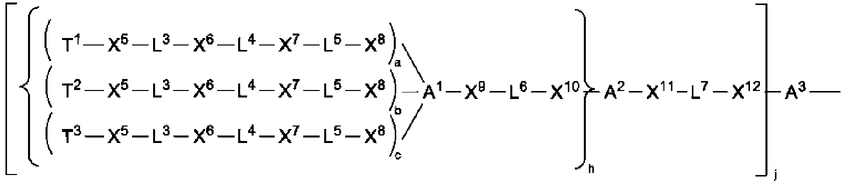
wherein, in Formula 2,
a, b, and c are each independently 0 or 1, wherein at least one of a, b, and c is 1, and h and j are 1 to 3;
T¹, T², and T³ are each independently one selected from the group consisting of a monosaccharide, a polysaccharide, folic acid, transferrin, an RGD peptide, a cyclic RGD peptide, a TAT peptide, an R9 peptide, a CADY peptide, an HA2 peptide, a monoclonal antibody, an antigen-binding fragment or an antibody fragment, a single-chain variable fragment (scFv), and an aptamer;
X⁵, X⁶, X⁷ X⁸, X⁹, X¹⁰, X¹¹, and X¹² are each independently one selected from the group consisting of a single bond, C₁-C₁₄ alkylene, C₁-C₁₄ heteroalkylene, C₂-C₁₄ alkenylene, C₁-C₆ alkyl-C(=O)-, C₂-C₆ alkenyl-C(=O)-, -(OCH₂CH₂)ₘ-, -(CH₂CH₂O)ₘ-, -O-, -S-, -S(=O)-, -S(=O)₂-, - S(=O)(=NR)-, -C(=O)-, -C(=O)O-, -OC(=O)-, -OC(=O)O-, - C(=O)C(=O)-, -C(=N-OR)-, -O-N=CR-, -OC=N-O-, -C(=O)NR-, - NRC(=O)-, -OC(=O)NR-, -NRC(=O)O-, -C(=O)NRC(=O)-, - S(=O)₂NR-, -NRS(=O)₂-, -NRC(=O)NR-, -NRC(=S)NR-, -C(=O)NH-N=CR-, -RC=N-NHC(=O)-, -NH-N=CR-, -RC=N-NH-, -NR-, -N(OR)-, -S-S-, 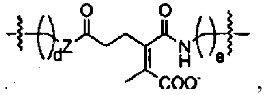 and functional groups bonded by a click chemistry, or a combination thereof,
wherein m is an integer from 0 to 10,
Z is -NH-, -O-, or -S-, and
d and e are each an integer from 1 to 10;
L³, L⁴, L⁵, L⁶, and L⁷ are each independently a single bond, C₁-C₁₄ alkylene, C₂-C₁₄ alkenylene, MR^{a}M¹, or R^{a}MR^{b};
A¹, A², and A³ are each independently a single bond, CR^{d}, or N; and The heteroalkyl and the heteroalkylene each independently comprise 1 to 6 heteroatoms selected from N, O, and S.

2. The lipid compound, an isomer thereof, or a pharmaceutically acceptable salt thereof of claim 1,
wherein the monosaccharide is one selected from the group consisting of glucose, N-acetylglucose, galactose, N-acetylgalactose, mannose, allose, altrose, arabinose, cladinose, erythrose, erythrulose, fructose, D-fucitol, L-fucitol, fucosamine, fucose, fuculose, galactosamine, D-galactosaminitol, N-acetylgalactosamine, glucosamine, N-acetylglucosamine, glucosaminitol, glucose-6-phosphate, glyceraldehyde, L-glycero-D-manno-heptose, glycerol, glycerone, gulose, idose, lyxose, mannosamine, mannose-6-phosphate, psicose, quinovose, quinovosamine, rhamnitol, rhamnosamine, rhamnose, ribose, ribulose, sedoheptulose, sorbose, tagatose, talose, tartaric acid, threose, xylose, xylulose, N,N,N-trimethylglucosamine, and N,N,N-trimethylgalactosamine, and
the polysaccharide comprises a disaccharide, a trisaccharide, and an oligosaccharide, and is one selected from the group consisting of abequose, acarbose, amicetose, amylopectin, amylose, apiose, arcanose, ascarylose, ascorbic acid, boivinose, cellobiose, cellotriose, cellulose, chacotriose, chalcose, chitin, colitose, cyclodextrin, cymarose, dextrin, 2-deoxyribose, 2-deoxyglucose, diginose, digitalose, digitoxose, evalose, evernitrose, fructooligosaccharide, galactooligosaccharide, gentianose, gentiobiose, glucan, glucogen, glycogen, hamamelose, heparin, inulin, isolevoglucosenone, isomaltose, isomaltotriose, isopanose, kojibiose, lactose, lactosamine, lactosediamine, laminarabiose, levoglucosan, levoglucosenone, β-maltose, maltotriose, mannan oligosaccharide, manninotriose, melezitose, melibiose, muramic acid, mycarose, mycinose, neuraminic acid, nigerose, nojirimycin, noviose, oleandrose, panose, paratose, planteose, primeverose, raffinose, rhodinose, rutinose, sarmentose, sedoheptulose, solatriose, sophorose, stachyose, streptose, sucrose, α,α-trehalose, trehalosamine, turanose, tyvelose, xylobiose, and umbelliferose.

3. The lipid compound, an isomer thereof, or a pharmaceutically acceptable salt thereof of claim 1,
wherein the functional groups bonded to each other by a click chemistry are 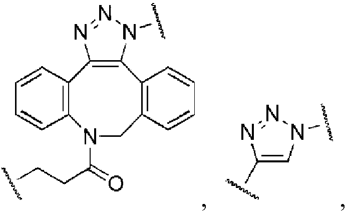 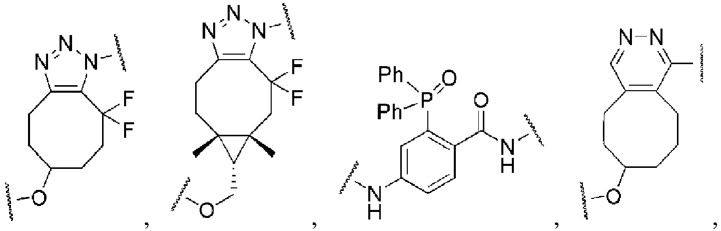 or

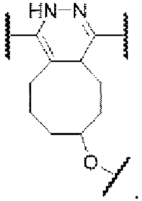

4. The lipid compound, an isomer thereof, or a pharmaceutically acceptable salt thereof of claim 1,
wherein T, T¹, T², and T³ are each independently one selected from the group consisting of glucose (Glu), N-acetylglucosamine (GlcNAc), galactose (Gal), N-acetylgalactosamine (GalNAc), and mannose (Man).

5. The lipid compound, an isomer thereof, or a pharmaceutically acceptable salt thereof of claim 4,
wherein T, T¹, T², and T³ are N-acetylgalactosamine (GalNAc), mannose (Man), or N-acetylglucosamine (GlcNAc).

6. The lipid compound, an isomer thereof, or a pharmaceutically acceptable salt thereof of claim 1,
wherein the lipid compound represented by Formula 1 is any one of Compounds 1-55 in Table 1.

7. The lipid compound, an isomer thereof, or a pharmaceutically acceptable salt thereof of claim 1,
wherein the lipid compound is selected from the group consisting of:
(1) Glu-DBCO-PEG2000-DSPE;
(2) GlcNAc-DBCO-PEG2000-DSPE;
(3) Gal-DBCO-PEG2000-DSPE;
(4) GalNAc-DBCO-PEG2000-DSPE;
(5) Man-DBCO-PEG2000-DSPE;
(6) Glu-succinamide-PEG2000-DMG;
(7) GlcNAc-succinamide-PEG2000-DMG;
(8) Gal-succinamide-PEG2000-DMG;
(9) GalNAc-succinamide-PEG2000-DMG;
(10) Man-succinamide-PEG2000-DMG;
(11) (R)-2,3-bis(stearoyloxy)propyl (2-(dimethyl(2-(((2R,3R,4S,5S,6R)-3,4,5-trihydroxy-6-(hydroxymethyl)tetrahydro-2H-pyran-2-yl)oxy)ethyl)ammonio)ethyl) phosphate;
(12) 2-((2-(((2R,3R,4S,5S,6R)-3-acetamido-4,5-dihydroxy-6-(hydroxymethyl)tetrahydro-2H-pyranyl)oxy)ethyl)dimethylammonio)ethyl ((R)-2,3-bis(stearoyloxy)propyl) phosphate;
(13) (R)-2,3-bis(stearoyloxy)propyl (2-(dimethyl(2-(((2R,3R,4S,5R,6R)-3,4,5-trihydroxy-6-(hydroxymethyl)tetrahydro-2H-pyran-2-yl)oxy)ethyl)ammonio)ethyl) phosphate;
(14) 2-((2-(((2R,3R,4S,5R,6R)-3-acetamido-4,5-dihydroxy-6-(hydroxymethyl)tetrahydro-2H-pyranyl)oxy)ethyl)dimethylammonio)ethyl ((R)-2,3-bis(stearoyloxy)propyl) phosphate;
(15) (R)-2,3-bis(stearoyloxy)propyl (2-(dimethyl(2-(((2S,3S,4S,5S,6R)-3,4,5-trihydroxy-6-(hydroxymethyl)tetrahydro-2H-pyran-2-yl)oxy)ethyl)ammonio)ethyl) phosphate;
(16) Man-1,2,3-triazole-PEG2000-DSPE;
(17) Man-DIFO-PEG2000-DSPE;
(18) Man-BCN-PEG2000-DSPE;
(19) Man-triarylphosphine-PEG2000-DSPE;
(20) Man-TCO-PEG2000-DSPE;
(21) Man-succinamide-PEG2000-carbamate-DSPE;
(22) Man-amide-PEG2000-amide-DSPE;
(23) Man-pentanoate-PEG2000-amide-DSPE;
(24) Man-succinamide-PEG2000-DMG;
(25) Man-amide-PEG2000-DMG;
(26) Man-pentanoate-PEG2000-DMG;
(27) (R)-2,3-bis(stearoyloxy)propyl (2-(dimethyl(2-(4-oxo-4-((2-(((2S,3S,4S,5S,6R)-3,4,5-trihydroxy-6-(hydroxymethyl)tetrahydro-2H-pyran-2-yl)oxy)ethyl)amino)butanamido)ethyl)ammonio)ethyl) phosphate;
(28) (R)-2,3-bis(stearoyloxy)propyl (3,3-dimethyl-10,13-dioxo-16-(((2S,3S,4S,5S,6R)-3,4,5-trihydroxy-6-(hydroxymethyl)tetrahydro-2H-pyran-2-yl)oxy)-6-oxa-3,9,14-triazahexadecan-3-ium-1-yl) phosphate;
(29) Man-succinamide-PEG2000-DSG;
(30) Man-succinamide-PEG2000-DOG;
(31) Man-succinamide-PEG2000-DLG;
(32) Man-PEG2000-(10Z,29Z)-tetraconta-10,29-dien-20-yl;
(33) Man-PEG2000-(7Z,10Z,29Z,32Z)-tetraconta-7,10,29,32-tetraen-20-yl;
(34) Tri-Man-DBCO-PEG2000-DMG;
(35) Tri-Man-PEG-DBCO-PEG2000-DMG;
(36) Tri-(PEG-Man)-PEG-DBCO-PEG2000-DMG;
(37) Tri-Man-PEG2000-DMG;
(38) Tri-Man-PEG2000-DSPE;
(39) (R)-2,3-bis(stearoyloxy)propyl (2-(4-((2-(((2R,3R,4R,5S,6R)-4,5-dihydroxy-6-(hydroxymethyl)-3-(trimethylammonio)tetrahydro-2H-pyran-2-yl)oxy)ethyl)amino)-4-oxobutanamido)ethyl) phosphate;
(40) (R)-2,3-bis(stearoyloxy)propyl (2-(4-((2-(((2R,3R,4R,5R,6R)-4,5-dihydroxy-6-(hydroxymethyl)-3-(trimethylammonio)tetrahydro-2H-pyran-2-yl)oxy)ethyl)amino)-4-oxobutanamido)ethyl) phosphate;
(41) (R)-2,3-bis(stearoyloxy)propyl (2-(4-((2-(((2R,3R,4R,5S,6R)-3-(dimethylammonio)-4,5-dihydroxy-6-(hydroxymethyl)tetrahydro-2H-pyran-2-yl)oxy)ethyl)amino)-4-oxobutanamido)ethyl) phosphate;
(42) (R)-2,3-bis(stearoyloxy)propyl (2-(4-((2-(((2R,3R,4R,5R,6R)-3-(dimethylammonio)-4,5-dihydroxy-6-(hydroxymethyl)tetrahydro-2H-pyran-2-yl)oxy)ethyl)amino)-4-oxobutanamido)ethyl) phosphate;
(43) Glu-carbamate-ethylethoxy-PEG2000-DMG;
(44) GlcNAc-carbamate-ethylethoxy-PEG2000-DMG;
(45) Gal-carbamate-ethylethoxy-PEG2000-DMG;
(46) GalNAc-carbamate-ethylethoxy-PEG2000-DMG;
(47) Man-carbamate-ethylethoxy-PEG2000-DMG;
(48) Di(tris(Man)methylamide)amidoamine-PEG2000-DMG;
(49) Tris(2-(tri-Man-amidoamine)ethoxy)methylamine-PEG2000-DMG;
(50) Di(tris(Man)methylamine)thioureaethylamine-PEG2000-DMG;
(51) G1-bis-MPA-(Man-succinamide)-PEG2000-DMG;
(52) Di((di(Man-ethylamidoamine))ethyl amidoamine)ethylamine-PEG2000-DMG;
(53) Di(di((di(Man-ethylamidoamine))ethyl amidoamine)ethylamidoamine)ethylamine-PEG2000-DMG;
(54) G1-polylysine-(Man-succinamide)-ethylamine-PEG2000-DMG;
(55) G2-polylysine-(Man-succinamide)-ethylamine-PEG2000-DMG.

8. A lipid nanoparticle composition comprising: ionizable lipids;
the lipid compound of claim 1, an isomer thereof, or a pharmaceutically acceptable salt thereof as phospholipid 1;
phospholipid 2;
cholesterol;
the lipid compound of claim 1, an isomer thereof, or a pharmaceutically acceptable salt thereof as PEG-lipid 1; and
PEG-lipid 2.

9. The lipid nanoparticle composition of claim 8,
wherein the ionizable lipid is one or more selected from the group consisting of (6Z,9Z,28Z,31Z)-heptatriaconta-6,9,28,31-tetraen-19-yl 4-(dimethylamino)butanoate (DLin-MC3-DMA), [(4-hydroxybutyl)azanediyl]di(hexane-6,1-diyl) bis(2-hexyldecanoate) (ALC-0315), 8-[(2-hydroxyethyl)[6-oxo-6-(undecyloxy)hexyl]amino]octanoic acid (SM-102), 1-linoleoyl-2-linoleyloxy-3-dimethylaminopropane (DLin-2-DMAP), 1,2-dilinoleylcarbamoyloxy-3-dimethylaminopropane (DLin-C-DAP), 1,2-dilinoleoyl-3-dimethylaminopropane (DLin-DAP), 1,2-dilinoleyloxy-N,N-dimethylaminopropane (DLin-DMA), 2,2-dilinoleyl-4-dimethylaminomethyl-[1,3]-dioxolane (DLin-K-DMA), 2,2-dilinoleyl-4-(2-dimethylaminoethyl)-[1,3]-dioxolane (DLin-KC2-DMA), 1,2-dioleoyl-3-dimethylammonium propane (DODAP), N,N-dimethyl-(2,3-dioleyloxy)propylamine (DODMA), dioctadecylamidoglycylcarboxyspermine (DOGS), spermine cholesteryl carbamate (GL-67), bis-guanidinium-spermidine-cholesterol (BGTC), 3β-(N-(N',N'-dimethylaminoethane)carbamoyl)cholesterol (DC-Chol), 1,1'-(2-(4-(2-((2-(bis(2-hydroxydecyl)amino)ethyl)(2-hydroxydecyl)amino)ethyl)piperazin-1-yl)ethylazanediyl)didodecan-2-ol (C12-200), N-tert-butyl-N'-tetradecylaminopropionamidine (diC14-amidine), dimethyldioctadecylammonium bromide (DDAB), N-(1,2-dimyristyloxyprop-3-yl)-N,N-dimethyl-N-hydroxyethylammonium bromide (DMRIE), N,N-dioleyl-N,N-dimethylammonium chloride (DODAC), dioleyloxypropyl-3-dimethylhydroxyethylammonium bromide (DORIE), N-(1-(2,3-dioleyloxy)propyl)-N-(2-(sperminecarboxamido)ethyl)-N,N-dimethylammonium trifluoroacetate (DOSPA), 1,2-dioleoyltrimethylammonium propane chloride (DOTAP), N-(1-(2,3-dioleyloxy)propyl)-N,N,N-trimethylammonium chloride (DOTMA), and aminopropyl-dimethyl-bis(dodecyloxy)propanaminium bromide (GAP-DLRIE).

10. The lipid nanoparticle composition of claim 8,
wherein phospholipid 2 is one or more selected from the group consisting of 1,2-dioleoyl-sn-glycero-3-phosphoethanolamine (DOPE), 1,2-distearoyl-sn-glycero-3-phosphocholine (DSPC), 1-palmitoyl-2-oleoyl-sn-glycero-3-phosphocholine (POPC), egg phosphatidylcholine (EPC), 1,2-dioleoyl-sn-glycero-3-phosphocholine (DOPC), 1,2-dipalmitoyl-sn-glycero-3-phosphocholine (DPPC), 1,2-dioleoyl-sn-glycero-3-phosphorylglycerol (DOPG), 1,2-dipalmitoyl-sn-glycero-3-phosphorylglycerol (DPPG), 1,2-distearoyl-sn-glycero-3-phosphoethanolamine (DSPE), phosphatidylethanolamine (PE), 1,2-dipalmitoyl-sn-glycero-3-phosphoethanolamine (DPPE), 1,2-dioleoyl-sn-glycero-3-phosphoethanolamine (DOPE), 1-palmitoyl-2-oleoyl-sn-glycero-3-phosphoethanolamine (POPE), 1-palmitoyl-2-oleoyl-sn-glycero-3-phosphocholine (POPC), 1,2-dioleoyl-sn-glycero-3-(phospho-L-serine) (DOPS), ceramide and sphingomyelin.

11. The lipid nanoparticle composition of claim 8,
wherein PEG-lipid 2 is one or more selected from the group consisting of PEG-dilauroylglycerol, PEG-dimyristoylglycerol (PEG-DMG), PEG-dipalmitoylglycerol, PEG-distearoylglycerol (PEG-DSG), PEG-dilaurylglycamide, PEG-dimyristylglycamide, PEG-dipalmitoylglycamide, PEG-distearoylglycamide, PEG-cholesterol, PEG-DMB (3,4-ditetradecoxylbenzyl-omega-methyl-poly(ethylene glycol) ether), 1,2-distearoyl-sn-glycero-3-phosphoethanolamine-N-(methoxy(polyethylene glycol)-2000) (PEG-DSPE), and 1,2-dimyristoyl-sn-glycero-3-phosphoethanolamine-N-(methoxy(polyethylene glycol)-2000) (PEG-DMPE).

12. The lipid nanoparticle composition of claim 8,
wherein the lipid nanoparticle is capable of encapsulating one or more active substances selected from the group consisting of RNA, siRNA, shRNA, rRNA, tRNA, mRNA, miRNA, saRNA, circRNA, DNA, cDNA, plasmid, DNAzyme, ribozyme, PNA, an aptamer, an antisense oligonucleotide (ASO), and CRISPR.

13. A method of preparing the active substance-lipid nanoparticle, comprising the mixing step of: the organic phase in which ionizable lipids, phospholipids, cholesterol, and PEG-lipids are mixed at a molar ratio of (20-60):(0-25):(30-60):(0-10), respectively; and the aqueous phase in which active substances are dissolved,
wherein the phospholipids comprise phospholipid 1 and phospholipid 2, which are mixed at a molar ratio of (0.005-100):(0-99.995), respectively, and phospholipid 1 comprising the lipid compound represented by Formula 1 of claim 1, an isomer thereof, or a pharmaceutically acceptable salt thereof,
wherein the PEG-lipids comprise PEG-lipid 1 and PEG-lipid 2, which are mixed at a molar ratio of (0.005-100):(0-99.995), respectively, and PEG-lipid 1 comprising the lipid compound represented by Formula 1 of claim 1, an isomer thereof, or a pharmaceutically acceptable salt thereof, and
wherein mass ratio between the ionizable lipid and the active substance is (2-80):1.

14. The method of preparing the active substance-lipid nanoparticle of claim 13,
wherein the phospholipids comprise:
the lipid compound of claim 1, an isomer thereof, or a pharmaceutically acceptable salt thereof as phospholipid 1; and
phospholipid 2,
wherein phospholipid 2 is one or more selected from the group consisting of 1,2-dioleoyl-sn-glycero-3-phosphoethanolamine (DOPE), 1,2-distearoyl-sn-glycero-3-phosphocholine (DSPC), 1-palmitoyl-2-oleoyl-sn-glycero-3-phosphocholine (POPC), egg phosphatidylcholine (EPC), 1,2-dioleoyl-sn-glycero-3-phosphocholine (DOPC), 1,2-dipalmitoyl-sn-glycero-3-phosphocholine (DPPC), 1,2-dioleoyl-sn-glycero-3-phosphorylglycerol (DOPG), 1,2-dipalmitoyl-sn-glycero-3-phosphorylglycerol (DPPG), 1,2-distearoyl-sn-glycero-3-phosphoethanolamine (DSPE), phosphatidylethanolamine (PE), 1,2-dipalmitoyl-sn-glycero-3-phosphoethanolamine (DPPE), 1,2-dioleoyl-sn-glycero-3-phosphoethanolamine (DOPE), 1-palmitoyl-2-oleoyl-sn-glycero-3-phosphoethanolamine (POPE), 1-palmitoyl-2-oleoyl-sn-glycero-3-phosphocholine (POPC), 1,2-dioleoyl-sn-glycero-3-(phospho-L-serine) (DOPS), ceramide and sphingomyelin.

15. The method of preparing the active substance-lipid nanoparticle of claim 13,
wherein the PEG-lipids comprise:
the lipid compound of claim 1, an isomer thereof, or a pharmaceutically acceptable salt thereof as PEG-lipid 1; and
PEG-lipid 2,
wherein PEG-lipid 2 is one or more selected from the group consisting of PEG-dilauroylglycerol, PEG-dimyristoylglycerol (PEG-DMG), PEG-dipalmitoylglycerol, PEG-distearoylglycerol (PEG-DSG), PEG-dilaurylglycamide, PEG-dimyristylglycamide, PEG-dipalmitoylglycamide, PEG-distearoylglycamide, PEG-cholesterol, PEG-DMB (3,4-ditetradecoxylbenzyl-omega-methyl-poly(ethylene glycol) ether), 1,2-distearoyl-sn-glycero-3-phosphoethanolamine-N-(methoxy(polyethylene glycol)-2000) (PEG-DSPE), and 1,2-dimyristoyl-sn-glycero-3-phosphoethanolamine-N-(methoxy(polyethylene glycol)-2000) (PEG-DMPE).

16. A composition for nucleic acid delivery, comprising the lipid compound of claim 1, an isomer thereof, or a pharmaceutically acceptable salt thereof.

17. A tissue-specific drug delivery system comprising the lipid nanoparticle composition of claim 8.

18. A drug delivery system for the treatment of retinal diseases, comprising the lipid nanoparticle composition of claim 8.

19. The drug delivery system for the treatment of retinal diseases of claim 18, wherein the drug delivery system for the treatment of retinal diseases is administered via subretinal injection or intravitreal injection.

20. A drug delivery system for the prevention of infectious diseases, comprising the lipid nanoparticle composition of claim 8.

21. The drug delivery system for the prevention of infectious diseases of claim 20,
wherein the drug delivery system for the prevention of infectious diseases is administered via intramuscular injection or intranasal injection.

22. A drug delivery system for the treatment of cancer, brain diseases, or liver diseases, comprising the lipid nanoparticle composition of claim 8.

23. The drug delivery system for the treatment of cancer, brain diseases, or liver diseases of claim 22,
wherein the drug delivery system is administered via intravenous injection.
